# EUROPÄISCHE PATENTANMELDUNG

(11) **EP 3 922 342 A2**
(43) Veröffentlichungstag der Anmeldung: **15.12.2021**
(21) Anmeldenummer: 21177345.2
(22) Anmeldetag: 02.06.2021
(51) Int. Cl.: B01D 53/04, A61M 16/10, B01D 53/30, A61M 16/00, A61M 11/00

(54) **AUFNAHME-ANORDNUNG UND VERFAHREN UMFASSEND EINE FILTEREINHEIT ZUM AUFNEHMEN VON GAS AUS EINEM MEDIZINISCHEN GERÄT**

(30) Priorität: 12.06.2020 DE 102020115602; 12.11.2020 LU 102192
(71) Anmelder: Drägerwerk AG & Co. KGaA, 23558 Lübeck (DE)
(72) Erfinder: Schüler, Hans Ulrich, 23558 Lübeck (DE); Heesch, Ralf, 23558 Lübeck (DE); Schmid, Robert, 23558 Lübeck (DE)

(57) **Zusammenfassung**

Die Erfindung betrifft eine Aufnahme-Anordnung (100) und ein Verfahren zum Aufnehmen von Gas aus einem medizinischen Gerät (1). Die Aufnahme-Anordnung umfasst eine Zuführleitung (6), eine Abführleitung (8), eine Filtereinheit (4) mit einem Filter und mindestens einen Pufferspeicher. Die Zuführleitung (6) stellt eine Fluidverbindung zwischen dem medizinischen Gerät (1) und der Filtereinheit (4) her. Die Abführleitung (8) stellt eine Fluidverbindung zwischen der Filtereinheit (4) und einer Fluidaufnahme (7) her. Das Gas wird von dem medizinischen Gerät (1) ausgestoßen und durch die Zuführleitung (6) zu der Filtereinheit (4) und von dort durch die Abführleitung (8) zu der Fluidaufnahme (7) geleitet. Der Filter filtert mindestens einen Gas-Bestandteil aus dem Gas, das durch die Filtereinheit (4) geleitet wird, heraus. Der oder jeder Pufferspeicher vermag zeitweise Gas aufzunehmen und wieder abzugeben.

## Beschreibung

Die Erfindung betrifft eine Aufnahme-Anordnung, welche Gas aus einem medizinischen Gerät aufzunehmen vermag, insbesondere aus einem Anästhesiegerät. Die Erfindung betrifft weiterhin ein Verfahren, um Gas mithilfe einer solchen Aufnahme-Anordnung aus einem medizinischen Gerät aufzunehmen.

In einer Anwendung der Aufnahme-Anordnung und des Verfahrens ist das medizinische Gerät ein Anästhesiegerät, welches mit einem Patienten verbunden ist und mittels eines Beatmungskreislaufs den Patienten beatmet und narkotisiert. Das Anästhesiegerät wird mit den benötigten Gasen versorgt und versorgt seinerseits den Patienten mit einem Gasgemisch, wobei das Gasgemisch Sauerstoff enthält und mit mindestens einem Narkosemittel angereichert ist, sodass der Patient vollständig oder wenigstens teilweise narkotisiert ist. Typischerweise führt das Anästhesiegerät Beatmungshübe aus, wobei mit jedem Beatmungshub eine bestimmte Menge von Gas zu dem Patienten gefördert wird. Die vom Patienten ausgeatmete Luft gelangt in einer Ausgestaltung wieder zu dem medizinischen Gerät, und das medizinische Gerät filtert Kohlendioxid aus der ausgeatmeten Luft heraus.

In US 2001 / 0 025 640 A1 wird ein Narkosemittel-Filter (anaesthetic gas filter 2) mit einem Gehäuse (enclosure 4), einem Einlass (inlet 6) und einem Auslass (outlet 8) beschrieben. Eine Trennwand (partition 18) mit einem Loch 20 unterteilt das Gehäuse 4 in eine erste Kammer (first absorption volume 10) für einen ersten Filter (first absorbent 12) und eine zweite Kammer (second absorption volume 14) für einen zweiten Filter (second absorbent 16). Falls der erste Filter 12 aus der ersten Kammer 10 entnommen wird, so verbindet eine erste Leitung (first shunt line 24) den Einlass 6 mit dem Loch 20 und damit mit der zweiten Kammer 18. Falls der zweite Filter 16 aus der zweiten Kammer 14 entnommen wird, so verbindet eine zweite Leitung (second shunt line 26) das Loch 20 und damit die zweite Kammer 14 mit dem Auslass 8. Zwei Narkosemittel-Indikatoren 22 und 22B zeigen an, wann der erste Filter 12 bzw. der zweite Filter 16 ausgetauscht werden müssen.

Häufig wird bei einer künstlichen Beatmung dem Beatmungskreislauf mehr Gas zugeführt als wieder entnommen. Im Beatmungskreislauf fällt in diesem Fall überschüssiges Gas an. Dieses überschüssige Gas muss aus dem Beatmungskreislauf entfernt werden. Es enthält häufig ein Narkosemittel. Selbstverständlich soll der Patient nicht gefährdet werden. Insbesondere müssen die künstliche Beatmung und den Narkotisierung aufrechterhalten werden.

Der Erfindung liegt die Aufgabe zugrunde, eine Aufnahme-Anordnung und ein Verfahren zur Aufnahme von Gas aus einem medizinischen Gerät bereitzustellen, welche ihre Funktion besser als bekannte Aufnahme-Anordnungen und Verfahren ausführen.

Die Aufgabe wird durch eine Aufnahme-Anordnung mit den Merkmalen des Anspruchs 1 sowie durch ein Verfahren mit den Merkmalen des Anspruchs 31 gelöst. Vorteilhafte Ausgestaltungen sind in den Unteransprüchen angegeben. Vorteilhafte Ausgestaltungen der erfindungsgemäßen Aufnahme-Anordnung sind, soweit sinnvoll, auch Vorteile des erfindungsgemäßen Verfahrens und umgekehrt.

Die erfindungsgemäße Aufnahme-Anordnung umfasst eine Zuführleitung und eine Abführleitung. Die Zuführleitung lässt sich mit dem medizinischen Gerät verbinden, aus dem aufzunehmendes Gas austritt, bevorzugt lösbar verbinden, und ist dazu ausgestaltet, nach Herstellen der Verbindung mindestens eine Fluidverbindung zwischen dem medizinischen Gerät und der Aufnahme-Anordnung herzustellen. Die Abführleitung lässt sich mit einer Fluidaufnahme verbinden, bevorzugt lösbar verbinden, und ist dazu ausgestaltet, nach Herstellen der Verbindung mindestens eine Fluidverbindung zwischen der Fluidaufnahme und der Aufnahme-Anordnung herzustellen. Die Fluidaufnahme kann eine stationäre oder eine mobile Fluidaufnahme sein und fungiert als eine Senke für Fluid. Die Fluidaufnahme kann auch eine räumlich beabstandete Ansaugung innerhalb oder außerhalb eines Gebäudes sein. Alternativ führt die Abführleitung in die Umgebung.

Die erfindungsgemäße Aufnahme-Anordnung umfasst weiterhin eine Filtereinheit. Die Filtereinheit umfasst mindestens einen Filter.

Der oder jeder Filter der Filtereinheit ist dazu ausgestaltet, aus einem Gas, welches durch den Filter geleitet wird, jeweils mindestens einen Gas-Bestandteil herauszufiltern. Die Konstruktion und / oder die Beschaffenheit des Filters und / oder seine Materialien legen fest, welcher Gas-Bestandteil oder welche Gas-Bestandteile der Filter herausfiltert. Bevorzugt vermag der Filter aus dem Gas mindestens ein Narkosemittel herauszufiltern, d.h. das oder mindestens ein Gas-Bestandteil ist ein Narkosemittel.

Die Aufnahme-Anordnung ist dazu ausgestaltet, die folgende Funktion zu realisieren: Gas, welches aus dem medizinischen Gerät ausgestoßen wird oder abgesogen wird oder auf andere Weise aus dem Gerät austritt, wird durch die Zuführleitung zu der Filtereinheit und durch die Filtereinheit hindurch geleitet, wobei die Filtereinheit einen Filter umfasst. Wenigstens ein Teil dieses Gases, bevorzugt das gesamte Gas, wird durch den Filter hindurch geführt, und der Filter filtert den oder mindestens einen Gas-Bestandteil aus dem Gas heraus, und zwar wenigstens teilweise, bevorzugt vollständig - es sei denn, der Filter ist verbraucht oder defekt. Das Gas, das durch die Filtereinheit geflossen ist und aus dem mindestens ein Gas-Bestandteil herausgefiltert worden ist, wird durch die Abführleitung hindurch zu der Fluidaufnahme oder in die Umgebung geleitet.

Das erfindungsgemäße Verfahren wird unter Verwendung einer solchen Aufnahme-Anordnung durchgeführt und umfasst die folgenden Schritte:
- Zwischen dem medizinischen Gerät und der Zuführleitung wird mindestens eine Fluidverbindung hergestellt.
- Zwischen der Abführleitung und der Fluidaufnahme wird mindestens eine weitere Fluidverbindung hergestellt. Alternativ ist oder wird die Abführleitung in die Umgebung geführt.
- Gas tritt aus dem medizinischen Gerät aus. Insbesondere stößt das medizinische Gerät dieses Gas aus, und / oder dieses Gas wird aus dem medizinischen Gerät abgesogen. Das ausgetretene Gas gelangt in die Zuführleitung.
- Die Aufnahme-Anordnung leitet das ausgetretene Gas durch die Zuführleitung, die Filtereinheit und die Abführleitung hindurch zu der Fluidaufnahme.
- Während dieses Gas durch die Filtereinheit hindurchgeleitet wird, fließt wenigstens ein Teil dieses Gases, bevorzugt dauerhaft oder wenigstens zeitweise das gesamte Gas, durch den Filter der Filtereinheit hindurch.
- Der Filter filtert aus dem Gas, welches durch den Filter fließt, den oder mindestens einen vorgegebenen Gas-Bestandteil heraus, insbesondere mindestens ein Narkosemittel. In die Abführleitung gelangt daher Gas, aus dem der oder mindestens ein Gas-Bestandteil vollständig oder wenigstens teilweise herausgefiltert ist.

Die erfindungsgemäße Aufnahme-Anordnung umfasst weiterhin mindestens einen Pufferspeicher, wobei der oder jeder Pufferspeicher jeweils wenigstens zeitweise in einer Fluidverbindung mit der Zuführleitung, wenigstens zeitweise in einer Fluidverbindung mit der Abführleitung oder wenigstens zeitweise sowohl mit der Zuführleitung als auch mit der Abführleitung in jeweils einer Fluidverbindung steht. Gas, welches aus dem medizinischen Gerät ausgetreten ist, kann daher in den oder einen Pufferspeicher gelangen und wieder aus diesem Pufferspeicher austreten und dann in die Abführleitung gelangen. Das erfindungsgemäße Verfahren wird unter Verwendung mindestens eines solchen Pufferspeichers durchgeführt.

Die erfindungsgemäße Aufnahme-Anordnung und das erfindungsgemäße Verfahren reduzieren einerseits die Gefahr, dass Gas mit einem unerwünschten Gas-Bestandteil in die Umgebung des medizinischen Geräts austritt. Insbesondere reduzieren die erfindungsgemäße Aufnahme-Anordnung und das erfindungsgemäße Verfahren die Gefahr, dass ein Narkosemittel in die Umgebungsluft austritt und das ausgetretene Narkosemittel Personen, die sich in der Nähe des medizinischen Geräts und / oder in der Nähe des künstlich beatmeten Patienten aufhalten, insbesondere Personen, die in einem Krankenhaus arbeiten, gesundheitlich gefährdet. Diese Wirkung wird insbesondere dadurch erzielt, dass die erfindungsgemäße Aufnahme-Anordnung und das erfindungsgemäße Verfahren das aus dem medizinischen Gerät austretende oder ausgetretene Gas in die Fluidaufnahme leiten. Dadurch wird vollständig oder wenigstens bis zu einem gewissen Grad verhindert, dass ausgestoßenes Gas austritt und in die Umgebung des medizinischen Geräts gelangt.

Andererseits reduziert die Erfindung die Gefahr, dass austretendes oder ausgetretenes Gas in ein Versorgungssystem oder in die Umwelt gelangt, beispielsweise in einen Außenbereich außerhalb eines Krankenhauses.

In einer Anwendung der Erfindung ist die Fluidaufnahme eine stationäre Fluidaufnahme und gehört insbesondere zu einer stationären Infrastruktur eines Gebäudes. Bei dieser Anwendung reduzieren die erfindungsgemäße Aufnahme-Anordnung und das erfindungsgemäße Verfahren in vielen Fällen die Gefahr, dass ein Gas mit einem unerwünschten Gas-Bestandteil über die Fluidaufnahme in ein stationäres Infrastruktursystem gelangt, mit welchem die stationäre Fluidaufnahme verbunden ist, insbesondere in ein Infrastruktursystem in einem Krankenhaus. Dieses Infrastruktursystem könnte das Gas mit dem unerwünschten Gas-Bestandteil wieder an ein medizinisches Gerät abgeben, was oft unerwünscht ist, oder dieses Gas in die Umwelt ausstoßen, was zu einer Umweltbelastung führen könnte. Diese unerwünschte Auswirkung wird insbesondere durch die Filtereinheit mit dem Filter verhindert. Als Bestandteil der Filtereinheit lässt sich ein solcher Filter verwenden, der den oder mindestens einen vorgegebenen Gas-Bestandteil aus einem Gas herauszufiltern vermag, während das Gas durch den Filter geleitet wird.

Möglich ist auch, dass die Fluidaufnahme aufgenommenes Gas über die Abführleitung in die Nähe einer Abluftanlage leitet und die Abluftanlage das Gas aus einem geschlossenen oder auch teilweise oder vollständig geöffneten Raum ansaugt und aufnimmt. Bei dieser Anwendung wird die Abführleitung nicht notwendigerweise mit einer stationären Fluidaufnahme verbunden. Auch bei dieser Anwendung verhindert die Erfindung, das unerwünschte Gas-Bestandteile eines Gases in die Umwelt gelangen.

Erfindungsgemäß umfasst die Aufnahme-Anordnung zusätzlich mindestens einen Pufferspeicher. Dieser Pufferspeicher steht in einer Alternative direkt oder indirekt in mindestens einer Fluidverbindung mit der Zuführleitung, in einer anderen Alternative direkt oder indirekt in mindestens einer Fluidverbindung mit der Abführleitung und in einer dritten Alternative direkt oder indirekt sowohl in einer Fluidverbindung mit der Zuführleitung als auch in einer Fluidverbindung mit der Abführleitung. "Indirekt" bedeutet, dass die Fluidverbindung durch einen anderen Bestandteil der erfindungsgemäßen Anordnung hindurch geführt ist.

Dieser Pufferspeicher vermag insbesondere dann Gas temporär aufzunehmen und zwischenzuspeichern, welches aus dem medizinischen Gerät ausgestoßen worden oder auf andere Weise ausgetreten ist, wenn die stationäre Fluidaufnahme dieses Gas aktuell überhaupt nicht oder nicht vollständig aufzunehmen vermag. Der Pufferspeicher reduziert die Gefahr, dass in diesem Fall ein Rückstau von ausgestoßenem Gas auftritt. Dieser Rückstau kann auf das medizinische Gerät rückwirken und eine Gefährdung eines mit dem Gerät verbundenen Patienten und / oder eine Beschädigung des medizinischen Geräts bewirken. Ein großer Rückstau könnte außerdem eine Fluidverbindung der Aufnahme-Anordnung undicht werden lassen, etwa aufgrund eines Überdrucks. Dank des Pufferspeichers ist es in vielen Fällen nicht erforderlich, diesen Überdruck dadurch abzubauen, dass das Gas in die Umgebung abgelassen wird. Durch das Ablassen in die Umgebung könnte Narkosemittel in der Umgebung auftreten, was wie oben dargelegt unerwünscht ist. Außerdem ist in vielen Fällen dank des Pufferspeichers keine Handlung eines Menschen erforderlich.

Wenn mehr Gas aus dem medizinischen Gerät austritt und in die Zuführleitung gelangt als die Abführleitung aufnehmen kann, so wird Gas in den oder mindestens einen Pufferspeicher geleitet, und zwar bevorzugt aus der Zuführleitung. Wenn weniger Gas austritt als die Abführleitung aufnehmen kann, so fließt Gas wieder aus dem Pufferspeicher heraus und wird bevorzugt in die Abführleitung geleitet und von dort in die Fluidaufnahme.

In vielen Fällen ist es erforderlich, den Filter von Zeit zu Zeit auszutauschen, insbesondere wenn der Filter sich nach einer längeren Benutzung zugesetzt hat. Häufig muss oder soll eine künstliche Beatmung des Patienten fortgesetzt werden, während der Filter oder die gesamte Filtereinheit ausgetauscht werden. Auch in diesem Fall soll vermieden werden, dass eine große Menge von überschüssigem Gas in die Umgebung gelangt. Insbesondere soll auch bei einem Austausch des Filters vermieden werden, dass eine große Menge von Narkosemittel in die Umgebung gelangt. Der oder mindestens ein erfindungsgemäßer Pufferspeicher der Aufnahme-Anordnung nimmt auch in diesem Fall wenigstens ein Teil des überschüssigen Gases auf, idealerweise das gesamte überschüssige Gas, und gibt es später wieder ab.

Weil erfindungsgemäß mindestens ein Pufferspeicher in einer Fluidverbindung mit der Zuführleitung und / oder mit der Abführleitung steht, ist es nicht erforderlich, zwei Filteraufnahmen in Reihe zu schalten, für jede Fluidaufnahme jeweils eine Umgehungsleitung (Bypass) vorzusehen und sicherzustellen, dass zu jedem Zeitpunkt in mindestens eine Filteraufnahme ein Filter eingesetzt ist. Eine solche Reihenschaltung von zwei Filteraufnahmen erfordert in vielen Fällen mehr Platz als eine einzige Filteraufnahme und ein Pufferspeicher. Der erfindungsgemäße Pufferspeicher kann räumlich entfernt von der Filteraufnahme angeordnet sein. Außerdem ist dank der Erfindung in vielen Fällen keine Umgehungsleitung (Bypass) um eine Filteraufnahme herum erforderlich.

Unter einen "Filter" im Sinne der Ansprüche wird ein Bauteil verstanden, welches mindestens einen Gas-Bestandteil aus einem Gasgemisch herauszufiltern vermag, während dieses Gasgemisch durch den Filter strömt. Der Filter kann das Gas-Bestandteil an sich binden, beispielsweise mit Aktivkohle und / oder weil der Filter Zeolithe enthält. Der Filter kann auch den Gas-Bestandteil chemisch oder thermisch aufbrechen. Der Filter kann auch mechanisch wirken und beispielsweise ein Molekularsieb umfassen. Mehrere Wirkungen können kombiniert werden.

Eine "Leitung" im Sinne der Ansprüche ist eine Fluidführungseinheit, welche zwei Punkte fluiddicht und damit auch gasdicht miteinander verbindet und Fluid von dem einen Punkt zu dem anderen Punkt zu leiten vermag. Die Leitung kann flexibel sein, insbesondere ein Faltenschlauch oder ein glatter Schlauch, oder starr sein, insbesondere eine Röhre oder ein Rohr. Eine Leitung im Sinne der Ansprüche kann auch eine fluiddichte Koppelstelle sein, welche die Filtereinheit mit einem anderen Gerät lösbar verbindet.

Unter einem "Pufferspeicher" für Gas wird eine Kammer zur Aufnahme von Gas verstanden, die von einer Wandung umgeben ist. Dieses Gas enthält in einer Anwendung mindestens ein Narkosemittel. Die Kammer ist so angeordnet, dass sie in einer ersten Zeitspanne mehr von diesem Gas aufzunehmen als abzugeben vermag und in einer nachfolgenden zweiten Zeitspanne das in der ersten Zeitspanne aufgenommene Gas oder eine gleiche Menge dieses Gases wieder abzugeben vermag. In der ersten Zeitspanne ist der Volumenfluss dieses Gases in den Pufferspeicher also größer als der Volumenfluss dieses Gases aus dem Pufferspeicher, in der zweiten Zeitspanne kleiner. Erfindungsgemäß fließt in der ersten Zeitspanne Gas aus der Zuführleitung und / oder aus der Abführleitung in den oder einen Pufferspeicher, und in der zweiten Zeitspanne fließt das Gas aus dem Pufferspeicher wieder in die Zuführleitung und / oder in die Abführleitung.

In einer Ausgestaltung ist der oder ein Pufferspeicher nur an einer Stelle mit der Zuführleitung und überhaupt nicht mit der Abführleitung oder nur an einer Stelle mit der Abführleitung und überhaupt nicht mit der Zuführleitung verbunden. Bei dieser Ausgestaltung fließt Gas an dieser Stelle in den Pufferspeicher und an derselben Stelle wieder aus dem Pufferspeicher heraus.

In einer anderen Ausgestaltung ist der oder ein Pufferspeicher an zwei verschiedenen, voneinander beabstandeten Stellen mit der Zuführleitung und / oder mit der Abführleitung verbunden. Bei dieser anderen Ausgestaltung ist die Querschnittsfläche des Pufferspeichers größer als die Querschnittsfläche der Zuführleitung und größer als die Querschnittsfläche der Abführleitung. Dank dieser größeren Querschnittsfläche vermag bei dieser anderen Ausgestaltung der Pufferspeicher Gas zwischenzuspeichern.

In einer Ausgestaltung vergrößert sich im Verlauf der ersten Zeitspanne das Volumen der Kammer und / oder der in der Kammer herrschende Druck. Bevorzugt verdoppelt sich das Volumen oder der Druck in dieser ersten Zeitspanne. Das Volumen oder der Druck verringern sich wieder im Verlaufe der zweiten Zeitspanne. Bevorzugt sind nach Ende der zweiten Zeitspanne das Volumen und der Druck wieder so klein wie zu Beginn der ersten Zeitspanne. Die Wandung des Pufferspeichers kann starr oder elastisch sein, sodass das Volumen des Pufferspeichers konstant oder aber abhängig von der Differenz zwischen dem Druck im Pufferspeicher und dem Druck von außen auf den Pufferspeicher veränderlich ist.

In einer Ausgestaltung trennt die Wandung die Kammer des Pufferspeichers fluiddicht von der Umgebung ab. Möglich ist auch, dass der Pufferspeicher in einer Fluidverbindung mit der Umgebung steht und das Gas in der ersten Zeitspanne Luft aus dem Pufferspeicher verdrängt, sodass Luft in die Umgebung fließt, und in der zweiten Zeitspanne das Gas aus dem Pufferspeicher heraus gefördert wird und wieder Luft in den Pufferspeicher fließt. Bei dieser Ausgestaltung können das Volumen und der Druck in der Kammer konstant bleiben. Auch bei dieser Ausgestaltung reduziert der Pufferspeicher die Gefahr, dass eine große Menge von Narkosemittel in die Umgebung austritt.

Eine Leitung für Gas hat hingegen bei störungsfreien Betrieb stets das gleiche Volumen, steht bei störungsfreien Betrieb nicht in einer Fluidverbindung mit der Umgebung und hat die Aufgabe, Gas von einem Zuführpunkt zu einem Abführpunkt zu leiten. In der Regel ist zu jedem Zeitpunkt der Volumenfluss in die Leitung gleich dem Volumenfluss aus der Leitung.

Erfindungsgemäß umfasst die Filtereinheit einen Filter. Bevorzugt umfasst die Filtereinheit zusätzlich eine Filteraufnahme. Der Filter ist in die Filteraufnahme eingesetzt oder lässt sich in die Filteraufnahme einsetzen und bevorzugt wieder aus der Filteraufnahme entnehmen. Die Filteraufnahme steht in jeweils einer Fluidverbindung mit der Zuführleitung und mit der Abführleitung. Der eingesetzte Filter lässt sich in einer Ausgestaltung wieder aus der Filteraufnahme herausnehmen, beispielsweise um durch einen neuen oder einen anderen Filter (Filter mit anderen Filtereigenschaften) ersetzt zu werden. Gemäß einer bevorzugten Ausgestaltung lässt sich der Filter in die Filteraufnahme einsetzen und später wieder aus der Filteraufnahme entnehmen. Dadurch wird ermöglicht, einen Filter auszutauschen, wenn dieser Filter von dem oder einem Gas-Bestandteil des aus dem medizinischen Gerät ausgetretenen Gases eine solche Menge aufgenommen hat, dass der Filter seine gewünschte Filterwirkung überhaupt nicht oder nicht mehr in ausreichendem Maße erfüllen kann, und / oder wenn der Filter sich zugesetzt hat.

Dank der Filteraufnahme ist es nicht erforderlich, eine Fluidverbindung zwischen der Zuführleitung und der Abführleitung oder mit dem medizinischen Gerät zeitweise zu trennen und später wieder herzustellen, um einen Filter auszutauschen. Dadurch lässt sich ein verbrauchter Filter rasch durch einen neuen Filter ersetzen. Die Gefahr eines Fehlers beim ersten oder einem erneuten Herstellen der Fluidverbindung wird reduziert. Außerdem ist es möglich, dieselbe Aufnahme-Anordnung nacheinander mit unterschiedlichen Filtern in der Filteraufnahme zu verwenden und dadurch verschiedene Gas-Bestandteile aus einem Gas herauszufiltern und / oder die Aufnahme-Anordnung bei unterschiedlichen Umgebungsbedingungen einzusetzen. Dieses Merkmal erleichtert es außerdem, mehrere erfindungsgemäße Aufnahme-Anordnungen für unterschiedliche Verwendungszwecke, insbesondere zum Herausfiltern unterschiedlicher Gas-Bestandteile, bereitzustellen. Diese erfindungsgemäßen Aufnahme-Anordnungen brauchen sich lediglich durch die unterschiedlich ausgestalteten Filter zu unterscheiden. Die übrigen Bestandteile der Aufnahme-Anordnung können übereinstimmen oder sich nur durch Abmessungen unterscheiden.

Bei der Ausgestaltung mit der Filteraufnahme umfasst das erfindungsgemäße Verfahren die folgenden zusätzlichen Schritte:
- Falls nicht bereits geschehen, wird ein Filter in die Filteraufnahme eingesetzt.
- Die Aufnahme-Anordnung leitet das Gas, das aus dem medizinischen Gerät ausgetreten ist, durch die Zuführleitung, die Filteraufnahme und die Abführleitung hindurch zu der Fluidaufnahme oder in die Umgebung.
- Die Aufnahme-Anordnung leitet das Gas dergestalt durch die Filteraufnahme, dass wenigstens ein Teil dieses Gases, bevorzugt das gesamte Gas, durch den Filter hindurchfließt, wobei der Filter in die Filteraufnahme eingesetzt ist.

In einer Alternative bildet der Filter einen festen Bestandteil der Filtereinheit, und die gesamte Filtereinheit ist ein einziges Bauteil. Bevorzugt lassen sich die Zuführleitung und die Abführleitung mit der Filtereinheit verbinden, besonders bevorzugt lösbar verbinden. Falls der Filter verbraucht ist und keine weitere Menge des Gas-Bestandteils aufzunehmen vermag oder falls der Filter zugesetzt ist, wird die Filtereinheit mitsamt dem Filter von der Zuführleitung und von der Abführleitung getrennt, und eine neue Filtereinheit wird mit der Zuführleitung und der Abführleitung verbunden.

Weiter oben wurde bereits eine Ausgestaltung beschrieben, bei der die Filtereinheit eine Filteraufnahme und mindestens einen Filter umfasst. Der oder ein Filter lässt sich in die Filteraufnahme einsetzen und wieder aus der Filteraufnahme entnehmen, beispielsweise um den Filter durch einen neuen Filter zu ersetzen. In einer bevorzugten Weiterbildung umfasst die Filtereinheit weiterhin einen Filter-Sensor. Dieser Filter-Sensor vermag automatisch festzustellen, ob ein Filter in die Filteraufnahme eingesetzt ist oder nicht. Bevorzugt erzeugt der Filter-Sensor eine Meldung, falls kein Filter in die Filteraufnahme eingesetzt ist. In einer Realisierungsform erzeugt der Filter-Sensor diese Meldung nur dann, wenn für einen Zeitraum, der länger als eine vorgegebene Zeitspanne ist, kein Filter in die Filteraufnahme eingesetzt ist. In einer Abwandlung erzeugt der Filter-Sensor nur dann eine Meldung, wenn für einen Zeitraum länger als die Zeitspanne kein Filter eingesetzt ist und zusätzlich Gas durch die Zuführleitung fließt. Die Ausgestaltung mit dem Filter-Sensor reduziert das Risiko, dass unbeabsichtigt kein Filter in die Filteraufnahme eingesetzt ist und daher kein Gas-Bestandteil herausgefiltert werden kann und eine größere Menge des Gas-Bestandteils in die Fluidaufnahme gelangt. Andererseits wird in vielen Fällen ein unnützer Alarm beim Austausch eines Filters vermieden.

Bevorzugt steht der oder mindestens ein Pufferspeicher nicht nur in jeweils einer Fluidverbindung mit der Zuführleitung und / oder der Abführleitung, sondern zusätzlich in einer Fluidverbindung mit der Umgebung, und zwar dauerhaft oder wenigstens bei einer großen Druckdifferenz zwischen dem Inneren des Pufferspeichers und der Umgebung. Der Pufferspeicher befindet sich also zwischen der Zuführleitung und / oder der Abführleitung und der Umgebung. Dadurch wird weiter die Gefahr reduziert, dass bei einer großen Menge von Gas, welches aus dem medizinischen Gerät ausgetreten ist, dann ein Rückstau auftritt, wenn weder die stationäre Fluidaufnahme noch der Pufferspeicher diese Menge vollständig aufnehmen können. Zwar ist unerwünscht, dass Gas in die Umgebung austritt. Ein Austritt des Gases in die Umgebung ist aber meistens weniger schädlich als ein Rückstau dieses Gases. Die Ausgestaltung, dass der Pufferspeicher in einer Fluidverbindung mit der Umgebung steht, ist insbesondere dann von Vorteil, wenn der Pufferspeicher starr ist, d.h. sein Volumen nicht veränderlich ist. Ein Pufferspeicher mit konstantem Volumen ist häufig mechanisch stabiler als ein Pufferspeicher mit veränderlichem Volumen, weil sein Gehäuse starr sein kann. Die Fluidverbindung mit der Umgebung führt außerdem dazu, dass im Pufferspeicher kein Überdruck entsteht, was in manchen Fällen unerwünscht ist.

In einer Ausgestaltung umfasst die Filtereinheit mehrere parallel geschaltete Filter. Die Aufnahme-Anordnung ist so ausgestaltet, dass Gas durch die Zuführleitung, durch mindestens einen dieser Filter und die Abführleitung hindurchströmt. Ein Filter befindet sich also im Strömungsweg von der Zuführleitung zu der Abführleitung. In einer Ausgestaltung strömt das Gas durch einen Filter, und mindestens ein weiterer Filter befindet sich in einer Reserve-Position. In einer anderen Ausgestaltung wird das Gas auf mindestens zwei parallele Ströme aufgeteilt, und jeder Strom fließt durch jeweils einen Filter.

In einer Realisierungsform umfasst die Filtereinheit eine Aufnahmeeinheit, insbesondere eine Revolveraufnahme, für mindestens zwei Filter. Diese Aufnahmeeinheit ist relativ zu der Zuführleitung und relativ zu der Abführleitung beweglich. Die aktuelle Position der Aufnahmeeinheit relativ zu der Zuführleitung legt fest, welcher Filter aktuell verwendet wird und welcher Filter sich in einer Reserve-Position befindet. In einer anderen Realisierungsform umfasst die Filtereinheit eine Weiche, welche das aus der Zuführleitung fließende Gas wahlweise zu dem ersten Filter oder zu den zweiten Filter leitet, oder ein Y-Stück, welches das Gas auf zwei parallele Ströme und damit auf zwei Filter aufteilt.

In einer Anwendung der Ausgestaltung mit mehreren Filtern sind alle Filter der Filtereinheit gleichartig ausgestaltet und vermögen insbesondere den gleichen Gas-Bestandteil herauszufiltern. Wenn ein Filter verbraucht oder zugesetzt ist, wird die Aufnahmeeinheit relativ zu der Zuführleitung und relativ zu der Abführleitung bewegt, sodass das Gas durch einen neuen Filter fließt. Beispielsweise umfasst die Filtereinheit einen Antrieb für die Aufnahmeeinheit.

In einer anderen Realisierungsform vermag ein erster Filter in der Aufnahmeeinheit einen ersten Gas-Bestandteil herauszufiltern, ein zweiter Filter einen zweiten Gas-Bestandteil und optional ein dritter Filter den ersten, den zweiten oder einen dritten Gas-Bestandteil. Die drei Gas-Bestandteile unterscheiden sich chemisch und / oder mechanisch voneinander. Je nach gewünschter oder erforderlicher Anwendung werden der erste Filter oder der zweite Filter oder der optionale dritte Filter in den Strömungsweg von der Zuführleitung in die Abführleitung bewegt. Bevorzugt umfasst die Filtereinheit eine Auswahleinheit, mit deren Hilfe ein Benutzer einen herauszufilternden Gas-Bestandteil auswählen kann. Möglich ist auch, dass flussaufwärts von den Filtern ein Sensor angeordnet ist, der erkennt, welcher herauszufilternde Gas-Bestandteil sich in dem Gas befindet, das zu den Filtern fließt. Abhängig von einem Signal dieses Sensors wird der richtige Filter in den Strömungsweg des Gases bewegt. Oder eine entsprechende Meldung wird an einen Benutzer ausgegeben.

Verschiedene Ausgestaltungen des Pufferspeichers sind möglich, und wenigstens einige dieser Ausgestaltungen lassen sich miteinander kombinieren. Möglich ist also, dass die erfindungsgemäße Aufnahme-Anordnung genau einen Pufferspeicher oder aber mindestens zwei gleichartige oder auch mindestens zwei unterschiedliche Pufferspeicher umfasst.

In einer bereits erwähnten Ausgestaltung umfasst die Filtereinheit eine Filteraufnahme, in die sich ein Filter einsetzen lässt oder eingesetzt ist. In einer Weiterbildung dieser Ausgestaltung ist der Raum, welcher von der Filteraufnahme umschlossen wird, in mindestens eine Richtung größer als der Filter - genauer gesagt: Zwischen mindestens einer Innenwand der Filteraufnahme und der gegenüberliegenden Außenwand des Filters befindet sich ein Zwischenraum. Möglich ist, dass dieser Zwischenraum an zwei Außenwänden des Filters angrenzt, z.B. an die Mantelfläche und an eine Stirnfläche, oder auch nur an eine Außenwand, z.B. nur an die Mantelfläche. Dieser Zwischenraum steht in jeweils einer Fluidverbindung mit der Zuführleitung und der Abführleitung und bildet den oder einen Pufferspeicher oder gehört zu dem oder einem Pufferspeicher. Bevorzugt nimmt dieser Zwischenraum mindestens 10%, besonders bevorzugt sogar mindestens 20%, insbesondere mindestens 30% des von der Filteraufnahme umgebenen Raums ein. Bevorzugt ist der Zwischenraum fluiddicht gegen die Umgebung abgegrenzt, sodass kein Gas aus dem Zwischenraum direkt in die Umgebung entweichen kann.

Die Ausgestaltung mit dem Zwischenraum nimmt verglichen mit anderen möglichen Ausgestaltungen des Pufferspeichers besonders wenig Platz ein und ist mechanisch robust. Außerdem erleichtert es der Zwischenraum in manchen Ausgestaltungen einem Menschen, den Filter im Zwischenraum zu greifen und aus der Filteraufnahme zu entfernen.

In einem Zeitraum, in dem kein Filter in die Filteraufnahme eingesetzt ist, steht häufig der gesamte Innenraum der Filteraufnahme als ein Pufferspeicher zur Verfügung. Bei der gerade beschriebenen Ausgestaltung ist dieser Innenraum größer, als wenn die Filteraufnahme ohne Zwischenraum den Filter umschließt, und vermag daher mehr Gas aufzunehmen. Häufig gelangt daher nur relativ wenig Gas, welches aus dem medizinischen Gerät ausgetreten ist, in die Umgebung, obwohl kein Filter eingesetzt ist und die Filteraufnahme daher zur Umgebung offen ist. Diese gewünschte Situation tritt insbesondere dann ein, wenn die Filteraufnahme nach oben offen ist und das Gas, das aus dem medizinischen Gerät austritt, schwerer als Luft ist. Die meisten Narkosemittel sind schwerer als Luft.

Bei der Ausgestaltung mit der Filteraufnahme, die einen Hohlraum umschließt, ist bevorzugt der Hohlraum des Pufferspeichers von dem Hohlraum zur Aufnahme des Filters räumlich getrennt und bevorzugt abgetrennt, insbesondere fluiddicht abgetrennt, beispielsweise durch eine stationäre oder bewegliche Platte.

In einer anderen Ausgestaltung umfasst der Pufferspeicher ein Gehäuse, welches einen Hohlraum umschließt und in einer Ausgestaltung ein starres Gehäuse und in einer anderen Ausgestaltung ein elastisches Gehäuse und in einer dritten Ausgestaltung ein Gehäuse mit mindestens einem starren Bereich und mindestens einem elastischen Bereich ist. Dieses Gehäuse steht in jeweils mindestens einer Fluidverbindung mit der Zuführleitung und der Abführleitung. Die Ausgestaltung mit dem Pufferspeicher, der ein Gehäuse umfasst, lässt sich auch mit einer Ausgestaltung, bei der die Filtereinheit ein einziges Bauteil bildet, kombinieren. In einer Ausgestaltung steht dieses Gehäuse des Pufferspeichers zusätzlich in mindestens einer Fluidverbindung mit der Filtereinheit, und die Fluidverbindung besteht zwischen dem Gehäuse und der Zuführleitung und / oder führt durch die Filtereinheit durch.

Bevorzugt sind in den Innenraum, den das Gehäuse des Pufferspeichers umschließt, mehrere Bleche oder sonstige geeignete Führungselemente eingebaut, welche einen bevorzugt mäandernden Pfad durch das Gehäuse bereitstellen. Diese Ausgestaltung ermöglicht es, dass das Gehäuse in einer Fluidverbindung mit der Umgebung steht und trotzdem eine nur relativ geringe Menge von Gas aus dem Gehäuse in die Umgebung austritt. Der mäandernde Pfad führt dazu, dass Gas nur langsam durch den Innenraum des Gehäuses fließt. Der Pufferspeicher kann ein starres Gehäuse aufweisen.

In einer Ausgestaltung steht dieses Gehäuse in einer Fluidverbindung mit der Umgebung, sodass überschüssiges Gas in die Umgebung austreten kann. Falls in dem Gehäuse ein mäandernder Pfad gebildet wird, so tritt häufig trotz der Fluidverbindung nur relativ wenig Gas in die Umgebung aus.

Bevorzugt ist in beiden Ausgestaltungen der Pufferspeicher so ausgestaltet, dass er nach dem Prinzip "LastIn - FirstOut" funktioniert.

In einer Ausgestaltung ist das Gehäuse des Pufferspeichers senkrecht oder schräg unterhalb der Filtereinheit angeordnet und steht bevorzugt in einer Fluidverbindung mit der Filtereinheit. Diese Ausgestaltung ist insbesondere dann von Vorteil, wenn ein Gas mit dem herauszufilternden Gas-Bestandteil schwerer als Luft ist und daher in der Filtereinheit nach unten sinkt. Die meisten Narkosemittel sind schwerer als Luft.

In einer Ausgestaltung ist das Gehäuse des Pufferspeichers flussabwärts von der Filtereinheit angeordnet. In einer alternativen Ausgestaltung ist die Filtereinheit flussabwärts von dem Gehäuse des Pufferspeichers angeordnet. Die Bezeichnung "flussabwärts" bezieht sich auf eine Fließrichtung von Fluid von der Zuführleitung zu der Abführleitung. Diese beiden Ausgestaltungen führen zu einer geringeren vertikalen Ausdehnung der erfindungsgemäßen Aufnahme-Anordnung verglichen mit anderen möglichen Ausgestaltungen.

Möglich ist, dass sowohl zwischen dem eingesetzten Filter und der Filteraufnahme ein Zwischenraum entsteht als auch ein separates Gehäuse des Pufferspeichers unterhalb oder flussabwärts oder flussaufwärts von der Filtereinheit angeordnet ist. Diese Ausgestaltung ermöglicht es, einen besonders großen Pufferspeicher bereitzustellen. Das Risiko, dass Gas in die Umgebung austritt, wird weiter reduziert.

Wie bereits erwähnt, umfasst die Filtereinheit in einer Ausgestaltung eine Filteraufnahme, in die sich ein Filter einsetzen lässt oder eingesetzt ist. In einer weiteren Fortbildung dieser Ausgestaltung ist in die Filteraufnahme eine Platte eingesetzt. Diese Platte unterteilt den Innenraum der Filteraufnahme in einen Filter-Hohlraum, der zur Aufnahme des Filters ausgestaltet ist, und einen Pufferspeicher-Hohlraum, der zu dem oder einem Pufferspeicher gehört oder einen Pufferspeicher ausbildet. Mindestens dann, wenn kein Filter in die Filteraufnahme eingesetzt ist, steht dieser Pufferspeicher-Hohlraum einerseits in einer Fluidverbindung mit der Zuführleitung und andererseits in einer Fluidverbindung mit der Abführleitung.

Die Platte reduziert die Gefahr, dass Gas aus der Fluidaufnahme in die Umgebung gelangt, und zwar auch dann, wenn kein Filter in die Filteraufnahme eingesetzt ist. Diese Ausgestaltung mit der Platte in der Filteraufnahme spart verglichen mit anderen möglichen Ausgestaltungen des Pufferspeichers ebenfalls Platz ein. Erspart wird die Notwendigkeit, ein separates Gehäuse für den Pufferspeicher vorzusehen.

In einer Ausgestaltung befindet sich der Pufferspeicher-Hohlraum unterhalb des Filter-Hohlraums. Bevorzugt ist die Platte horizontal angeordnet. Diese Ausgestaltung ist insbesondere dann von Vorteil, wenn ein Gas mit dem herauszufilternden Gas-Bestandteil schwerer als Luft ist und daher in der Filteraufnahme nach unten sinkt. Die meisten Narkosemittel sind schwerer als Luft.

Die Platte kann stationär in der Filteraufnahme befestigt sein. In einer Ausgestaltung lässt sich die Platte hingegen relativ zu der Filteraufnahme zwischen einer Park-Position und einer Pufferspeicher-Position hin- und herbewegen. Wenn ein Filter in die Filteraufnahme eingesetzt ist, befindet sich die Platte in der Park-Position, ansonsten in der Pufferspeicher-Position. Die Zuführleitung umfasst mindestens eine Austrittsöffnung, die bevorzugt im Inneren der Filteraufnahme angeordnet ist. Die Abführleitung umfasst mindestens eine Eintrittsöffnung, die bevorzugt im Inneren der Filteraufnahme angeordnet ist.

Wenn die Platte in der Pufferspeicher-Position ist, ist gemäß dieser Ausgestaltung folgende Situation hergestellt:
- Die oder eine Austrittsöffnung verbindet die Zuführleitung mit dem Pufferspeicher-Hohlraum.
- Die oder eine Eintrittsöffnung verbindet den Pufferspeicher-Hohlraum mit der Abführleitung.
- Dadurch steht der Pufferspeicher-Hohlraum sowohl mit der Zuführleitung als auch mit der Abführleitung in jeweils einer Fluidverbindung.
- In einer Ausgestaltung dichtet die Platte den Pufferspeicher-Hohlraum weitgehend fluiddicht gegen die Umgebung und gegen den Filter-Hohlraum ab. Die Filteraufnahme dichtet beide Hohlräume gegen die Umgebung ab.

Wenn die Platte in der Park-Position ist, so ist gemäß dieser Ausgestaltung folgende Situation hergestellt:
- Die oder eine Austrittsöffnung verbindet die Zuführleitung mit dem Filter-Hohlraum.
- Die oder eine Eintrittsöffnung verbindet die Abführleitung mit dem Filter-Hohlraum.
- Dadurch stehen der Filter-Hohlraum und auch ein richtig eingesetzter Filter sowohl mit der Zuführleitung mit der Abführleitung in einer Fluidverbindung.
- In einer Ausgestaltung trennt die Platte in der Park-Position die beiden Öffnungen, welche die Zuführleitung bzw. die Abführleitung mit dem Filter-Hohlraum verwenden, fluiddicht von dem Pufferspeicher-Hohlraum.
- In einer anderen Ausgestaltung steht auch dann, wenn die Platte in der Park-Position ist, die Zuführleitung und / oder die Abführleitung in einer Fluidverbindung mit dem Pufferspeicher-Hohlraum.

Wenn die Platte in der Pufferspeicher-Position ist, stellt der Pufferspeicher-Hohlraum einen Pufferspeicher im Inneren der Filteraufnahme bereit. Dieser Pufferspeicher vermag daher dann überschüssiges Gas aus dem medizinischen Gerät aufzunehmen, wenn aktuell kein Filter eingesetzt ist. Diese Situation tritt insbesondere dann auf, wenn der Filter in der Filteraufnahme ausgetauscht wird. Die Platte in der Pufferspeicher-Position trennt den Pufferspeicher-Hohlraum vor der Umgebung und reduziert die Menge von Gas, welches in die Umgebung austritt.

Dank der beweglichen Platte braucht kein weiterer Mechanismus vorgesehen zu werden, um einerseits eine gewünschte Filterung zu erzielen und andererseits zu verhindern, dass Gas in die Umgebung auftritt. Die Platte ermöglicht eine relativ einfache mechanische Konstruktion.

In einer Ausgestaltung ist die Filtereinheit so ausgestaltet, dass dann, wenn ein Filter in die Filteraufnahme eingesetzt wird, die Platte aus der Pufferspeicher-Position in die Park-Position bewegt wird. Wenn der Filter wieder aus der Filteraufnahme entnommen wird, wird die Platte aus der Park-Position wieder in die Pufferspeicher-Position bewegt. Beispielsweise ist eine mechanische oder pneumatische Feder bestrebt, die Platte in die Pufferspeicher-Position zu bewegen und in dieser zu halten. Bevorzugt stützt diese Feder sich an einem Gehäuse der Filteraufnahme ab.

In einer Ausgestaltung ist die Filtereinheit so ausgestaltet, dass eine Bewegung der Platte aus der Park-Position in die Pufferspeicher-Position den Filter aus der Filteraufnahme heraus schiebt.

In einer Ausgestaltung ist die Platte mit einem Betätigungselement verbunden, welches bevorzugt von außerhalb der Filteraufnahme zugänglich ist und sich bevorzugt manuell betätigen lässt, um die Platte zwischen den beiden Positionen hin und her zu bewegen. Beispielsweise wird ein Filter in die Filteraufnahme eingesetzt, und anschließend wird das Betätigungselement betätigt, um die Platte in die Park-Position zu bewegen. Dadurch wird auch der Filter vollständig in die Filteraufnahme eingeführt. Um den Filter zu entfernen, wird das Betätigungselement erneut betätigt und verschiebt die Platte in die Pufferspeicher-Position. Dadurch wird der Filter aus der Filteraufnahme herausgeschoben.

In einer weiteren Ausgestaltung vermag ein Antrieb die Platte zwischen den beiden Positionen hin und her zu bewegen. Der Antrieb lässt sich bevorzugt von außen ansteuern und / oder aktivieren.

Bevorzugt rastet die Platte in wenigstens einer Endposition ein, besonders bevorzugt in beide Endpositionen.

Bevorzugt ist der Park-Hohlraum kleiner als der Pufferspeicher-Hohlraum. Diese Ausgestaltung spart daher verglichen mit anderen möglichen Ausgestaltungen des Pufferspeichers Platz ein.

In einer Realisierungsform sind ein manuell zu betätigendes Betätigungselement und / oder ein Antrieb mit der Platte verbunden. Mit Hilfe des Betätigungselements lässt sich die Platte von der einen Position in die andere Position bewegen. Der Antrieb vermag die Platte von der einen Position in die andere Position zu bewegen.

In einer bevorzugten Realisierungsform bewirken hingegen das Einsetzen sowie das Herausnehmen des Filters, dass die Platte von der einen Position in die andere Position bewegt wird und dadurch der Fluss von Gas durch den Filter-Hohlraum bzw. durch die Pufferspeicher-Hohlraum geleitet wird. Diese Ausgestaltung erspart ein separates Betätigungselement oder einen separaten Antrieb. Möglich ist, dass eine Rasteinheit die Platte in einer Position hält.

Bevorzugt ist die Platte in der Filteraufnahme federnd gelagert. Mindestens ein Federelement ist bestrebt, die Platte in die Pufferspeicher-Position zu bewegen, insbesondere nach oben zu bewegen. Das Federelement stützt sich bevorzugt an der Filteraufnahme ab. Ein eingesetzter Filter bewegt die Platte gegen die Federkraft in die Park-Position, bevorzugt aufgrund seines eigenen Gewichts und / oder aufgrund einer Krafteinwirkung beim Einsetzen des Filters. Diese Ausgestaltung erspart die Notwendigkeit, ein Betätigungselement und / oder eine Handlung und / oder einen Antrieb vorzusehen, um die Platte von der einen Position in die andere Position zu bewegen. Vielmehr wird die Platte automatisch beim Einsetzen und beim Herauslösen von der einen in die andere Position bewegt. Möglich ist auch, dass eine Rastverbindung oder eine Arretierung die Platte in der Park-Position hält.

Gemäß der gerade beschriebenen Ausgestaltung trennt eine Platte im Inneren der Filteraufnahme einen Filter-Hohlraum von einem Pufferspeicher-Hohlraum, und zwar in einer Ausgestaltung fluiddicht.

In einer Fortbildung dieser fluiddichten Ausgestaltung befindet sich im Inneren der Filteraufnahme zusätzlich ein Schieber, der bevorzugt mechanisch mit der Platte verbunden ist. Auch der Schieber lässt sich zwischen einer Park-Position und einer Pufferspeicher-Position hin und her bewegen, bevorzugt gemeinsam mit der Platte.

Die Zuführleitung umfasst gemäß dieser Ausgestaltung zwei räumlich voneinander beabstandete Austrittsöffnungen, nämlich eine Filter-Austrittsöffnung und eine Pufferspeicher-Austrittsöffnung. Auch die Abführleitung umfasst zwei räumlich voneinander beabstandete Eintrittsöffnungen, nämlich eine Filter-Eintrittsöffnung und eine Pufferspeicher-Eintrittsöffnung.

Wenn die Platte und der Schieber in der Park-Position sind, so ist die Zuführleitung über die Filter-Austrittsöffnung mit dem Filter-Hohlraum verbunden. Die Abführleitung ist über die Filter-Eintrittsöffnung mit dem Filter-Hohlraum verbunden. Der Schieber versperrt die Pufferspeicher-Austrittsöffnung und die Pufferspeicher-Eintrittsöffnung. Gas, welches aus der Zuführleitung austritt, wird durch den Filter-Hohlraum und damit durch den Filter hindurch in die Abführleitung geführt. Der Schieber in der Park-Position verhindert, dass Gas den Filter in der Filteraufnahme umgeht und von der Zuführleitung durch den Pufferspeicher-Hohlraum direkt in die Abführleitung fließt.

Wenn die Platte und der Schieber in der Pufferspeicher-Position sind, so ist die Zuführleitung über die Pufferspeicher-Austrittsöffnung mit dem Pufferspeicher-Hohlraum verbunden. Die Abführleitung ist über die Pufferspeicher-Eintrittsöffnung mit dem Pufferspeicher-Hohlraum verbunden. Der Schieber versperrt die Filter-Austrittsöffnung und die Filter-Eintrittsöffnung. Gas, welches aus der Zuführleitung austritt, wird durch den Pufferspeicher-Hohlraum hindurch in die Abführleitung geführt. Der Schieber in der Pufferspeicher-Position verhindert, dass Gas aus der Zuführleitung oder der Abführleitung austritt und in den Filter-Hohlraum und von dort in die Umgebung gelangt. Auch in dieser Realisierungsform trennt die Platte in der Pufferspeicher-Position den Pufferspeicher-Hohlraum von dem Filter-Hohlraum, und zwar bevorzugt fluiddicht.

Die gerade beschriebene Ausführungsform mit der Platte und dem Schieber führt zu einer besonders einfachen mechanischen Realisierung. Möglich ist, dass ein manuell zu betätigendes Betätigungselement und / oder ein Antrieb den Schieber zu bewegen vermögen. Bevorzugt bewirkt das Einsetzen eines Filters, dass die Platte und der Schieber in die Park-Position bewegt und in dieser gehalten werden. Das Herausnehmen des Filters bewirkt, dass die Platte und der Schieber in die Pufferspeicher-Position bewegt werden und in dieser gehalten werden. In einer Realisierungsform ist mindestens eine mechanische oder pneumatische Feder bestrebt, die Platte und den Schieber in die Pufferspeicher-Position zu bewegen und in dieser zu halten. Die Feder stützt sich bevorzugt an der Filteraufnahme ab. Anstelle einer Feder lässt sich auch ein anderes elastisches Element verwenden, welches eine Kraft auf den Schieber ausübt.

In einer Ausgestaltung lässt sich das Volumen des oder mindestens eines Pufferspeichers verändern, bevorzugt reversibel verändern. Insbesondere umfasst der Pufferspeicher eine Wand, die vollständig oder wenigstens in einem Bereich elastisch ist. Dadurch kann der Pufferspeicher sich dehnen und wieder zusammenziehen. Die Zufuhr von Gas dehnt den Pufferspeicher und vergrößert dadurch dessen Volumen. Der gedehnte Pufferspeicher vermag gespeichertes Gas wieder abzugeben, wobei der Pufferspeicher sich zusammenzieht und sein Volumen sich verringert. Diese Ausgestaltung führt in vielen Fällen zu einem besonders einfach aufgebauten Pufferspeicher. Im Inneren des Pufferspeichers entsteht ein geringerer Überdruck verglichen mit einem Pufferspeicher, der ein starres Gehäuse aufweist.

Bevorzugt steht dieser dehnbare Pufferspeicher in einer Fluidverbindung mit der Zuführleitung, ist also flussaufwärts von der Filtereinheit angeordnet. Diese Ausgestaltung bewirkt, dass die Menge von Gas, die pro Zeiteinheit die Filtereinheit erreicht, weniger stark zeitlich variiert. Dann, wenn die Aufnahme-Anordnung mit einem medizinischen Gerät verbunden ist, reduziert diese Ausgestaltung die Gefahr, dass ein unerwünscht hoher Druck auf das medizinische Gerät und damit auf einen Patienten einwirkt, der mit dem medizinischen Gerät verbunden ist. Der dehnbare Pufferspeicher kann auch in einer Fluidverbindung mit der Abführleitung stehen und flussabwärts von der Filtereinheit angeordnet sein.

In einer Ausgestaltung umfasst die Aufnahme-Anordnung einen dehnbaren Pufferspeicher und einen Pufferspeicher mit einem starren Gehäuse und / oder einen Pufferspeicher im Inneren der Filteraufnahme. Bevorzugt ist der elastische Pufferspeicher flussaufwärts von dem Pufferspeicher mit dem starren Gehäuse angeordnet. Dadurch variiert die Menge von Gas, welche pro Zeiteinheit den starren Pufferspeicher erreicht, zeitlich weniger stark. Möglich ist auch, dass der dehnbare Pufferspeicher in einer Fluidverbindung mit dem anderen Pufferspeicher steht.

In einer Anwendung wird die erfindungsgemäße Aufnahme-Anordnung mit einem medizinischen Gerät, insbesondere einem Anästhesiegerät, verbunden. Als der oder ein Pufferspeicher wird ein Beutel für die manuelle Beatmung eines Patienten verwendet. Dieser Beutel ist mit der Zuführleitung oder mit der Abführleitung verbunden. Ein derartiger Handbeatmungs-Beutel ist oft bereits für die medizinische Verwendung zugelassen und besitzt standardisierte Abmessungen. Ein Anschlussstück der erfindungsgemäßen Aufnahme-Anordnung lässt sich an diese Abmessungen anpassen.

In einer Ausgestaltung umfasst die Aufnahme-Anordnung zusätzlich mindestens ein Überdruck-Ventil in oder an der Zuführleitung. Dieses Überdruck-Ventil öffnet sich, wenn ein am Überdruck-Ventil anliegender Differenzdruck oberhalb einer vorgegebenen Überdruck-Schranke liegt. Das geöffnete Überdruckventil lässt Gas in die Umgebung ab. Das Überdruck-Ventil ist so positioniert, dass es sich automatisch öffnet, wenn die Differenz zwischen dem Druck in der Zuführleitung und dem Umgebungsdruck um die Zuführleitung herum oberhalb der Überdruck-Schranke liegt. Dadurch baut das Überdruck-Ventil einen Überdruck in der Zuführleitung ab und reduziert insbesondere die Gefahr, dass ein Rückstau in der Zuführleitung auftritt. Dieser Rückstau kann sich in unerwünschter Weise auf ein Gerät auswirken, das Gas in die Zuführleitung ausstößt.

Diese Überdruck-Schranke ist in einer Ausgestaltung durch die Konstruktion des Überdruck-Ventils vorgegeben. In einer anderen Ausgestaltung misst ein Druck-Sensor die am Überdruck-Ventil anliegende Druckdifferenz. In einer Realisierungsform empfängt ein Steuergerät oder ein Komparator Messwerte von diesem Druck-Sensor und steuert das Überdruck-Ventil an, und zwar so, dass das Überdruck-Ventil sich dann öffnet, wenn die Druckdifferenz oberhalb der Überdruck-Schranke liegt. In einer anderen Realisierungsform werden die Messwerte von dem Druck-Sensor in einer von einem Menschen wahrnehmbaren Form ausgegeben. Beispielsweise wird ein Alarm ausgegeben, wenn eine Druckdifferenz oberhalb der Überdruck-Schranke detektiert wird. Die beiden Realisierungsformen, dass das Steuergerät / der Komparator das Überdruck-Ventil ansteuert und dass die Messwerte und / oder ein Alarm ausgegeben werden, lassen sich miteinander kombinieren.

In einer Ausgestaltung ist das oder ein Überdruck-Ventil in Reihe mit der Filtereinheit und flussaufwärts von der Filtereinheit angeordnet, insbesondere in oder an der Zuführleitung. In einer anderen Ausgestaltung ist das oder ein Überdruck-Ventil parallel zu der Filtereinheit angeordnet. Bei dieser anderen Anordnung fließt bei geöffnetem Überdruck-Ventil ein Teil eines Gases durch die Zuführleitung und um die Filtereinheit herum. In beiden Ausgestaltungen führt die Zuführleitung bei geschlossenem Überdruck-Ventil das gesamte Gas zu der Filtereinheit.

In einer Ausgestaltung gibt das geöffnete Überdruck-Ventil Gas in die Umgebung ab. In einer anderen Ausgestaltung verbindet eine Fluidführungseinheit, insbesondere ein Schlauch oder eine sonstige Leitung, das Überdruck-Ventil mit der Abführleitung. Bei geöffnetem Überdruck-Ventil ist eine Fluidverbindung von der Zuführleitung durch diese Fluidführungseinheit hindurch zu der Abführleitung hergestellt. In einer dritten Ausgestaltung ist das Überdruck-Ventil im Inneren der Filtereinheit angeordnet. Auch bei geöffnetem Überdruck-Ventil fließt Gas durch die Zuführleitung und die Filtereinheit hindurch in die Abführleitung, ohne in die Umgebung auszutreten. Jedoch fließt mindestens ein Teil dieses Gases nicht durch den Filter der Filtereinheit, sondern um den Filter herum. Diese beiden bevorzugten Ausgestaltungen reduzieren die Gefahr, dass bei geöffnetem Überdruck-Ventil Gas in die Umgebung austritt. Vielmehr fließt bei einem Überdruck Gas um den Filter herum in die Abführleitung.

Eine weitere mögliche Anordnung des Überdruck-Ventils lässt sich mit der Ausgestaltung kombinieren, dass die Filtereinheit eine Filteraufnahme umfasst, wobei sich ein Filter in die Filteraufnahme einsetzen lässt und wobei die Filteraufnahme in jeweils einer Fluidverbindung mit der Zuführleitung und mit der Abführleitung steht. Bei dieser Ausgestaltung wird bei geschlossenem Überdruck-Ventil das Gas aus der Zuführleitung durch den Filter hindurch zu der Abführleitung geführt. Auch bei geöffnetem Überdruck-Ventil fließt das Gas aus der Zuführleitung durch das Innere der Filteraufnahme hindurch in die Abführleitung. Jedoch fließt dann wenigstens ein Teil des Gases an dem Filter vorbei. Diese Ausgestaltung reduziert das Risiko, dass bei geöffnetem Überdruck-Ventil Gas in die Umgebung entweichen kann.

Das oder ein Überdruck-Ventil kann auch an der Filteraufnahme oder an oder in der Abführleitung angeordnet sein und bei einem Überdruck Gas in die Umgebung ablassen.

In einer Fortbildung der Ausgestaltung mit dem Überdruck-Ventil ist in eine Wand der Filtereinheit mindestens eine Öffnung eingelassen. Das Überdruck-Ventil verschließt diese Öffnung. Wenn das Überdruck-Ventil sich aufgrund eines Überdrucks öffnet, so steht die Filtereinheit durch die Öffnung hindurch in einer Fluidverbindung mit der Umgebung. Möglich ist auch, dass eine Öffnung parallel zu dem Überdruck-Ventil angeordnet ist. Bevorzugt verschließt das Überdruck-Ventil sich wieder automatisch, wenn Überdruck abgebaut ist.

In einer weiteren Ausgestaltung umfasst die Aufnahme-Anordnung mindestens ein Unterdruck-Ventil. Dieses Unterdruck-Ventil öffnet sich, wenn die Differenz zwischen dem Druck in der Zuführleitung oder im Inneren der Filtereinheit einerseits und dem umgebenden Druck andererseits unterhalb einer vorgegebenen Unterdruck-Schranke liegt. Ein solcher großer Unterdruck ist häufig unerwünscht, weil dieser Unterdruck Gas aus einem Gerät saugen könnte, das mit der Zuführleitung verbunden ist. Falls das Gerät ein medizinisches Gerät ist und mit einem Patienten verbunden ist, so könnte der Patient durch einen Unterdruck gefährdet werden. Ein solcher Unterdruck kann beispielsweise dann entstehen, wenn Gas aktiv aus der Abführleitung abgesogen wird und die Filtereinheit und / oder die Zuführleitung verstopft sind oder die Zuführleitung ein Gerät nicht korrekt mit der Filtereinheit verbindet (Fehlkonnektierung).

Analog zu dem Überdruck-Ventil ist in einer Realisierungsform die Unterdruck-Schranke durch die Konstruktion des Unterdruck-Ventils vorgegeben. In einer anderen Realisierungsform bewirkt ein Steuergerät oder ein Komparator, dass das Unterdruck-Ventil sich öffnet, wenn der Unterdruck unter die Unterdruck-Schranke gefallen ist. In einer dritten Realisierungsform wird bei einem Unterdruck unterhalb der Unterdruck-Schranke eine Meldung in einer von einem Menschen wahrnehmbaren Form ausgegeben. Die Realisierungsformen, dass ein Steuergerät automatisch das Unterdruck-Ventil öffnet und dass eine Meldung ausgegeben wird, lassen sich miteinander kombinieren.

In einer Ausgestaltung ist in eine Wand der Filtereinheit mindestens eine Öffnung eingelassen, bevorzugt mehrere Öffnungen. Durch diese Öffnungen ist eine Fluidverbindung zwischen dem Inneren der Filtereinheit und der Umgebung herstellbar. Falls die Filtereinheit eine Filteraufnahme umfasst, in die sich ein Filter einsetzen lässt oder eingesetzt ist, so sind die Öffnungen bevorzugt in eine Wand der Filteraufnahme eingelassen.

Bei geöffnetem Unterdruck-Ventil ist die Fluidverbindung zwischen dem Inneren der Filtereinheit und der Umgebung hergestellt, so dass Gas aus der Umgebung durch das Unterdruck-Ventil und die Öffnung oder Öffnungen hindurch in das Innere der Filtereinheit fließen kann. Das oder ein geschlossenes Unterdruck-Ventil verschließt diese Öffnung oder Öffnungen bei einem Unterdruck kleiner als die Unterdruck-Schranke und unterbricht die Fluidverbindung zwischen dem Inneren der Filtereinheit und der Umgebung. Die Öffnungen begrenzen den Fluss von Gas in die Filtereinheit hinein.

In einer Abwandlung dieser Realisierung ist ein Unterdruck-Ventil in eine Wand des Pufferspeichers oder in eine Wand der Zuführleitung eingelassen. Bei einem Unterdruck im Pufferspeicher, der unterhalb der Unterdruck-Schranke liegt, fließt Gas aus der Umgebung in den Pufferspeicher hinein. Diese Ausgestaltung reduziert das Risiko, dass ein zu großer Unterdruck im Pufferspeicher Gas aus der Zuführleitung saugt oder dass der Pufferspeicher aufgrund eines zu großen Unterdrucks beschädigt wird.

In einer bevorzugten Realisierung der Ausgestaltung, dass die Filtereinheit eine Filteraufnahme umfasst, umfasst die Filteraufnahme einen Topf oder ist als ein Topf ausgestaltet. Ein Filter lässt sich von oben in diesen Topf einsetzen und wieder aus dem Topf entnehmen. Bevorzugt lässt sich dieser Topf mit einem Deckel fluiddicht verschließen, und der Deckel lässt sich wieder entfernen, beispielsweise um den Filter auszutauschen. Wenn das zu filternde Gas schwerer als Luft ist, sinkt es in diesem Topf nach unten, und keine relevante Menge kann direkt in die Umgebung entweichen. Auch dann, wenn in die topfförmige Filteraufnahme aktuell kein Filter eingesetzt ist, beispielsweise während der Filter ausgetauscht wird, gelangt nur relativ wenig ausgetretenes Gas in die Umgebung, selbst dann, wenn der Topf nicht verschlossen ist, sondern zur Umgebung offen ist, insbesondere während der Filter ausgetauscht wird. Diese Ausgestaltung spart Zeit beim Austausch des Filters ein.

Erfindungsgemäß steht die Filtereinheit in mindestens einer Fluidverbindung mit der Zuführleitung. In einer Ausgestaltung ist ein Abschnitt dieser Zuführleitung durch den Innenraum der Filtereinheit hindurchgeführt. Dieser Abschnitt befindet sich bevorzugt zwischen einer Wand der Filtereinheit und dem in die Filteraufnahme eingesetzten Filter. Falls die Filtereinheit eine Filteraufnahme umfasst, ist bevorzugt ein Abschnitt der Zuführleitung durch den Innenraum der Filteraufnahme hindurchgeführt und befindet sich bei eingesetztem Filter zwischen dem Filter und der Wand der Filteraufnahme. Eine Austrittsöffnung in diesem Abschnitt der Zuführleitung legt fest, an welcher Stelle Gas aus der Zuführleitung austritt und in die Filteraufnahme und damit in den Filter eintritt. Diese Austrittsöffnung kann sich nahe einem Boden der Filtereinheit, insbesondere nahe der Filteraufnahme befinden. Diese Ausgestaltung ist insbesondere dann von Vorteil, wenn das Gas, aus dem der oder mindestens ein Gas-Bestandteil herausgefiltert werden soll, schwerer als Luft ist, was insbesondere bei einem Gasgemisch mit Narkosemittel der Fall ist. Die Austrittsöffnung kann auch in der Nähe des oberen Abschlusses der Filtereinheit positioniert sein. Möglich ist auch, dass eine erste Austrittsöffnung zu dem Filter und eine zweite Austrittsöffnung zu einem Pufferspeicher in der Filteraufnahme führt. Je nachdem, ob ein Filter eingesetzt ist oder nicht, ist die eine Austrittsöffnung geöffnet und die andere geschlossen oder umgekehrt.

Auch die Abführleitung kann einen Abschnitt umfassen, der im Inneren der Filtereinheit angeordnet ist und sich zwischen einer Wand der Filtereinheit und einem eingesetzten Filter befindet. Falls die Filtereinheit eine Filteraufnahme umfasst, befindet sich bevorzugt dieser Abschnitt der Abführleitung zwischen dem Filter und einer Wand der Filtereinheit. Dieser Abschnitt kann eine Eintrittsöffnung aufweisen, optional mindestens zwei Eintrittsöffnungen.

Diese Ausgestaltung lässt sich mit der oben beschriebenen Ausgestaltung kombinieren, bei der ein Hohlraum zwischen der Filteraufnahme und dem Filter auftritt und zu dem Pufferspeicher gehört. Bevorzugt sind der Abschnitt der Zuführleitung und der Abschnitt der Abführleitung in der Filteraufnahme fluiddicht von dem Pufferspeicher in der Filteraufnahme getrennt.

Möglich ist, dass ein Gas ausschließlich dadurch in die erfindungsgemäße Aufnahme-Anordnung gelangt, dass es in die Zuführleitung hinein gefördert wird, beispielsweise von einem Gerät ausgestoßen wird, welches mit der Zuführleitung verbunden ist. In einer anderen Ausgestaltung wird zusätzlich oder stattdessen Gas aus der Abführleitung herausgesogen. Gemäß einer Realisierungsform dieser anderen Ausgestaltung umfasst die Aufnahme-Anordnung weiterhin einen Unterdruck-Erzeuger, insbesondere eine Ansaugpumpe, die bevorzugt an der Abführleitung oder flussabwärts von der Abführleitung angeordnet ist. Der Unterdruck-Erzeuger steht in einer Fluidverbindung mit der Abführleitung und erzeugt einen Unterdruck in der Abführleitung. Dieser Unterdruck in der Abführleitung saugt ein Gas an. Das abgesogene Gas wird aus einem mit der Zuführleitung verbundenen Gerät, insbesondere einem medizinischen Gerät, durch die Zuführleitung, die Filtereinheit und die Abführleitung hindurch angesogen. Bevorzugt verbindet mindestens eine Fluidverbindung den oder einen Pufferspeicher mit der Abführleitung. Der Unterdruck, den der Unterdruck-Erzeuger bewirkt, leert den Pufferspeicher durch diese Fluidverbindung hindurch, auch wenn aktuell kein oder nur wenig Gas aus dem medizinischen Gerät austritt.

In einer bereits beschriebenen Ausgestaltung umfasst die Filtereinheit eine Filteraufnahme und einen Filter. Die Filteraufnahme steht in jeweils einer Fluidverbindung mit der Zuführleitung und der Abführleitung. Der Filter lässt sich in die Filteraufnahme einsetzen und wieder aus der Filteraufnahme herausnehmen, beispielsweise um ihn durch einen neuen Filter zu ersetzen. Die gerade beschriebene Ausgestaltung mit dem Unterdruck-Erzeuger erzielt einen weiteren Vorteil, wenn sie in Kombination mit einer Filteraufnahme und einem Filter realisiert wird. Beim Austausch des Filters steht die Filteraufnahme bei vielen Ausgestaltungen wenigstens teilweise mit der Umgebung in einer Fluidverbindung. Der Unterdruck-Erzeuger saugt auch dann Gas aus der Filteraufnahme, wenn kein Filter eingesetzt ist. Dadurch reduziert der Unterdruck-Erzeuger die Menge von Gas, die aus der Filteraufnahme in die Umgebung austritt.

Der Unterdruck-Erzeuger ist auch dann von Vorteil, wenn der oder mindestens ein Pufferspeicher dauerhaft oder wenigstens zeitweise mit der Umgebung in einer Fluidverbindung steht, beispielsweise um einen Überdruck abzubauen. Der Unterdruck-Erzeuger baut einen Überdruck ab und reduziert die Gefahr, dass eine große Menge von Gas in die Umgebung austritt.

In einer Abwandlung steht der Unterdruck-Erzeuger in einer Fluidverbindung mit der Zuführleitung der Aufnahme-Anordnung. Die Filtereinheit und / oder mindestens ein Pufferspeicher sind flussabwärts von dem Unterdruck-Erzeuger angeordnet. Diese Ausgestaltung erleichtert es, einen pneumatischen Widerstand des Filters der Filtereinheit zu überwinden.

Möglich ist, dass das von dem Unterdruck-Erzeuger angesogene Gas durch die Zuführleitung in die oben beschriebene Fluidaufnahme und von dort weiter in die Abführleitung gesogen wird. Die Abführleitung und die Fluidaufnahme verhindern auch dann, dass eine große Menge des Gas-Bestandteils in die Umgebung austritt, wenn kein Filter in die Filteraufnahme eingesetzt ist. Diese Wirkung wird vor allem deshalb erzeugt, weil der Unterdruck, den der Unterdruck-Erzeuger bewirkt, Gas aus dem Pufferspeicher absaugt und dadurch verhindert, dass dieses Gas in die Umgebung austritt.

Eine weitere Wirkung des Unterdruck-Erzeugers ist die folgende: Der bewirkte Unterdruck überwindet einen pneumatischen Widerstand des Filters der Aufnahme-Anordnung und reduziert die Gefahr, dass ein Rückstau flussaufwärts von dem Filter auftritt.

In einer Fortbildung dieser Ausgestaltung steuert ein Steuergerät der Aufnahme-Anordnung ein Stellglied des Unterdruck-Erzeugers an und bewirkt, dass der Unterdruck-Erzeuger einen gewünschten Volumenfluss in der Abführleitung erzielt. Ein Sensor misst den tatsächlichen Volumenfluss, und das Steuergerät empfängt Messwerte von diesem Sensor für den tatsächlichen Volumenfluss und steuert das Stellglied so an, dass die Differenz zwischen dem gewünschten und dem tatsächlichen Volumenfluss verringert wird, regelt oder steuert also den Volumenfluss in der Abführleitung.

Bevorzugt umfasst der Unterdruck-Erzeuger eine Ansaugpumpe. Anstelle einer Ansaugpumpe kann auch ein sonstiger Unterdruck-Erzeuger in einer Fluidverbindung mit der Abführleitung stehen, beispielsweise eine Ejektoranlage.

Bevorzugt wird das Gas auf seinem Weg von der Zuführleitung zu der Abführleitung zwangsweise auf einem vorgegebenen Weg durch die Filtereinheit geleitet, das Gas wird also zwangsgeführt. Diese Zwangsführung bewirkt, dass das gesamte Gas durch den Filter fließt und kein Gas an dem Filter vorbeifließt. Dadurch wird die Gefahr verringert, dass unerwünschtes Gas in die Fluidaufnahme gelangt.

Bevorzugt wird außerdem das Gas durch den Filter hindurch zwangsgeführt. In einer Ausgestaltung unterteilt eine Wand den Filter in mindestens zwei Bereiche. Bevorzugt erstreckt diese Wand sich parallel zu oder schräg zu einer Richtung, in der Gas durch den Filter fließt. Der erste Bereich des Filters steht in einer Fluidverbindung mit der Zuführleitung, der zweite Bereich des Filters in einer Fluidverbindung mit der Abführleitung. Der Filter ist so ausgestaltet, dass das Gas durch die Zuführleitung fließt, dann durch den ersten Bereich, dann durch den zweiten Bereich und dann in die Abführleitung. Bei dieser Ausgestaltung wird das Gas zweimal gefiltert, nämlich einmal im ersten Bereich und einmal im zweiten Bereich. Auch dann, wenn ein Bereich sich zugesetzt hat und eine geringere oder gar keine filternde Wirkung erzielt, filtert in vielen Fällen noch der andere Bereich. Daher steigert diese Ausgestaltung die Zuverlässigkeit.

Bevorzugt umfasst die Aufnahme-Anordnung mindestens ein Rückschlag-Ventil. Dieses Rückschlag-Ventil ist beispielsweise in der Abführleitung oder in der Zuführleitung angeordnet. Es ermöglicht einen Fluss in Richtung von der Zuführleitung durch die Abführleitung hindurch und verhindert einen Fluss in die entgegengesetzte Richtung. Dieses Rückschlag-Ventil verhindert dann, wenn die Abführleitung mit einer Fluidaufnahme verbunden ist, dass Fluid aus der Fluidaufnahme durch die Abführleitung und die Zuführleitung hindurch in ein Gerät gelangt, welches mit der Zuführleitung verbunden ist. Ein solcher Rückfluss kann das Gerät beschädigen oder - im Falle eines medizinischen Geräts - einen mit dem Gerät verbunden Patienten den Patienten gefährden. Die Aufnahme-Anordnung kann auch mehrere Rückschlag-Ventile umfassen, beispielsweise eines in der Zuführleitung und eines in der Abführleitung.

In der Regel vermag der Filter der Filtereinheit nur eine maximale Menge des oder jedes herauszufilternden Gas-Bestandteils aufzunehmen und ist dann verbraucht. Nach Aufnahme dieser maximalen Menge muss die Filtereinheit ausgetauscht werden. Diese maximale Menge wird vorgegeben, beispielsweise von einem Hersteller der Filtereinheit, oder lässt sich vorab berechnen. Außerdem ist es bei manchen Ausgestaltungen des Filters möglich, dass der Filter aufgrund von Flüssigkeit aufquillt und aus diesem Grunde keine weitere Menge eines Gas-Bestandteils mehr aufzunehmen vermag und deshalb ausgetauscht werden muss.

In einer Ausgestaltung umfasst die Aufnahme-Anordnung einen Bestandteil-Sensor für den oder mindestens einen herauszufilternden Gas-Bestandteil, insbesondere einen Narkosemittel-Sensor. Dieser Sensor misst an einer Messposition flussabwärts von den Filter ein Maß für die Menge oder Konzentration des Gas-Bestandteils in dem vorbeifließenden Gas. Falls dieses Maß eine vorgegebene Bestandteil-Schranke übersteigt, so löst der Bestandteil-Sensor die Ausgabe eines Alarms aus. Eine Menge oder Konzentration oberhalb der Narkosemittel-Schranke zeigt an, dass der Filter nicht mehr ausreichend den Gas-Bestandteil aus dem durchströmenden Gasstrom herauszufiltern vermag. In einer Ausgestaltung wird dieser Alarm in einer von einem Menschen wahrnehmbaren Form an einer räumlich entfernten Stelle ausgegeben, beispielsweise in einer Zentrale. Möglich ist, dass die Aufnahme-Anordnung verschiedene Bestandteil-Sensoren für unterschiedliche Gas-Bestandteile aufweist.

In einer bevorzugten Ausgestaltung umfasst dieser Sensor einen Messfühler oder einen sonstigen Messwertaufnehmer, der im Inneren einer Filteraufnahme der Filtereinheit angeordnet ist. Falls der herauszufilternde Gas-Bestandteil schwerer als Luft ist und daher nach unten sinkt, befindet sich der Messwertaufnehmer bevorzugt unterhalb des Filters in der Filteraufnahme. In einer anderen Ausgestaltung ist dieser Sensor flussabwärts von der Filtereinheit angeordnet. Dieser Messwertaufnehmer ist in beiden Ausgestaltungen flussabwärts von dem Filter positioniert und die misst die Konzentration mindestens eines herauszufilternden Gas-Bestandteils in dem vorbeifließenden Gas. Falls der Filter keine ausreichende Menge des Gas-Bestandteils mehr aus dem durchfließenden Gasstrom herauszufiltern vermag, registriert der Sensor daher eine erhöhte Konzentration des herauszufilternden, aber tatsächlich nicht herausgefilterten Gas-Bestandteils.

Weiter oben wurde bereits eine mögliche Ausgestaltung der Filtereinheit mit der Filteraufnahme beschrieben, nämlich dass sich ein Überdruck-Ventil im Inneren der Filteraufnahme öffnet, wenn der Druck im Inneren der Filteraufnahme eine vorgegebene Überdruck-Schranke übersteigt. Bei geöffnetem Überdruck-Ventil wird in einer Realisierungsform wenigstens ein Teil des Gases, welches die Filteraufnahme erreicht, um den Filter in der Filteraufnahme herumgeleitet. In dieser Situation misst der Sensor mit dem Messwertaufnehmer im Inneren der Filteraufnahme eine erhöhte Konzentration des herauszufilternden Gas-Bestandteils. Der Sensor detektiert diese erhöhte Konzentration sowohl dann, wenn ein Überdruck aufgetreten und das Überdruck-Ventil sich geöffnet hat und das Gas daher in der Filteraufnahme um den Filter herumfließt, als auch dann, wenn das Überdruck-Ventil geschlossen ist und das Gas zwar durch den Filter fließt, der Filter aber den Gas-Bestandteil nicht mehr in ausreichendem Maße herauszufiltern vermag.

In einer Ausgestaltung wird ein Alarm ausgegeben, wenn mindestens eine der folgenden Situationen eingetreten ist:
- Der gerade beschriebene Sensor misst eine erhöhte Konzentration des Gas-Bestandteils im Inneren der Filteraufnahme.
- Ein Überdruck-Ventil an der Zuführleitung, der Filtereinheit, der Abführleitung oder an dem oder einem Pufferspeicher hat sich oder wurde geöffnet.
- Ein Unterdruck-Ventil an der Zuführleitung, der Filtereinheit, der Abführleitung oder an dem oder einem Pufferspeicher hat sich oder wurde geöffnet.

Diese Ausgestaltung stellt sicher, dass ein Alarm sowohl dann ausgegeben wird, wenn der Filter verbraucht ist, weil er eine hohe Menge des Gas-Bestandteils aufgenommen hat, als auch dann, wenn der Filter sich aus einem anderen Grund zugesetzt hat, beispielsweise verstopft oder falsch eingesetzt ist oder einer Verpackung nicht entfernt wurde. In vielen Fällen wird ein Alarm auch dann ausgegeben, wenn flussaufwärts von dem Filter eine Verstopfung aufgetreten ist.

In einer anderen Ausgestaltung umfasst die Aufnahme-Anordnung einen Bestandteil-Mengen-Ermittler. Dieser Bestandteil-Mengen-Ermittler ermittelt näherungsweise ein Maß für die Menge an dem vorgegebenen Gas-Bestandteil, insbesondere Narkosemittel, welche der Filter bislang aufgenommen hat. "Bislang" bedeutet bevorzugt: seit dem Zeitpunkt, an dem die Verwendung des aktuell benutzten Filters in der Filtereinheit begonnen worden ist. Der Bestandteil-Mengen-Ermittler kann im Inneren eines Geräts angeordnet sein, das mit der Zuführleitung verbunden ist, oder auf einem Rechner implementiert sein, beispielsweise auf einem Smartphone. Um diese Menge näherungsweise zu ermitteln, empfängt und verwendet der Bestandteil-Mengen-Ermittler in einer Ausgestaltung
- einerseits ein Signal für den zeitlich veränderlichen Volumenfluss von Gas durch die Zuführleitung zur Filtereinheit hin und
- andererseits ein Signal für die zeitlich veränderliche Konzentration von dem oder mindestens einem Gas-Bestandteil in der Zuführleitung.

In einer anderen Ausgestaltung empfängt der Bestandteil-Mengen-Ermittler ein Signal für die Einspeisung des Gas-Bestandteils in die Zuführleitung. Beispielsweise wird ein Maß für die Menge von Narkosemittel gemessen, welche einem Trägergas zugesetzt wird.

Der Bestandteil-Mengen-Ermittler empfängt kabelgebunden oder per Funkwellen diese Signale. Aus diesen beiden Signalen berechnet der Bestandteil-Mengen-Ermittler näherungsweise die bislang vom Filter aufgenommene Menge. Das Produkt aus dem Gas-Volumenfluss und der Konzentration liefert den Volumenfluss des Gas-Bestandteils. Bei einer zeitlich veränderlichen Konzentration wird über dieses Produkt numerisch aufintegriert.

Diese Ausgestaltung erspart die Notwendigkeit, einen Sensor flussabwärts von der Filtereinheit vorzusehen. Ein Gerät, welches sich mit der Zuführleitung verbinden lässt, umfasst häufig bereits einen Sensor für den Volumenfluss. Die Konzentration des Gas-Bestandteils lässt sich in manchen Fällen von einem weiteren Sensor dieses Geräts messen oder wird am Gerät eingestellt. Der Bestandteil-Mengen-Ermittler steht wenigstens zeitweise in einer drahtgebundenen oder drahtlosen (per Funkwellen) Datenverbindung mit diesen beiden Sensoren.

In einer Ausgestaltung wird in einem Datenspeicher die Information abgespeichert, welche Menge des Gas-Bestandteils der Filter der Filtereinheit bislang aufgenommen hat. Falls es möglich ist, dass der Filter unterschiedliche Gas-Bestandteile aufnimmt, so wird in einer Fortbildung dieser Ausgestaltung auf dem Datenträger für jeden möglichen Gas-Bestandteil, welches der Filter aufnehmen kann, jeweils eine Kennzeichnung dieses Gas-Bestandteils sowie eine Information, welche Menge dieses Gas-Bestandteils der Filter bislang aufgenommen hat, geschrieben.

Der Datenträger kann ein Bestandteil der Filtereinheit sein und beispielsweise an der Filtereinheit befestigt sein. Bevorzugt ist ein Datenspeicher, der zu der Filtereinheit gehört, als ein RFID-Chip ausgestaltet, er kann auch als ein Barcode ausgestaltet sein. Der Datenspeicher kann räumlich getrennt von der Filtereinheit angeordnet sein. Die bislang aufgenommene Menge kann von einem Sensor der Filtereinheit gemessen werden oder näherungsweise von dem gerade beschriebenen Bestandteil-Mengen-Ermittler ermittelt worden sein. Bevorzugt lässt sich diese Information über die abgespeicherte Menge aus der Entfernung ermitteln und auslesen und mit einer vorgegebenen Festlegung vergleichen, wobei die Festlegung vorgibt, welche maximale Menge der Filter aufzunehmen vermag. Diese Ausgestaltung ermöglicht es, rechtzeitig einen neuen Filter oder eine neue Filtereinheit mit einem Filter bereitzustellen. Außerdem erleichtert die Ausgestaltung mit dem Datenspeicher für die aufgenommenen Mengen es, den verbrauchten Filter aufzubereiten und / oder Materialien aus dem Filter zu gewinnen. Informationen, die im Datenspeicher abgespeichert sind, lassen sich verwenden, um die Aufbereitung an die vom Filter aufgenommenen Gas-Bestandteile und / oder Gas-Mengen anzupassen.

Möglich ist, dass derselbe Filter im Verlaufe eines Einsatzes unterschiedliche Gas-Bestandteile aufzunehmen vermag. Bevorzugt ist in dem Datenspeicher für jeden Gas-Bestandteil jeweils eine Information über die maximal mögliche Menge abgespeichert. Für jeden Gas-Bestandteil wird die Menge, welche der Filter bislang aufgenommen hat, mit der maximal möglichen Menge, die im Datenspeicher abgespeichert ist, verglichen.

Der oder ein Gas-Bestandteil, welchen der Filter aus einem durchfließenden Gas herauszufiltern vermag, ist in einer Ausgestaltung ein Narkosemittel. In einer anderen Ausgestaltung ist der Bestandteil ein Gas, welches brennbar und / oder für einen Menschen schädlich ist. Der Gas-Bestandteil kann auch eine Menge von Partikeln sein, die im Gas enthalten sind oder sein können, beispielsweise Staub oder Mikroben oder Viren oder sonstige Krankheitserreger, die in der Luft sein können. Möglich ist, dass der Filter mehrere Gas-Bestandteile, beispielsweise mehrere unterschiedliche Narkosemittel und / oder mindestens ein Narkosemittel sowie Partikel, aus einem durchfließenden Gas herauszufiltern vermag.

Die Erfindung betrifft weiterhin eine medizinische Anordnung, welche mindestens ein medizinisches Gerät sowie eine erfindungsgemäße Aufnahme-Anordnung umfasst. Das oder ein medizinisches Gerät ist insbesondere ein Anästhesiegerät. Die Zuführleitung der Aufnahme-Anordnung steht wenigstens zeitweise in einer Fluidverbindung mit dem oder einem medizinischen Gerät. Bevorzugt lässt diese Fluidverbindung sich herstellen und später wieder lösen. Dadurch lässt dieselbe Aufnahme-Anordnung sich nacheinander mit unterschiedlichen medizinischen Geräten verbinden, und dasselbe medizinische Gerät lässt sich nacheinander mit unterschiedlichen Aufnahme-Anordnungen verbinden.

In einer Ausgestaltung ist die erfindungsgemäße Aufnahme-Anordnung räumlich von dem medizinischen Gerät getrennt. Die Zuführleitung der Aufnahme-Anordnung steht in einer Fluidverbindung mit dem medizinischen Gerät.

Bevorzugt lässt sich die Aufnahme-Anordnung nacheinander mit unterschiedlichen medizinischen Geräten verbinden. In einer anderen Ausgestaltung ist die erfindungsgemäße Aufnahme-Anordnung ein Bestandteil des medizinischen Geräts und ist im Inneren des medizinischen Geräts angeordnet. Bevorzugt stellt die Abführleitung eine Koppelstelle für Fluid bereit, welches das medizinische Gerät ausgestoßen hat und / oder welches abgesogen wird.

In einer Ausgestaltung umfasst die medizinische Anordnung ein erstes und ein zweites medizinisches Gerät. Wahlweise oder auch gleichzeitig lässt sich eine Fluidverbindung zwischen der Zuführleitung und dem ersten medizinischen Gerät und / oder der Zuführleitung und dem zweiten medizinischen Gerät herstellen. Diese Ausgestaltung erspart die Notwendigkeit, für beide medizinischen Geräte jeweils eine eigene Aufnahme-Anordnung mit einer Filtereinheit bereitzustellen.

Falls die beiden medizinischen Geräte gleichzeitig mit der Aufnahme-Anordnung verbunden sind, so variiert in manchen Fällen der Volumenfluss und / oder der Druck in der Zuführleitung weniger stark, als wenn die Aufnahme-Anordnung nur mit einem einzigen medizinischen Gerät verbunden wäre.

Möglich ist auch, dass die erfindungsgemäße Aufnahme-Anordnung wahlweise oder auch gleichzeitig mit drei oder noch mehr medizinischen Geräten in jeweils einer Fluidverbindung steht.

Erfindungsgemäß lässt sich die Abführleitung mit einer Fluidaufnahme verbinden, welche in einer Ausgestaltung eine stationäre Fluidaufnahme ist. Diese stationäre Fluidaufnahme gehört bevorzugt zu einem medizinischen Infrastruktursystem, insbesondere zu einem stationären Infrastruktursystem in einem Krankenhaus. Dieses medizinische Infrastruktursystem vermag auch die benötigten Gase und sonstigen Fluide für das medizinische Gerät bereitzustellen. Optional steht ein Unterdruck-Erzeuger, beispielsweise eine Absaugpumpe, der Fluidaufnahme wenigstens zeitweise in einer Fluidverbindung mit der Abführleitung und vermag Gas anzusaugen.

Die Erfindung betrifft weiterhin ein medizinisches System, wobei das medizinische System eine medizinische Anordnung mit mindestens einem medizinischen Gerät und mit einer erfindungsgemäßen Aufnahme-Anordnung sowie eine Fluidaufnahme umfasst. Die Abführleitung der Aufnahme-Anordnung steht wenigstens zeitweise in einer Fluidverbindung mit der Fluidaufnahme.

In einer Fortbildung dieser Ausgestaltung umfasst das medizinische System mindestens eine weitere Fluidaufnahme. Besonders bevorzugt sind die beiden Fluidaufnahmen des medizinischen Systems stationäre Fluidaufnahmen. Wahlweise oder gleichzeitig lässt sich eine Fluidverbindung zwischen der Fluidaufnahme und der Abführleitung oder der weiteren Fluidaufnahme und der Abführleitung herstellen. Möglich ist auch, dass das medizinische System drei oder noch mehr Fluidaufnahme umfasst, wobei die Abführleitung wahlweise oder gleichzeitig in jeweils einer Fluidverbindung mit den wenigstens drei Fluidaufnahmen steht.

Die Ausgestaltung mit mindestens zwei Fluidaufnahmen ermöglicht es, eine besonders große Menge von Gas aus dem medizinischen Gerät aufzunehmen. Möglich ist auch, ein Gas, welches das medizinische Gerät ausstößt oder welches abgesogen wird, wahlweise in die eine oder in die andere Fluidaufnahme zu lenken, was insbesondere dann manchmal von Vorteil ist, wenn das medizinische Gerät nacheinander unterschiedliche Gase ausstößt, beispielsweise Gase mit unterschiedlichen Narkosemitteln.

In einer anderen Anwendung wird die Aufnahme-Anordnung eingesetzt, um die Luft in einem Bereich, beispielsweise in einem geschlossenen Raum in einem Gebäude oder in einem Fahrzeug, von Mikroben, sonstigen Krankheitserregern oder Partikeln zu reinigen. Bevorzugt umfasst die Aufnahme-Anordnung eine Förderereinheit oder steht mit einer Förderereinheit in einer Fluidverbindung. Diese Förderereinheit fördert die zu reinigende Luft aus dem Raum durch die Zuführleitung, die Filtereinheit und die Abführleitung hindurch zurück in den Raum.

Im Folgenden wird die Erfindung anhand eines Ausführungsbeispiels beschrieben. Hierbei zeigt
- Figur 1: eine medizinische Anordnung mit einem Anästhesiegerät und einer erfindungsgemäßen Aufnahme-Anordnung;
- Figur 2: eine Ausgestaltung mit einem pneumatisch wirkenden Pufferspeicher im Inneren der Filteraufnahme und um den Filter herum, wobei eine Kartusche mit einem Filter eingesetzt ist;
- Figur 3: die Ausgestaltung von Figur 2, wobei die Kartusche entnommen ist;
- Figur 4: eine Abwandlung der Ausgestaltung von Figur 2, wobei das Gas durch die Kartusche zwangsgeführt wird und die Kartusche eingesetzt ist;
- Figur 5: eine Abwandlung der Ausgestaltung von Figur 4, wobei in der Zuführleitung ein Überdruck-Ventil angeordnet ist, welches bei Überdruck Gas in die Umgebung ablässt;
- Figur 6: eine Abwandlung der Ausgestaltung von Figur 5, wobei das geöffnete Überdruck-Ventil Gas nicht in die Umgebung ablässt, sondern in die Abführleitung;
- Figur 7: eine Abwandlung der Ausgestaltung von Figur 6, wobei der Messfühler der Filtereinheit auch eine Eigenschaft des vom Überdruck-Ventil abgelassenen Gases misst;
- Figur 8: verschiedene mögliche Realisierungsformen des Überdruck-Ventils von Figur 5 bis Figur 7;
- Figur 9: eine Querschnittsdarstellung der Ausgestaltung von Figur 4 bis Figur 7 in der Ebene A - A von Figur 4 bis Figur 7;
- Figur 10: die Ausgestaltung von Figur 4, wobei die Kartusche entnommen ist;
- Figur 11: eine Ausgestaltung mit einem Unterdruck-Ventil am Topf;
- Figur 12: verschiedene mögliche Realisierungsformen des Unterdruck-Ventils von Figur 11;
- Figur 13: verschiedene mögliche Realisierungsformen eines elastischen Pufferspeichers in der Zuführleitung;
- Figur 14: eine Ausgestaltung mit einem mechanisch wirkenden Pufferspeicher im Inneren der Filteraufnahme und zusätzlich unterhalb des Filters, wobei die Kartusche eingesetzt ist;
- Figur 15: die Ausgestaltung von Figur 14, wobei die Kartusche entnommen ist;
- Figur 16: eine Abwandlung der Ausgestaltung von Figur 14 und Figur 15, wobei das Gas durch die Kartusche zwangsgeführt wird und die Kartusche eingesetzt ist;
- Figur 17: die Ausgestaltung von Figur 16, wobei die Kartusche entnommen ist;
- Figur 18: eine weitere Abwandlung der Ausgestaltung von Figur 14 und Figur 15, wobei ein eingesetzter Filter einen beweglichen Schieber in einer Park-Position hält;
- Figur 19: die Ausgestaltung von Figur 18, wobei der Filter entnommen ist und der Schieber in einer Pufferspeicher-Position ist;
- Figur 20: eine Ausgestaltung mit einem separaten Pufferspeicher unterhalb der Filteraufnahme, wobei die Kartusche eingesetzt ist;
- Figur 21: die Ausgestaltung von Figur 20, wobei die Kartusche entnommen ist;
- Figur 22: eine Kombination von mehreren Ausgestaltungen des Pufferspeichers, wobei die Kartusche eingesetzt ist;
- Figur 23: die Kombination von Figur 22, wobei die Kartusche entnommen ist;
- Figur 24: eine Ausgestaltung mit einem separaten Pufferspeicher flussabwärts von der Filteraufnahme;
- Figur 25: eine Abwandlung der Anordnung nach Figur 1, die zusätzlich einen Narkosemittel-Mengen-Ermittler und einen Datenspeicher an der Filtereinheit umfasst;
- Figur 26: eine Filtereinheit mit zwei Filtern, die in einer Revolveraufnahme eingesetzt sind;
- Figur 27: eine Ausgestaltung, bei der zwei medizinische Geräte mit derselben erfindungsgemäßen Aufnahme-Anordnung verbunden sind;
- Figur 28: eine Ausgestaltung, bei der zwei stationäre Gasaufnahmen mit derselben erfindungsgemäßen Aufnahme-Anordnung verbunden sind.

Im Ausführungsbeispiel wird die Erfindung verwendet, um einen Patienten künstlich zu beatmen und ihm hierbei mindestens ein Narkosemittel zuzuführen. Der Patient befindet sich in einem umschlossenen Raum, während er künstlich beatmet wird, beispielsweise in einem Saal eines Krankenhauses oder an Bord eines Fahrzeugs.

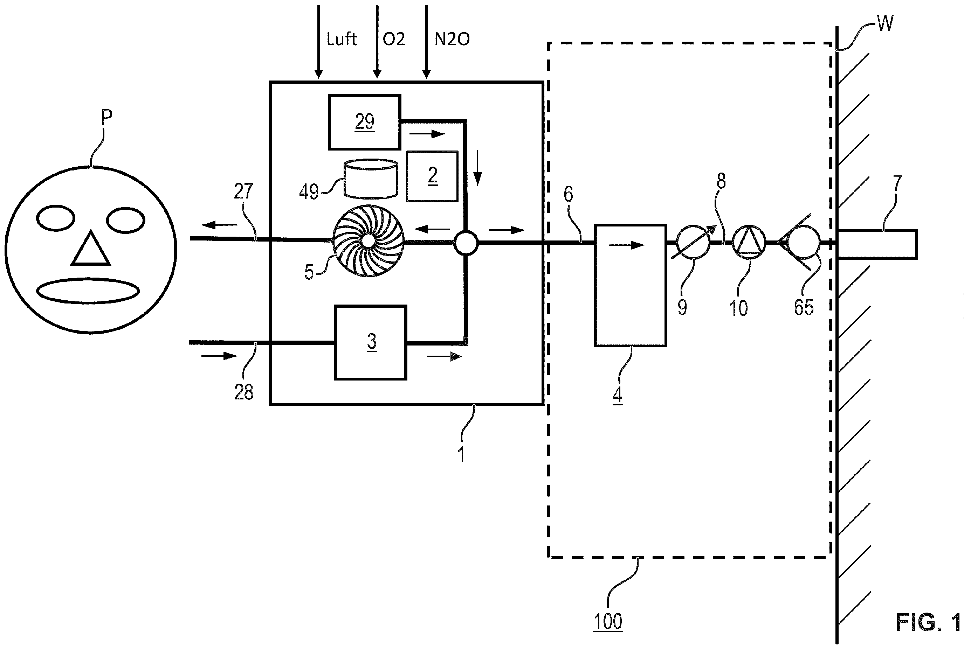

Figur 1 zeigt eine medizinische Anordnung, um diesen Patienten P zu narkotisieren und ihn künstlich zu beatmen. Der Patient P wird über eine Inspirations-Gasleitung 27 mit Atemluft versorgt. Diese Atemluft ist mit mindestens einem Narkosemittel versetzt, um den Patienten P narkotisiert zu halten. Die vom Patienten P ausgeatmete Atemluft enthält Kohlendioxid (CO2) und wird über eine Exspirations-Gasleitung 28 abgesogen. Beide Gasleitungen 27, 28 sind mit einem medizinischen Gerät in Form eines Anästhesiegeräts 1 verbunden, welches einen Beatmungskreislauf aufrechterhält, um den Patienten P mit Atemluft und Narkosemittel zu versorgen und um ausgeatmete Luft abzusaugen und aufzunehmen.

Das Anästhesiegerät 1 wird von einer Krankenhaus-Infrastruktur mit unter Druck stehender Atemluft, reinem Sauerstoff (O2) und Lachgas (N2O) versorgt. Das Anästhesiegerät 1 umfasst im Ausführungsbeispiel folgende Bestandteile:
- einen Mischer 29, der aus zwei der drei zugeführten Gase Luft, O2 und N2O ein Gemisch erzeugt, welches als Trägergas für Narkosemittel verwendet wird, wobei der Mischer 29 so wie in DE 10 2008 057 180 B3 beschrieben aufgebaut sein kann,
- eine Ventilatoreinheit 5, welche Gas im Beatmungskreislauf bewegt und dadurch den Beatmungskreislauf aufrechterhält,
- einen Narkosemittelverdampfer 2, der einen Tank 49 für flüssiges Narkosemittel umfasst, und
- eine geräteinterne Filtereinheit 3 mit einem Kalkfilter, welche aus der vom Patienten P ausgeatmeten und über die Exspirations-Gasleitung 28 abgeleiteten Atemluft CO2 herausfiltert.

Der Narkosemittelverdampfer 2 setzt dem Trägergas Narkosemittel aus dem Tank 49 zu. Beispielsweise verdampft der Narkosemittelverdampfer 2 das Narkosemittel im Tank 49 und / oder spritzt es in das Trägergas ein.

Das Anästhesiegerät 1 führt dem Beatmungskreislauf Gas zu. Die Filtereinheit 3 entzieht dem Beatmungskreislauf Gas, insbesondere CO2. Im Saldo wird dem Beatmungskreislauf dadurch mehr Gas zugeführt als entnommen. Daher ist es erforderlich, überschüssiges Gas aus dem Beatmungskreislauf abzuführen. Dieses überschüssige Gas, im Folgenden "Überschussgas" genannt, wird mithilfe zweier Gasleitungen 6 und 8 aus dem Anästhesiegerät 1 entfernt, nämlich mittels einer Zuführleitung 6 und einer Abführleitung 8. Zum einen stößt die Ventilatoreinheit 5 Gas aus und fördert das ausgestoßene Überschussgas in die Zuführleitung 6 hinein, wobei der Volumenfluss des ausgestoßenen Überschussgases mit der Zeit variiert und der zeitliche Verlauf des Volumenstroms beispielsweise die Form einer Halbsinuskurve aufweist. Zum anderen wird das ausgestoßene Überschussgas durch die Abführleitung 8 hindurch geleitet und in einer Ausgestaltung angesogen.

Im Ausführungsbeispiel führt die Abführleitung 8 in eine stationäre Fluidaufnahme 7, die in eine Wand W eingelassen ist. Die Fluidaufnahme 7 ist bevorzugt ein Bestandteil einer stationären Infrastruktur eines Krankenhauses, welche von verschiedenen medizinischen Geräten ausgestoßenes Gas aufnimmt und weiterleitet.

Bevorzugt ist in die Abführleitung 8 ein Rückschlag-Ventil 65 eingelassen, welches Gas aus der Abführleitung 8 in Richtung der Fluidaufnahme 7 passieren lässt, jedoch verhindert, dass Gas aus der Fluidaufnahme 7 rückwärts durch die Abführleitung 8 strömt. In einer alternativen Realisierungsform ist das Rückschlag-Ventil 65 in die Fluidaufnahme 7 integriert. Möglich ist auch, dass das oder ein Rückschlag-Ventil 65 in der Zuführleitung 6 oder in der Verbindung zwischen der Zuführleitung 6 und dem Anästhesiegerät 1 positioniert ist. In allen möglichen Positionierungen ermöglicht das Rückschlag-Ventil 65, dass das Anästhesiegerät 1 Gas ausstößt, verhindert aber, dass ein Rückfluss auf das Anästhesiegerät 1 und damit auf den Patienten P einwirken kann.

Die medizinische Anordnung umfasst weiterhin eine erfindungsgemäße Aufnahme-Anordnung 100 mit einer Filtereinheit 4 sowie mit den Leitungen 6 und 8. Die Filtereinheit 4 umfasst einen Filter 11, 20 sowie einen Topf 13, der als eine Filteraufnahme fungiert. Diese Bestandteile werden nachfolgend detailliert beschrieben.

In der Ausgestaltung, die in Figur 1 gezeigt wird, befindet die Zuführleitung 6 sich teilweise außerhalb des Anästhesiegeräts 1. Möglich ist auch, dass die Zuführleitung 6 sich vollständig innerhalb des Anästhesiegeräts 1 befindet und die Filtereinheit 4 mit dem Anästhesiegerät 1 lösbar gekoppelt ist. Möglich ist auch, dass die erfindungsgemäße Aufnahme-Anordnung 100 sich vollständig innerhalb des Anästhesiegeräts 1 befindet.

In einer Ausgestaltung veranlasst ausschließlich das Anästhesiegerät 1, dass Überschussgas durch die Leitungen 6 und 8 ausgestoßen wird.

In einer anderen Ausgestaltung saugt eine Ansaugpumpe 10, die in einer Fluidverbindung mit der Abführleitung 8 steht, Überschussgas aus der Aufnahme-Anordnung 100 an. Diese Ansaugpumpe 10 lässt sich bevorzugt einschalten und ausschalten und erzeugt einen Volumenstrom von der Filtereinheit 4 weg und durch die Abführleitung 8 hindurch. In der gezeigten Realisierungsform ist die Ansaugpumpe 10 vor der Wand W positioniert und steht in einer Fluidverbindung mit der Abführleitung 8. In einer anderen Realisierungsform befindet die Ansaugpumpe 10 sich in oder hinter der Wand W und steht in einer Fluidverbindung mit der Fluidaufnahme 7 und über die Fluidaufnahme 7 indirekt auch in einer Fluidverbindung mit der Abführleitung 8.

In einer Ausgestaltung dieser Ansaugung ist der Volumenfluss, also das Volumen, das pro Zeiteinheit durch die Abführleitung 8 hindurchfließt, zeitlich konstant oder folgt einem anderen vorgegebenen zeitlichen Verlauf. Ein optionaler Volumenstrom-Sensor 9 misst den Volumenstrom, also das Volumen pro Zeiteinheit, in der Abführleitung 8. In einer Ausgestaltung wird die Ansaugpumpe 10 abhängig von Messwerten des Volumenstrom-Sensors 9 angesteuert, beispielsweise von einem Steuergerät der Aufnahme-Anordnung 100, damit der tatsächliche Volumenfluss durch die Abführleitung 8 gleich einem gewünschten vorgegebenen Volumenfluss ist. Somit wird der tatsächliche Volumenstrom in der Abführleitung 8 automatisch geregelt.

In einer anderen Ausgestaltung misst ein nicht gezeigter Druck-Sensor den Druck an einem Messpunkt in der oder flussaufwärts von der Abführleitung 8, bevorzugt auch flussaufwärts von der nachfolgend beschriebenen Filtereinheit 4. Sobald der gemessene Druck eine vorgegebene Druck-Schranke übersteigt, wird die Ansaugpumpe 10 aktiviert und saugt Überschussgas in die Abführleitung 8. Die Ansaugpumpe 10 bleibt aktiviert, bis der gemessene Druck wieder unter die Druck-Schranke sinkt.

Das aus dem Anästhesiegerät 1 ausgestoßene und abgesogene Überschussgas kann O2, N2O und / oder Narkosemittel enthalten. Daher wird dieses Überschussgas zu einer erfindungsgemäßen Aufnahme-Anordnung 100 geleitet. Diese Aufnahme-Anordnung 100 steht über die Zuführleitung 6 in einer fluiddichten Fluidverbindung mit dem Anästhesiegerät 1 und über die Abführleitung 8 in einer fluiddichten Fluidverbindung mit einer stationären Fluidaufnahme in Form einer Gasaufnahme 7. Die Gasaufnahme 7 ist an einer Wand W und teils hinter dieser Wand W angeordnet und gehört zu der Krankenhaus-Infrastruktur. Bevorzugt lassen sich sowohl die Verbindung zwischen dem Anästhesiegerät 1 und der Zuführleitung 6 als auch die Verbindung zwischen der Abführleitung 8 und der Gasaufnahme 7 lösen und später wieder herstellen.

Das Überschussgas wird durch eine Filtereinheit 4 geleitet. Die Zuführleitung 6 führt vom Anästhesiegerät 1 zu dieser Filtereinheit 4. Die Abführleitung 8 führt von der Filtereinheit 4 zu der Gasaufnahme 7. Die Gasaufnahme 7 nimmt das gefilterte Gas auf, welches durch die Abführleitung 8 fließt. Der Filter 11, 20 der Filtereinheit 4 filtert aus dem Überschussgas unerwünschte Bestandteile heraus, insbesondere das oder jedes Narkosemittel. Bevorzugt ist die Aufnahme-Anordnung 100 so ausgestaltet, dass das Überschussgas zwangsläufig durch die Filtereinheit 4 hindurch geführt wird und diese nicht umgehen kann.

Weil die Gasaufnahme 7 das gefilterte Überschussgas aufnimmt, nachdem aus dem Überschussgas unerwünschte Bestandteile herausgefiltert worden sind, wird in vielen Fällen verhindert, dass eine relevante Menge von unerwünschten Gas-Bestandteilen, beispielsweise von O2, N2O und / oder Narkosemitteln, in die Krankenhaus-Infrastruktur oder in einen Raum des Krankenhauses gerät oder in die Umgebung des Krankenhauses ausgestoßen wird. Das erste Ereignis (Narkosemittel in der Krankenhaus-Infrastruktur oder in einem Raum) ist unerwünscht, weil ausgetretenes Gas, insbesondere ein Narkosemittel, Personen in dem Raum gesundheitlich beeinträchtigen könnte und O2 zusätzlich Geräte beschädigen könnte. Das zweite Ereignis ist unerwünscht, weil die Umweltemissionen gering gehalten werden sollen.

In den nachfolgend gezeigten Ausführungsformen verbindet die Zuführleitung 6 die Filtereinheit 4 mit dem medizinischen Gerät 1. Möglich ist auch, dass die Filtereinheit 4 sich direkt lösbar mit dem medizinischen Gerät 1 koppeln lässt, beispielsweise durch zwei korrespondierende Kopplungselemente. Diese Ausgestaltung spart eine Zuführleitung 6 außerhalb des medizinischen Geräts 1 ein.

In den nachfolgend beschriebenen Ausgestaltungen umfasst die Filtereinheit 4 eine Filteraufnahme in Form eines Topfs 13, der rotationssymmetrisch zu einer Mittelachse ist, wobei diese Mittelachse im Einsatz vertikal angeordnet ist. Ein annähernd zylinderförmiger Filter lässt sich von oben in diesem Topf 13 einsetzen und wieder aus dem Topf 13 entnehmen. Optional lässt sich ein Deckel 42 von oben auf den Topf 13 aufsetzen und wieder abnehmen. Zur Filtereinheit 4 gehört weiterhin der Filter 11, 20.

In einer Ausgestaltung umfasst der Topf 13 einen Filter-Sensor 47, der feststellt, ob ein Filter 11, 20 in den Topf 13 eingesetzt ist oder nicht. Beispielsweise schließt ein Kontaktschalter des Filter-Sensors 47 einen elektrischen Stromkreis, wenn ein Filter 11, 20 eingesetzt ist, oder unterbricht bei eingesetztem Filter 11, 20 einen solchen Stromkreis. Oder der eingesetzte Filter 11, 20 unterbricht eine Lichtschranke. Diese Ausgestaltung ermöglicht es, einen Alarm zu erzeugen und in einer von einem Menschen wahrnehmbaren Form auszugeben, wenn für eine Zeitspanne, die größer als eine vorgegebene Zeitspannen-Schranke ist, kein Filter 11, 20 in den Topf 13 eingesetzt ist. In einer Fortbildung dieser Ausgestaltung wird diese Warnung nur dann ausgegeben, wenn zusätzlich festgestellt wird, dass ein Gasgemisch durch die Zuführleitung 6 und / oder in die Filtereinheit 4 fließt.

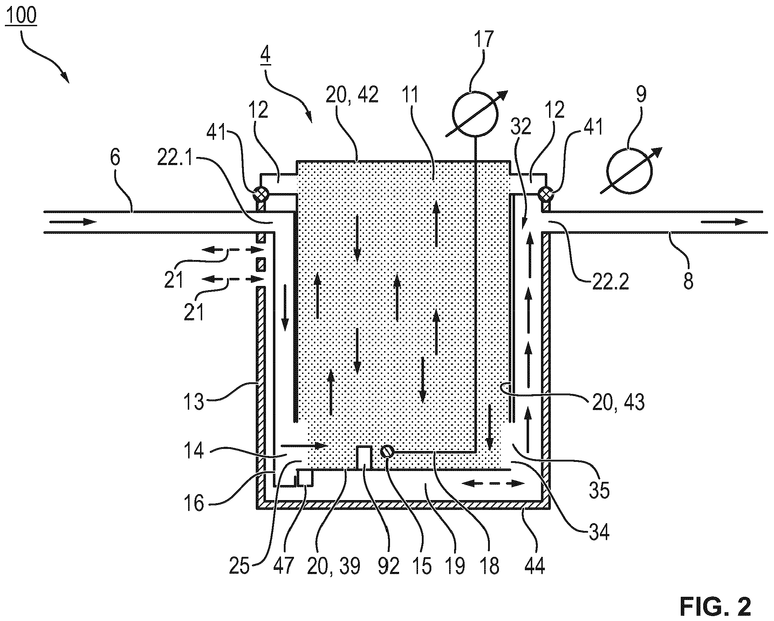

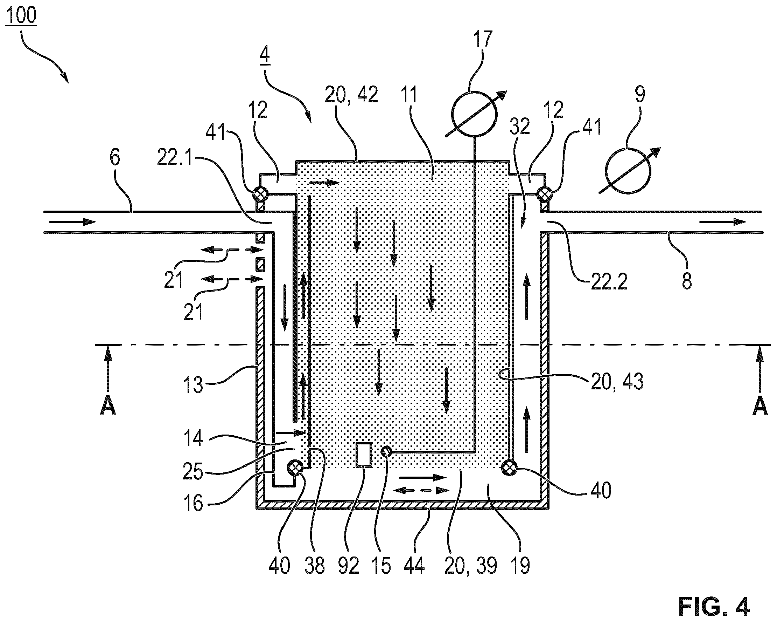

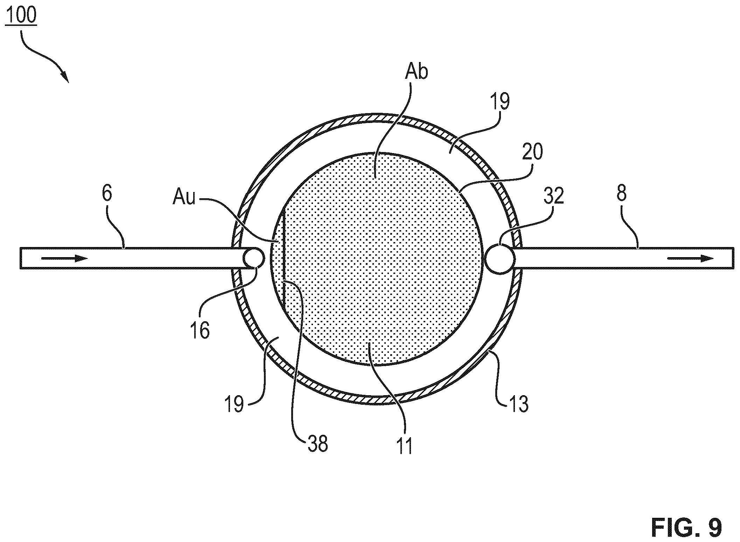

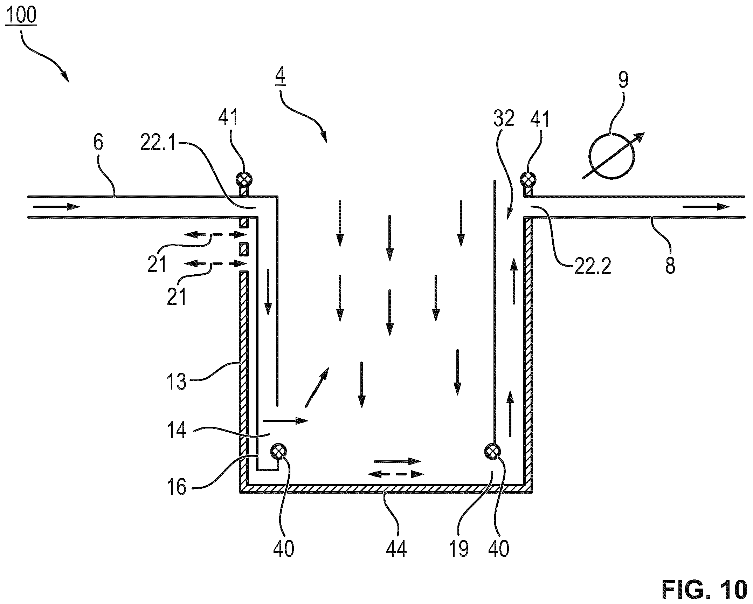

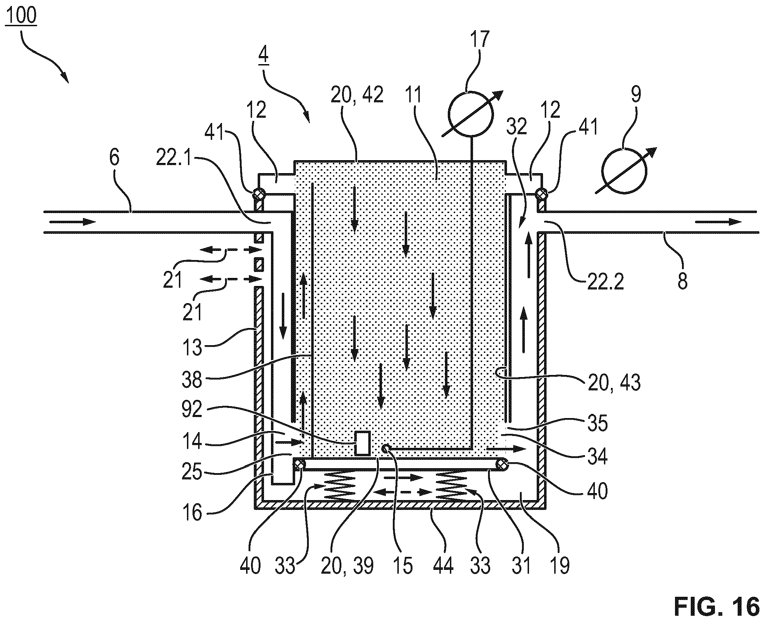

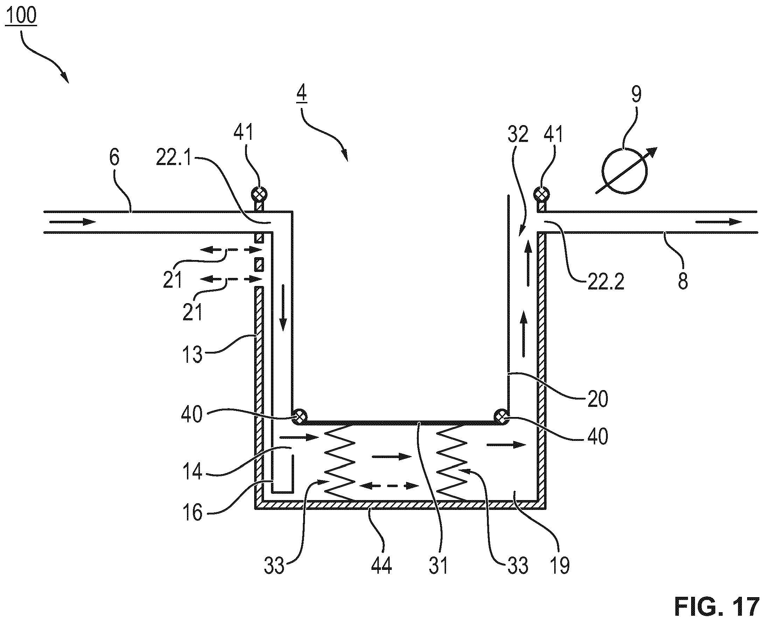

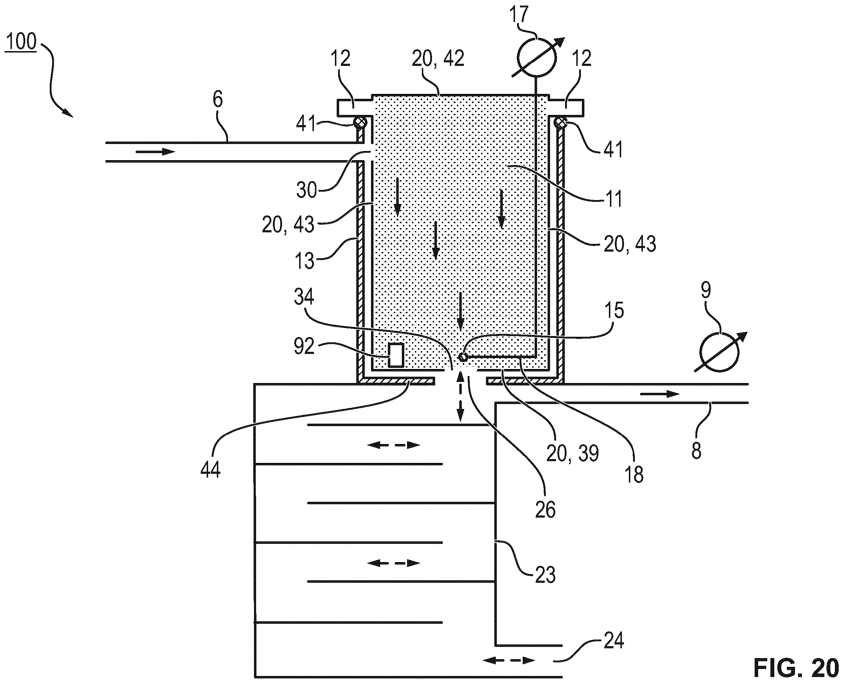

Figur 2 bis Figur 17 zeigen mehrere mögliche Ausgestaltungen der Aufnahme-Anordnung 100, wobei ein nachfolgend beschriebener Pufferspeicher zwischen einer Kartusche in der Filtereinheit 4 und dem Topf 13 angeordnet ist. Gleiche Bezugszeichen haben gleiche Bedeutung. Figur 2 bis Figur 7, Figur 10, Figur 11 und Figur 14 bis Figur 17 zeigen die Filtereinheit 4 in einer Seitenansicht, wobei die Mittelachse der Filtereinheit 4 in der Zeichenebene liegt. Figur 9 zeigt die Filtereinheit 4 in einer Querschnittsdarstellung in der Ebene A - A von Figur 4 bis Figur 7, wobei die Mittelachse der zylinderförmigen Filtereinheit 4 senkrecht auf der Zeichenebene von Figur 9 steht. Figur 20 bis Figur 24 zeigen unterschiedliche Ausgestaltungen, wobei der Pufferspeicher außerhalb des Topfs 13 angeordnet ist und wobei die Filtereinheit 4 ebenfalls in einer Seitenansicht gezeigt wird. Möglich ist auch, dass die Aufnahme-Anordnung 100 sowohl einen Pufferspeicher im Topf 13 als auch einen Pufferspeicher außerhalb des Topfs 13 aufweist.

In den gezeigten Ausgestaltungen umfasst die Filtereinheit 4
- den eigentlichen Filter 11 für Narkosemittel, der im Folgenden als "Filterelement" oder auch "Narkosemittelfilter" bezeichnet wird,
- eine zylinderförmige Kartusche 20, welche den Narkosemittelfilter 11 umgibt, bevorzugt - bis auf nachfolgend beschriebene Öffnungen - vollständig und gasdicht,
- einen umlaufenden Vorsprung 12 oben an der Kartusche 20,
- einen zylinderförmigen Topf 13, der die Kartusche 20 mit dem Narkosemittelfilter 11 aufnimmt und einen Boden 44 sowie eine Mantelfläche aufweist,
- eine Topf-Zuführleitung 16, welche fluiddicht mit der Zuführleitung 6 verbunden ist, zugeführtes Überschussgas von einer Öffnung 22.1 zum Boden 44 des Topfs 13 hin leitet und in einer Austrittsöffnung 14 endet,
- eine Topf-Abführleitung 32, welche fluiddicht mit der Abführleitung 8 verbunden ist, aus dem Narkosemittelfilter 11 austretendes Überschussgas zur Abführleitung 8 hin leitet, in einer Eintrittsöffnung 35 oder in Höhe des Bodens 39 der Kartusche 20 beginnt und zu einer Öffnung 22.2 führt,
- einen Messfühler 15, der ein Maß für die Menge des Narkosemittels unterhalb oder in einem unteren Bereich des Narkosemittelfilters 11 misst,
- eine Verbrauchsanzeige 17, welche insbesondere anzeigt, wenn die Kartusche 20 mit dem Narkosemittelfilter 11 erneuert werden muss, und
- eine Messleitung 18, die vom Messfühler 15 zur Verbrauchsanzeige 17 führt.

Im Folgenden wird das Bauteil, welches das Filterelement 11 und die Kartusche 20 umfasst, abkürzend als "Filter 11, 20" oder auch als "Narkosemittelfilter 11, 20" bezeichnet. In einer Ausgestaltung bilden das Filterelement 11 und die Kartusche 20 ein einziges Bauteil, das nur als Ganzes ausgetauscht werden kann. Möglich ist auch, dass das Filterelement 11 sich in die Kartusche 20 einsetzen und wieder aus der Kartusche 20 entnehmen lässt, sodass dieselbe Kartusche 20 sich nacheinander für verschiedene Filterelemente 11 verwenden lässt.

Das Filterelement 11 für Narkosemittel hat bevorzugt die Form eines Zylinders und ist bevorzugt als ein starrer Körper ausgestaltet, der besonders bevorzugt aus Aktivkohle besteht oder Aktivkohle enthält. Das Filterelement 11 kann auch Zeolithe enthalten. Die Mittelachse dieses Zylinders ist vertikal angeordnet, wenn der Filter 11, 20 in den Topf 13 eingesetzt ist, und liegt in den Zeichenebenen von Figur 2 bis Figur 7, Figur 10, Figur 11, Figur 14 bis Figur 24 und Figur 26 und steht senkrecht auf der Zeichenebene von Figur 9. Besonders bevorzugt ist das Filterelement 11 als ein Monolith aus Aktivkohle mit mehreren parallelen Kanälen ausgestaltet. Ein derartig ausgestaltetes Filterelement 11 übt einen geringeren pneumatischen Widerstand aus als andere Filter mit vergleichbarer Filterleistung und ist leicht. Jedoch ist ein solches Filterelement 11 empfindlich gegen mechanische Einwirkungen und kann insbesondere relativ leicht zerbrechen. Ein solches Filterelement aus Aktivkohle und mit Kanälen wird beispielsweise in DE 10 2015 012 410 A1 beschrieben.

Die Kartusche 20 schützt das Filterelement 11 bis zu einem gewissen Grad vor mechanischer Beschädigung. Die Kartusche 20 hat bevorzugt ebenfalls die Form eines Zylinders, dessen Mittelachse bevorzugt mit der Mittelachse des Filterelements 11 übereinstimmt, und weist einen Deckel 42, einen Boden 39 und eine Mantelfläche 43, welche sich zwischen dem Deckel 42 und dem Boden 39 erstreckt, auf. Der Deckel 42 und die Mantelfläche 43 sind für Fluide undurchlässig - bis auf optionale Öffnungen, die weiter unten beschrieben werden. Der Boden 39 ist in einer Ausgestaltung ebenfalls für Fluid undurchlässig und in einer anderen Ausgestaltung für Fluid durchlässig. Bevorzugt ist die Kartusche 20 aus einem harten Kunststoff oder aus Metall gefertigt. Bevorzugt ist außen an der Kartusche 20 mindestens ein Führungselement (nicht gezeigt) angeordnet, welches mit einem korrespondierenden Führungselement innen an der Wand des Topfs 13 korrespondiert. Diese beiden korrespondierenden Führungselemente stellen sicher, dass ein Filter 11, 20 korrekt (in einer korrekten Drehposition) in den Topf 13 eingesetzt wird.

In einer Ausgestaltung umfasst der Filter 11, 20 einen Datenspeicher 92, der bevorzugt außen an der Kartusche 20 befestigt ist und beispielsweise als RFID-Chip oder als Barcode ausgestaltet ist. Auf diesem Datenspeicher 92 sind Informationen über den Filter 11, 20 abgespeichert. Ein nicht gezeigtes Lesegerät der Aufnahme-Anordnung 100 vermag diese Informationen auszulesen. In einer bevorzugten Ausgestaltung ist dieses Lesegerät ein Lese- und Schreibgerät, d. h. es vermag auch Informationen auf den Datenspeicher 92 zu schreiben.

Bevorzugt ist in dem Datenspeicher 92 eine eindeutige Kennung des Filters 11, 20 abgespeichert. Diese Kennung unterscheidet diesen Filter 11, 20 von allen anderen Filtern, die in dem Krankenhaus verwendet werden. In einer Realisierungsform liest das Lesegerät diese eindeutige Kennung ein, und in einem zentralen Datenspeicher (nicht gezeigt) wird abgespeichert, welcher Filter 11, 20 aktuell mit welchem Anästhesiegerät oder sonstigem medizinischen Gerät des Krankenhauses in einer Fluidverbindung steht. Diese Informationen werden bei Bedarf aktualisiert.

In der Regel soll oder darf der Filter 11, 20 nur in einer einzigen Aufnahme-Anordnung 100 verwendet werden, bis er verbraucht ist, und darf danach nicht - oder erst nach einer Aufbereitung - erneut verwendet werden. Eine unerwünschte Wiederverwendung lässt sich beispielsweise durch Auswertung des gerade beschriebenen Datenspeichers 92 verhindern. In einer bevorzugten Realisierungsform enthält hingegen der Datenspeicher 92 die Information, beispielsweise in Form eines Flag, ob dieser Datenspeicher 92 und damit der Filter 11, 20, an dem dieser Datenspeicher 92 befestigt ist, bereits in eine Aufnahme-Anordnung 100 eingesetzt wurde oder nicht. Der Datenspeicher 92 eines neuen Filters 11, 20 enthält die Information, dass der Filter 11, 20 noch nicht eingesetzt wurde. Das Lese- und Schreibgerät liest nach Einsetzen eines Filters 11, 20 diese Informationen aus. Falls der Filter 11, 20 bereits früher in eine Aufnahme-Anordnung 100 eingesetzt wurde, so erzeugt das Lese- und Schreibgerät eine entsprechende Meldung. Ansonsten speichert sie auf dem Datenspeicher 92 die Information ab, dass der Filter 11, 20 nunmehr in eine Aufnahme-Anordnung 100 eingesetzt wurde. Bevorzugt erzeugt das Lese- und Schreibgerät eine Fehlermeldung, falls es nach Einsetzen eines Filters 11, 20 keinen Datenspeicher 92 findet oder den Datenspeicher 92 nicht auslesen kann. Im Falle eines Barcodes liest das Lesegerät beispielsweise die eindeutige Kennung des Filters 11, 20 ein und macht anschließend den Barcode 92 unleserlich, beispielsweise durch Aufsprühen einer Flüssigkeit oder Übermalen oder Überkleben.

In einer Ausgestaltung lässt sich auf dem Datenspeicher 92 die Information abspeichern und wieder auslesen, wann ein Filter 11, 20 mit diesem Datenspeicher 92 erstmals in einen Topf 13 einer Aufnahme-Anordnung 100 eingesetzt wurde. Bei einem neuen Filter 11, 20 ist kein Datum des Einsetzens abgespeichert. Das Lese- und Schreibgerät speichert das Datum, optional den Zeitpunkt, ab, an dem ein Filter 11, 20 in einen Topf 13 eingesetzt wurde. Diese Information lässt sich später wieder auslesen, um zu ermitteln, wie lange der Filter bereits in dem Topf 13 verwendet wurde. In manchen Fällen ist eine maximale Verwendungsdauer eines Filters 11, 20 vorgegeben. In einer anderen Realisierungsform enthält der Barcode 92 die Information, wann der Filter 11, 20 hergestellt wurde. Das Lesegerät liest dieses Herstellungsdatum ein und macht danach den Barcode unleserlich.

Die gerade beschriebene Ausgestaltung mit dem Datum des Einsetzens lässt sich auch dafür verwenden, um zu verhindern, dass derselbe Filter 11, 20 mehrmals nacheinander eingesetzt wird. Falls das Lese- und Schreibgerät bei einem gerade eingesetzten Filter bereits in dem Datenspeicher 92 ein früheres Datum des Einsetzens entdeckt, so wurde dieser Filter 11, 20 bereits früher verwendet, und das Lese- und Schreibgerät erzeugt eine entsprechende Meldung. Falls der Barcode 92 nicht leserlich ist, wird ebenfalls eine Fehlermeldung ausgegeben.

In einer Ausgestaltung ist in dem Datenspeicher 92 die Information abgespeichert, für welche Narkosemittel das Filterelement 11 verwendet werden darf, und / oder die Information, für welche es nicht verwendet werden darf. Das Lesegerät liest diese Information aus dem Datenspeicher 92 aus. Bevorzugt steht das Lesegerät in einer Datenverbindung mit dem Anästhesiegerät 1 und übermittelt die Information über die zulässigen und / oder nicht zulässigen Narkosemittel des aktuell verwendeten Filterelements 11 an das Anästhesiegerät 1. Beispielsweise aufgrund einer Benutzereingabe ist in einem internen Datenspeicher des Anästhesiegeräts 1 die Information abgespeichert, welche Narkosemittel aktuell verwendet werden. Das Anästhesiegerät 1 prüft automatisch, ob das Filterelement 11 für das oder jedes aktuell verwendete Narkosemittel zugelassen ist oder nicht. Bei einem nicht zugelassenen Filterelement 11 erzeugt das Anästhesiegerät 1 eine entsprechende Fehlermeldung in einer von einem Menschen wahrnehmbaren Form.

Der Topf 13 ist fluiddicht mit der Zuführleitung 6 und mit der Abführleitung 8 verbunden. Das Material, aus dem die Wände des Topfs 13 gefertigt sind, ist für Fluid undurchlässig und wird nicht chemisch von einem Narkosemittel angegriffen. Bevorzugt sind die Wände des Topfs 13 aus einem harten Kunststoff oder aus Metall gefertigt.

Wenn die Kartusche 20 korrekt und insbesondere in richtiger Drehposition in den Topf 13 eingesetzt ist, so überlappt in einer Ausgestaltung die Austrittsöffnung 14 der Topf-Zuführleitung 16 mit einer Einlassöffnung 25 in der Kartusche 20. Die Eintrittsöffnung 35 überlappt mit einer Auslassöffnung 34 in der Kartusche 20. Die beiden Öffnungen 14, 35 befinden sich nahe dem Boden 44 des Topfs 13, die beiden Öffnungen 25, 34 in der Mantelfläche 43 und nahe dem Boden 39 der Kartusche 20. Überschussgas kann durch die Einlassöffnung 25 in der Kartusche 20 in das Filterelement 11 hineinfließen und durch die Auslassöffnung 34 in der Kartusche 20 wieder aus das Filterelement 11 hinausfließen. Der Narkosemittelfilter 11, 20 bewirkt eine Zwangsführung von Gas, welches durch den Narkosemittelfilter 11, 20 fließt, sodass das Überschussgas über einen relativ langen Weg von der Einlassöffnung 25 zu der Auslassöffnung 34 durch den Narkosemittelfilter 11, 20 fließt.

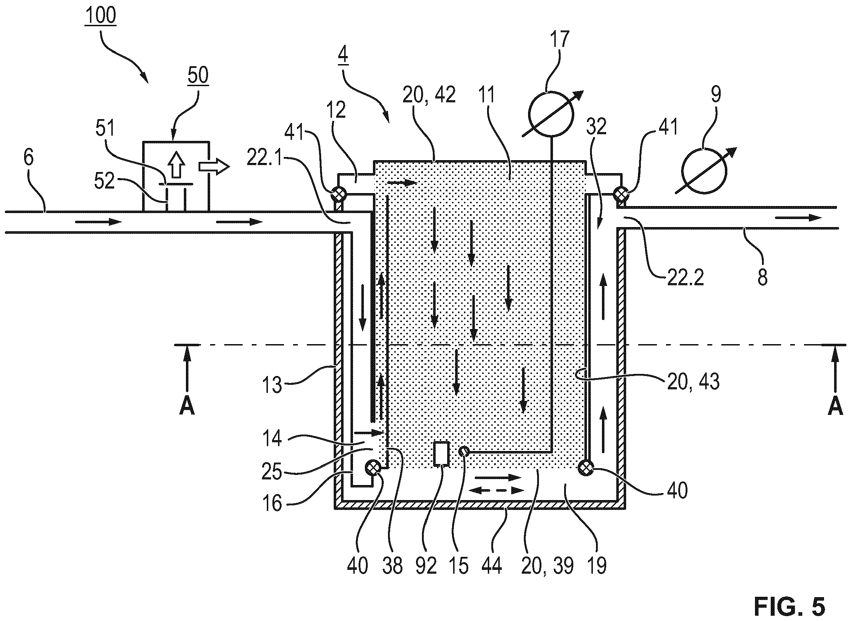

In einer Ausgestaltung, die in Figur 5 gezeigt wird, ist an der Zuführleitung 6 ein Überdruck-Ventil 50 angeordnet, wobei das Überdruck-Ventil 50 in einer Fluidverbindung mit der Zuführleitung 6 steht. Das Überdruck-Ventil 50 befindet sich also flussaufwärts von der Filtereinheit 4. Abhängig von der Differenz zwischen dem Druck in der Zuführleitung 6 und den Druck in der Umgebung um die Zuführleitung 6 herum ist das Überdruck-Ventil 50 geschlossen oder geöffnet. Solange die Druckdifferenz unterhalb einer vorgegebenen Überdruck-Schranke liegt, ist dieses Überdruck-Ventil 50 geschlossen. Sobald die Druckdifferenz die vorgegebene Überdruck-Schranke erreicht oder übersteigt, öffnet sich das Überdruck-Ventil 50 automatisch oder wird geöffnet und stellt eine Fluidverbindung zwischen der Zuführleitung 6 und der Umgebung her. Überschüssiges Gas kann in die Umgebung entweichen. Bevorzugt verschließt das Überdruck-Ventil 50 sich automatisch wieder oder wird geschlossen, sobald die Druckdifferenz wieder unterhalb der Überdruck-Schranke liegt.

In einer einfachen mechanischen Realisierung umfasst das Überdruck-Ventil 50 eine Ventilplatte 51, die auf einem nach oben offenen Ventilkrater 52 liegt. Das Verhältnis zwischen dem Gewicht der Ventilplatte 51 und der Fläche der oberen Öffnung des Ventilkraters 52 legt die erzielte Überdruck-Schranke fest. Ein Gasdruck oberhalb der Überdruck-Schranke hebt die Ventilplatte 51 vom Ventilkrater 52 an, sodass das Gas entweichen kann. Die Schwerkraft senkt die Ventilplatte 51 wieder ab.

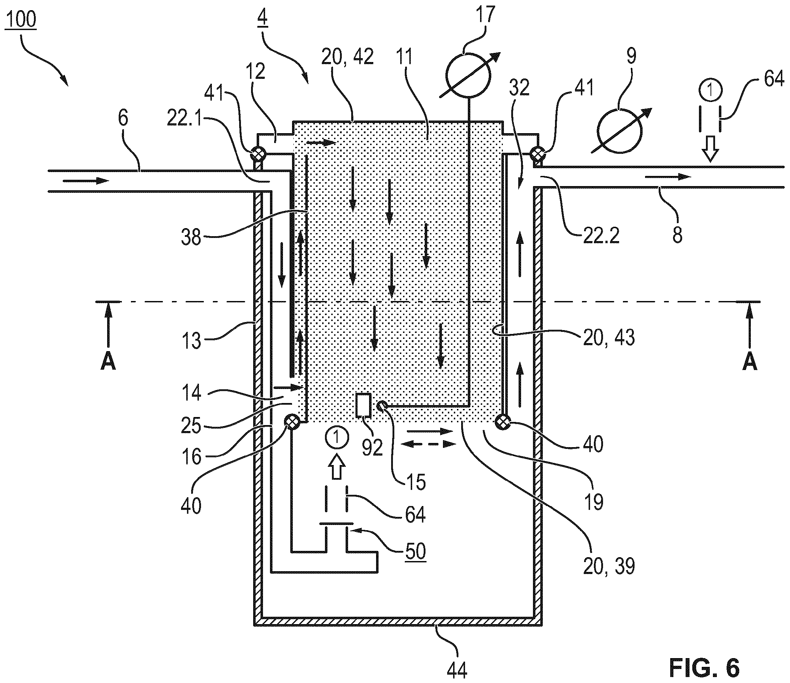

Figur 6 zeigt eine Abwandlung, bei der ebenfalls ein Überdruck-Ventil 50 verwendet wird, dieses Überdruck-Ventil 50 das überschüssige Gas aber nicht in die Umgebung ablässt. Vielmehr ist das Überdruck-Ventil 50 parallel zu der Filtereinheit 4 geschaltet. Das Überdruck-Ventil 50 befindet sich im Inneren des Topfs 13 und dort am oder nahe dem Ende der Topf-Zuführleitung 16. Gas tritt durch das Überdruck-Ventil 50 aus der Topf-Zuführleitung 16 in den Topf 13 aus, wenn die Druckdifferenz in der Topf-Zuführleitung 16 oberhalb der Überdruck-Schranke liegt. Ein nur schematisch angedeuteter Schlauch 64 führt das überschüssige Gas, welches im Falle eines Überdrucks aus dem Überdruck-Ventil 50 austritt, an der Filtereinheit 4 vorbei zu der Abführleitung 8. Dieser Schlauch 64 kann wenigstens teilweise im Inneren des Topfs 13 angeordnet sein.

Der Messfühler 15 misst in einer Ausgestaltung ein Maß für die Menge an Narkosemittel, welches nicht vom Filterelement 11 aufgenommen wird und daher den Filter 11, 20 verlässt. Bevorzugt befindet der Messfühler 15 sich flussabwärts von der Filtereinheit 4 und misst die Konzentration des oder mindestens eines Narkosemittels in dem Gasstrom um den Messfühler 15 herum. Idealerweise filtert der Narkosemittelfilter 11, 20 aus dem durchströmenden Gas das gesamte Narkosemittel heraus und nimmt es auf. Wenn die Menge an Narkosemittel, das nicht von dem Filterelement 11 aufgesogen oder anderweitig aufgenommen wird, oberhalb einer vorgegebenen Schranke liegt, so ist dies ein Indiz dafür, dass das Filterelement 11 mit Narkosemittel gefüllt ist oder sich zugesetzt hat, kein weiteres Narkosemittel aufnehmen kann und daher ausgetauscht werden muss. Im Ausführungsbeispiel gehören der Messfühler 15, die Messleitung 18 und die Verbrauchsanzeige 17 zur Kartusche 20. Dieselbe Kartusche 20 kann nacheinander für mehrere Filterelemente 11 verwendet werden. Möglich ist auch, dass der Messfühler 15, die Messleitung 18 und die Verbrauchsanzeige 17 getrennt von der Kartusche 20 positioniert sind.

Die Kartusche 20 hängt in einer Ausgestaltung im Topf 13, wobei der umlaufende Rand 12 oben auf der Oberkante des Topfs 13 aufliegt. Zwei obere Dichtelemente 41 oder ein einziges kreisförmiges Dichtelement 41 verschließen den Bereich zwischen dem oberen Rand des Topfs 13 und dem umlaufenden Rand 12. Bevorzugt ist der Dichtring oder ein anderes geeignetes Dichtelement 41 vollständig um die Oberkante des Topfs 13 herumgeführt, sodass die eingesetzte Kartusche 20 gasdicht in Topf 13 hängt. Weil die Kartusche 20 im Topf 13 hängt und im Ausführungsbeispiel nicht so hoch wie der Topf 13 ist, entsteht ein Zwischenraum zwischen dem Boden 39 der Kartusche 20 und dem Boden 44 des Topfs 13, der zu einem nachfolgend beschriebenen Pufferspeicher gehört. Möglich ist auch, dass die Kartusche 20 keinen umlaufenden Rand 12 aufweist und nicht im Topf 13 hängt, sondern auf dem Boden 44 des Topfs 13 steht.

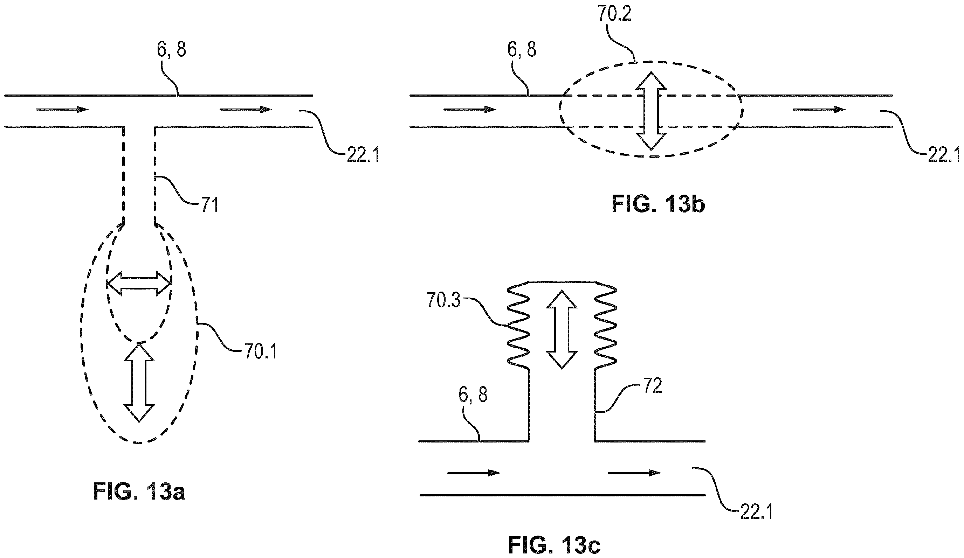

In allen Ausgestaltungen umfasst die Filtereinheit 4 einen Pufferspeicher 19 (Ausgestaltung gemäß Figur 2 bis Figur 11 und Figur 14 bis Figur 19) im Inneren des starren Topfs 13 bzw. einen dehnbaren Pufferspeicher 70 (Figur 13) bzw. einen starren Pufferspeicher 23 (Ausgestaltung gemäß Figur 20 bis Figur 23) an der Zuführleitung 6 oder der Abführleitung 8, wobei der Pufferspeicher 19, 23, 70 sowohl mit den Gasleitungen 6 und 8 als auch mit der Umgebung in einer Fluidverbindung steht und Fluid aufzunehmen und abzugeben vermag. Dieser Pufferspeicher 19, 23, 70 nimmt Gas auf, welches aus dem Anästhesiegerät 1 ausgetreten ist, solange die Rate des Fluidzuflusses in der Zuführleitung 6 größer ist als die Rate des Fluidabflusses in der Abführleitung 8. Der Pufferspeicher 19, 23, 70 gibt das aufgenommene Gas wieder ab, wenn umgekehrt der Volumenfluss in der Abführleitung 8 größer ist als der Volumenfluss in der Zuführleitung 6. Insbesondere dann, wenn der Patient P ausatmet, also in der Exspirationsphase, fließt in der Regel mehr Fluid vom Anästhesiegerät 1 in die Zuführleitung 6 als aus der Zuführleitung 6 abfließt. Wenn der Patient P einatmet, also in der Inspirationsphase, fließt weniger Fluid in die Zuführleitung 6 als abfließt.

Die beschriebenen Ausgestaltungen eines Pufferspeichers 19, 23, 70 lassen sich kombinieren. Möglich ist also, dass eine erfindungsgemäße Aufnahme-Anordnung 100 einen, zwei oder drei der gerade beschriebenen verschiedenartigen Pufferspeicher 19, 23, 70 und / oder zwei gleichartige Pufferspeicher aufweist.

In einer Ausgestaltung befindet sich ein starrer Pufferspeicher 23 unterhalb des Topfs 13. Das aufzunehmende Gas ist in der Regel schwerer als Luft und sinkt durch eine Öffnung 26 im Boden 44 des Topfs 13 hindurch in den Pufferspeicher 23, der sich unterhalb des Topfs 13 befindet.

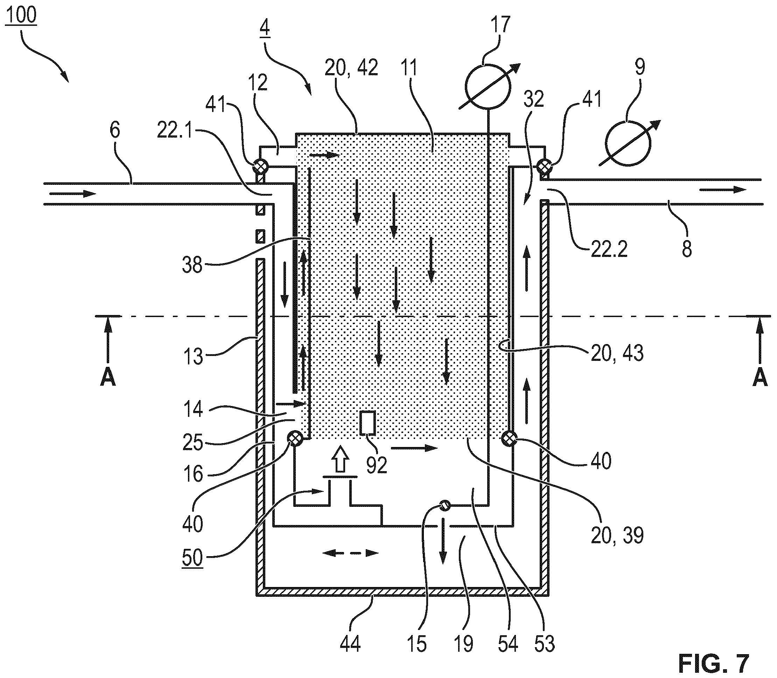

Im Beispiel von Figur 7 trennt eine bevorzugt horizontale Trennwand 53 den Pufferspeicher 19 von der Filtereinheit 4. Sowohl das Gas, das durch die Filtereinheit 4 fließt und in dieser gefiltert wird, als auch das überschüssige Gas, welches aus dem Überdruck-Ventil 50 austritt, gelangt in einen Raum 54 zwischen der Filtereinheit 4 und der Trennwand 53. Das Gas aus diesem Raum 54 gelangt durch eine Öffnung in der Trennwand 53 in den Pufferspeicher 19 und fließt dann aus dem Pufferspeicher 19 durch die Topf-Abführleitung 32 hindurch in die Abführleitung 8.

Der Messfühler 15 befindet sich in der Ausgestaltung von Figur 7 im Raum 54, also flussabwärts vom Filter 11, 20, und misst die Konzentration von Narkosemittel im Raum 54. Eine Konzentration oberhalb einer vorgegebenen Schranke hat mindestens eine der folgenden beiden Ursachen:
- Die Filtereinheit 4 hat sich zugesetzt oder ist aus einem anderen Grund verbraucht und vermag aus diesem oder einem anderen Grunde nicht mehr ausreichend Narkosemittel aus dem durchfließenden Gasstrom herauszufiltern.
- Der Druck in der Topf-Zuführleitung 16 liegt oberhalb der Überdruck-Schranke, und das Überdruck-Ventil 50 hat sich geöffnet und gibt Gas in den Raum 54 im Topf 13 ab.

Beide Ereignisse erfordern, die Filtereinheit 4 auszutauschen. Die Anzeigeeinheit 17 signalisiert einem Benutzer, dass die Filtereinheit 4 auszutauschen ist.

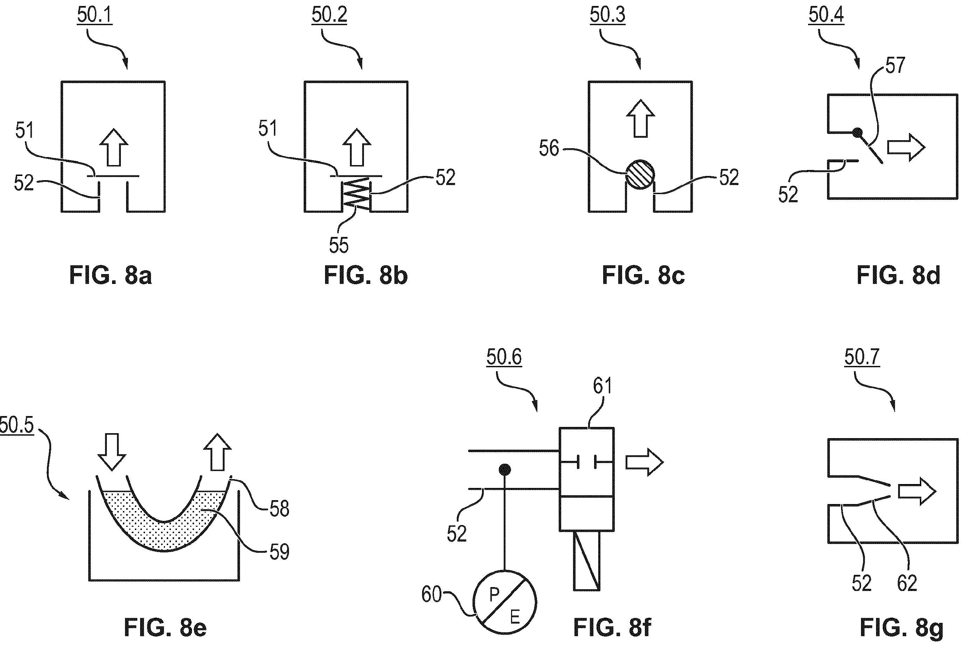

Figur 8 zeigt sieben verschiedene mögliche Realisierungsformen des Überdruck-Ventils 50. Das Überdruck-Ventil 50 in den Ausgestaltungen gemäß Figur 4, Figur 6 oder Figur 7 lässt sich mit jeder dieser Realisierungsformen implementieren.

Figur 8a) zeigt die bereits beschriebene Realisierungsform 50.1, bei der eine Ventilplatte 51 auf einem Ventilkrater 52 liegt. Bei einem ausreichend großen Druck wird die Ventilplatte 51 angehoben, sodass Gas aus einem Zwischenraum zwischen der angehobenen Ventilplatte 51 und dem Ventilkrater 52 austreten kann. Das Verhältnis zwischen dem Gewicht der Ventilplatte 51 und der Fläche der Öffnung des Ventilkraters 52 legt die Überdruck-Schranke fest.

Das Überdruck-Ventil 50.2 gemäß Figur 8b) umfasst zusätzlich eine Druckfeder 55, welche sich an einem Gehäuse des Überdruck-Ventils 50 abstützt und bestrebt ist, die Ventilplatte 51 gegen die Schwerkraft nach oben zu drücken. Bevorzugt ist die Ventilplatte 51 schwerer ausgestaltet als bei der Realisierungsform gemäß Figur 8a). Die Überdruck-Schranke wird zusätzlich durch die Federkonstante der Druckfeder 55 festgelegt. Das Überdruck-Ventil 50.2 gemäß Figur 8b) öffnet sich erst dann, wenn ein Druck oberhalb der Überdruck-Schranke ausreichend lange anliegt. Kurzfristige Druckspitzen werden also abgefedert. Die Gefahr wird verringert, dass eine Leckage in Überdruck-Ventil 50 auftritt. Ein derartiges Überdruck-Ventil wird beispielsweise in US 8 997 741 B2 beschrieben.

Bei dem Überdruck-Ventil 50.3 gemäß Figur 8c) ist die Ventilplatte 51 durch eine Ventilkugel 56 ersetzt, welche auf dem Ventilkrater 52 aufliegt. Die Überdruck-Schranke wiederum durch das Verhältnis zwischen dem Gewicht der Ventilkugel 56 und der Fläche des Ventilkraters 52 festgelegt.

Das Überdruck-Ventil 50.4 gemäß Figur 8d) umfasst eine Ventilklappe 57, die an dem Ventilkrater 52 befestigt ist und sich relativ zu dem Ventilkrater 52 um eine horizontale Drehachse, die senkrecht auf der Zeichenebene von Figur 8 steht, drehen lässt. Ein Überdruck dreht diese Ventilklappe 57 gegen die Schwerkraft weg von dem Ventilkrater 52, sodass am freien Ende des Ventilkraters 52 eine Öffnung entsteht. Die Schwerkraft sowie optional eine nicht gezeigte Zugfeder sind bestrebt, die Ventilklappe 57 in der geschlossenen Position zu halten. Alternativ ist die optionale Zugfeder bestrebt, die Ventilklappe 57 gegen die Schwerkraft in die geöffnete Position zu ziehen und in dieser zu halten. Insbesondere eine relativ schwere Ventilklappe 57 in Verbindung mit der optionalen Zugfeder führt dazu, dass das Überdruck-Ventil 50.4 sich erst dann öffnet, wenn ein Überdruck ausreichend lange anliegt. Kurzfristige Druckschwankungen werden abgefedert.

Das Überdruck-Ventil 50.5 gemäß Figur 8e) umfasst ein U-förmiges Rohr 58, in dem sich in einem unteren Teil eine Flüssigkeit 59 befindet. Diese Flüssigkeit 59 verdampft erst bei einer Temperatur oberhalb derjenigen Temperaturen, bei dem das Überdruck-Ventil 50 eingesetzt und gelagert wird. Falls der Druck die Überdruck-Schranke erreicht oder übersteigt, so tritt das Gas in Blasenform aus dem Rohr 58 aus. Diese Ausgestaltung reduziert besonders gut die Gefahr, dass Gas ungewollt durch ein Leck austritt.

Das Überdruck-Ventil 50.6 gemäß Figur 8f) umfasst ein ansteuerbares Schaltventil 61 und einen Druck-Sensor 60 in der Zuführleitung 52 zum ansteuerbaren Schaltventil 61. Die Signale vom Druck-Sensor 60 werden an einen elektrischen Komparator oder einen Prozessor weitergeleitet. Falls der Komparator oder Prozessor oder auch der Druck-Sensor 60 selber das Ereignis detektiert, dass der Druck in der Zuführleitung 52 oberhalb der Überdruck-Schranke liegt, so steuert er das Schaltventil 61 an und öffnet es dadurch. Falls der Druck wieder unterhalb der Überdruck-Schranke sinkt, so schließt er das Schaltventil 61 wieder. Optional werden ein Signal für den gemessenen Druck und / oder eine Meldung, dass die Überdruck-Schranke überschritten wurde, an eine nicht gezeigte Ausgabeeinheit weitergeleitet, bevorzugt in Verbindung mit einer Kennzeichnung der Position des Überdruck-Ventils 50.6 in dem Krankenhaus. Die Ausgabeeinheit gibt den gemessenen Druck bzw. die Meldung in einer von einem Menschen wahrnehmbaren Form aus. Diese Ausgabeeinheit kann räumlich entfernt vom der Aufnahme-Anordnung 100 angeordnet sein, beispielsweise in einer Zentrale.

Bei dem Überdruck-Ventil 50.7 gemäß Figur 8g) ist eine Art Entenschnabel 62 auf den Ventilkrater 52 aufgesetzt. Bei einem Druck oberhalb der Überdruck-Schranke öffnet der Entenschnabel 62 sich und schließt sich bei einem geringeren Druck wieder.

Verhindert werden müssen folgende beiden unerwünschten Ereignisse, die beide zu einer Schädigung des Patienten P führen könnten:
- In der Zuführleitung 6 tritt ein Rückstau auf, beispielsweise weil das Filterelement 11 viel Narkosemittel aufgenommen hat und sich dadurch oder aufgrund von Flüssigkeit teilweise zugesetzt hat oder weil das Anästhesiegerät 1 aktuell relativ viel Gas in die Zuführleitung 6 ausgestoßen hat. Dieser Rückstau könnte sich in das Anästhesiegerät 1 fortpflanzen und auf den Beatmungskreislauf auswirken.
- Durch die Zuführleitung 6 und die Abführleitung 8 hindurch wird Überschussgas aus dem Anästhesiegerät 1 abgesogen, beispielsweise weil das Anästhesiegerät 1 aktuell relativ wenig Gas in die Zuführleitung 6 ausgestoßen hat.

Das mit Bezug auf Figur 4 bis Figur 8 beschriebene Überdruck-Ventil 50 verhindert einen Überdruck, der beispielsweise aufgrund eines Rückstaus auftreten kann. Um beide unerwünschten Ereignisse zu verhindern, steht in einer Ausgestaltung der Pufferspeicher 19 über mindestens eine Öffnung 21, bevorzugt mehrere Öffnungen 21, in der Wand des Topfs 13 mit der Umgebung in einer Fluidverbindung. Wenn mehrere Öffnungen 21 vorhanden sind, bleibt die Fluidverbindung auch dann erhalten, wenn eine Öffnung 21 sich zugesetzt hat, beispielsweise weil ein Gegenstand angesogen wurde. Die Ausgestaltung, dass in die Wand des Pufferspeichers 19 mindestens eine Öffnung 21 eingelassen ist, ist insbesondere sinnvoll in Kombination mit einer Ansaugung, also mit einer Ansaugpumpe 10, die Gas in die Abführleitung 8 saugt, vgl. Figur 1. Falls Überschussgas ausschließlich dadurch in den Pufferspeicher 19 gelangt, dass das Anästhesiegerät 1 das Überschussgas ausstößt, falls also kein Ansaugen durchgeführt wird, weist der Pufferspeicher 19 bevorzugt keine dauerhafte Fluidverbindung mit der Umgebung auf.

Möglich ist, sowohl das Überdruck-Ventil 50 als auch die Öffnungen 21 in der Wand des Topfs 13 vorzusehen oder auch nur das Überdruck-Ventil 50 oder nur die Öffnungen 21. Beispielhaft werden in Figur 2 bis Figur 4, Figur 10, Figur 11 und Figur 14 bis Figur 17 die Öffnungen 21 gezeigt und in Figur 5 bis Figur 7 das Überdruck-Ventil 50.

Wie bereits erwähnt, steht in einer Realisierungsform der Pufferspeicher 19 im starren Topf 13 über mindestens eine Öffnung 21 mit der Umgebung in einer Fluidverbindung. Der starre Pufferspeicher 23 unterhalb des Topfs 13 oder flussabwärts vom Topf 13 steht in einer Realisierungsform über mindestens eine Öffnung 24 mit der Umgebung in einer Fluidverbindung. Durch diese Öffnung 21 bzw. 24 kann Überschussgas zwischen dem Pufferspeicher 19, 23 und der Umgebung hin- und herfließen. Dies wird in Figur 2 bis Figur 24 durch gestrichelte Pfeile angedeutet.

In einer Abwandlung ist vor der oder an die Stelle der Öffnung 24 in eine Wand des Pufferspeichers 23 ein Überdruck-Ventil 50 angeordnet, welches sich dann öffnet oder dann geöffnet wird, wenn die Differenz zwischen dem Druck im Pufferspeicher 23 und dem Umgebungsdruck oberhalb einer vorgegebenen Überdruck-Schranke liegt. Dieses Überdruck-Ventil 50 kann so wie mit Bezug auf Figur 8 beschrieben aufgebaut sein.

Figur 20 bis Figur 24 zeigen eine Ausgestaltung mit einem starren Pufferspeicher 23, der über eine Öffnung 24 oder ein Überdruck-Ventil 50 mit der Umgebung in einer Fluidverbindung steht - im Falle des Überdruck-Ventil 50 natürlich nur bei geöffnetem Ventil. Möglich ist auch, dass der starre Pufferspeicher 23 über die Öffnung 24 in einer Fluidverbindung mit einem elastischen Pufferspeicher 70 steht, also einem Pufferspeicher mit veränderbaren Volumen. Dieser elastische Pufferspeicher 70 kann so aufgebaut sein, wie dies mit Bezug auf Figur 13 beschrieben wird.

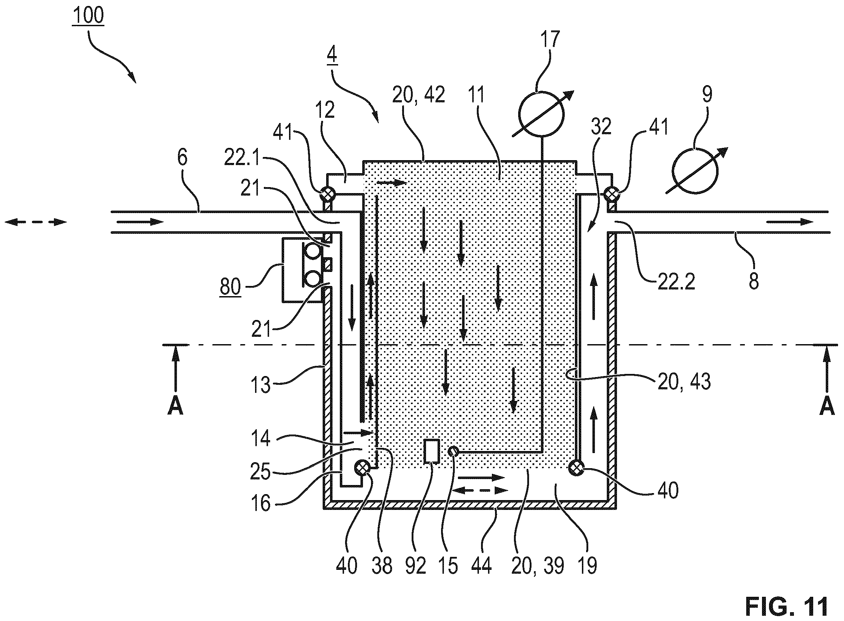

Figur 11 zeigt eine Alternative oder auch Ergänzung zu der Ausgestaltung, dass das Innere des Topfs 13 stets über die Öffnungen 21 in einer Fluidverbindung mit der Umgebung steht. Diese Alternative reduziert die Menge von Narkosemittel, welches aus dem Topf 13 austritt. Außerdem bewirkt die nachfolgend beschriebene Alternative, dass der Druck im Topf 13 um nicht mehr als eine vorgegebene Unterdruck-Schranke unterhalb des Drucks in der Umgebung um den Topf 13 herum liegt. Ein zu großer Unterdruck ist oft unerwünscht, weil er Gas aus dem Anästhesiegerät 1 absaugen kann. Die nachfolgend beschriebene Ausgestaltung, um einen zu großen Unterdruck im Topf 13 zu vermeiden, ist besonders sinnvoll in Verbindung mit einer Ansaugung, also mit einer Ansaugpumpe 10, die Gas in die Abführleitung 8 saugt. Falls der Topf 13 mindestens eine Öffnung 21 aufweist, so kann ein unerwünschter Unterdruck insbesondere dann auftreten, wenn die oder jede Öffnung 21 verstopft ist. Ein verbrauchter oder zugesetzter Filter 11, 20 kann ebenfalls bei Ansaugung zu einem zu großen Unterdruck führen.

Ein Unterdruck-Ventil 80 überdeckt die Öffnungen 21 in der Mantelwand des Topfs 13. Dieses Unterdruck-Ventil 80 öffnet sich, wenn im Topf 13 ein Unterdruck größer als die Unterdruck-Schranke herrscht, also wenn der Druck im Topf 13 um mehr als die Unterdruck-Schranke unterhalb des Umgebungsdrucks liegt. Ansonsten ist das Unterdruck-Ventil 80 geschlossen. Die Öffnungen 21 begrenzen den maximalen Volumenfluss in den Topf 13 hinein. Sie können zu dem Unterdruck-Ventil 80 gehören.

In einer Ausgestaltung wird auf einer räumlich entfernten Alarmierungseinheit ein Alarm angezeigt oder auf andere Weise ausgegeben, sobald das Unterdruck-Ventil 80 sich geöffnet hat. Beispielsweise wird dieser Alarm in einer Zentrale ausgegeben. Als Reaktion auf diesen Alarm kann ein Benutzer den Topf 13 überprüfen.

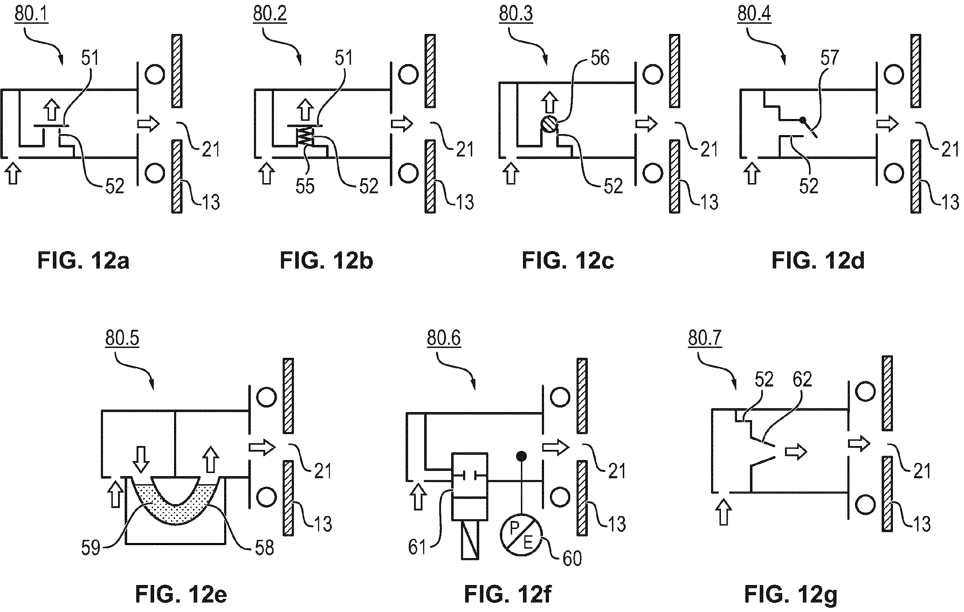

Figur 12 zeigt sieben verschiedene mögliche Realisierungsformen des Unterdruck-Ventils 80 von Figur 11. In Figur 12 werden beispielhaft ein Teil der Mantelwand des Topfs 13 sowie eine überdeckte Öffnung 21 gezeigt. Jede gezeigte Realisierungsform des Unterdruck-Ventils 80 entspricht jeweils einer Realisierungsform des Überdruck-Ventils 50, die in Figur 8 gezeigt wird. Gleiche Bezugszeichen haben in Figur 12 die gleiche Bedeutung wie in Figur 8.

Bei der Ausgestaltung gemäß Figur 2 bis Figur 17 mündet die Zuführleitung 6 in die Topf-Zuführleitung 16, die vertikal oder schräg entlang der Innenwand des Topfes 13 geführt ist und die eine Austrittsöffnung 14 nahe dem Boden 39 der Kartusche 20 aufweist. In der Mantelfläche 43 und nahe des Bodens 39 der Kartusche 20 ist eine Einlassöffnung 25 vorhanden. Überschussgas, welches aus dem Anästhesiegerät 1 ausgetreten ist, fließt durch die Zuführleitung 6 und die Öffnung 22.1 im Topf 13 in die Topf-Zuführleitung 16. Dieses Gas ist in der Regel schwerer als Luft. Daher fließt das ausgestoßene Überschussgas in der vertikal oder schräg angeordneten Topf-Zuführleitung 16 nach unten. Das ausgestoßene Überschussgas fließt durch die Gasleitungen 6 und 16 bis zur Austrittsöffnung 14 und gelangt durch die Einlassöffnung 25 in den Narkosemittelfilter 11, 20. Das Überschussgas wird von dem medizinischen Gerät 1 ausgestoßen und / oder von der Ansaugpumpe 10 angesogen und durchdringt das Filterelement 11, wobei das Überschussgas auf einem bevorzugt schlangenlinienförmigen Weg zwangsgeführt wird, wodurch Narkosemittel aus dem Gas herausgefiltert wird. Das gefilterte Überschussgas tritt aus einer Auslassöffnung 34 aus der Kartusche 20 aus, fließt durch die Eintrittsöffnung 35 in die Topf-Abführleitung 32 und gelangt durch eine Öffnung 22.2 im Topf 13 in die Abführleitung 8 und von dort in die Gasaufnahme 7.

Der Pufferspeicher 19 wird bei der Ausgestaltung gemäß Figur 2 bis Figur 17 durch einen Zwischenraum zwischen der Innenwand des Topfs 13 und der Außenwand der Kartusche 20 gebildet. Dieser Zwischenraum 19 grenzt sowohl an die Mantelfläche als auch an den Boden 44 des Topfs 13 an.

Figur 13 zeigt drei mögliche Realisierungsformen eines Pufferspeichers 70, der in Fluidverbindung mit der Zuführleitung 6 steht und in Reihe und flussaufwärts von der Filtereinheit 4 angeordnet ist. Dieser Pufferspeicher 70 ist in allen Realisierungsformen aus einem elastischen Material aufgebaut. Der Umgebungsdruck wirkt von außen auf den Pufferspeicher 70 ein. Wenn ausreichend viel Gas in die Zuführleitung 6 strömt und dadurch im Pufferspeicher 70 ein ausreichend großer Druck vorhanden ist, so wird der elastische Pufferspeicher 70 gedehnt, und zwar umso stärker, je größer die Differenz zwischen dem Druck in der Zuführleitung 6 und dem Umgebungsdruck ist. Wenn der Druck abnimmt, so zieht der elastische Pufferspeicher 70 sich wieder zusammen und gibt dadurch aufgenommenes Gas wieder in die Zuführleitung 6 ab.

Möglich ist auch, dass der elastische Pufferspeicher 70 in einer Fluidverbindung mit der Abführleitung 8 steht. Möglich ist auch, dass ein erster elastischer Pufferspeicher 70 in Fluidverbindung mit der Zuführleitung 6 und ein zweiter elastischer Pufferspeicher 70 in Fluidverbindung mit der Abführleitung 8 steht.

Gemäß der Realisierungsform gemäß Figur 13a) weist die Zuführleitung 6 ein Anschlussstück 71 auf, welches bevorzugt unter der Zuführleitung 6 oder auch der Abführleitung 8 angeordnet ist und nach unten zeigt (Narkosemittel ist schwerer als Luft). An diesem Anschlussstück 71 hängt ein Pufferspeicher 70 in Form eines elastischen Beutels 70.1. Bei einem hohen Druck fließt Gas aus der Zuführleitung 6 / Abführleitung 8 durch das Anschlussstück 71 in den Beutel 70.1 hinein und dehnt diesen. Bei abnehmendem Druck zieht der Beutel 70.1 sich wieder zusammen und gibt aufgenommenes Gas wieder an die Zuführleitung 6 / Abführleitung 8 ab. Bevorzugt hat das Anschlussstück 71 einen genormten Durchmesser von 22 mm. Als Beutel 70.1 lässt sich bevorzugt ein Beutel verwenden, der ursprünglich für die manuelle Beatmung vorgesehen war oder ausgestaltet ist, denn derartige Handbeatmungsbeutel sind bereits standardisiert und für den medizinischen Gebrauch zugelassen.

In der Realisierungsform gemäß Figur 13b) wird ein Abschnitt der Zuführleitung 6 oder auch der Abführleitung 8 von einem Pufferspeicher 70 in Form eines elastischen Beutels 70.2 vollständig oder wenigstens teilweise umgeben. Dieser Abschnitt der Zuführleitung 6 oder der Abführleitung 8 weist Löcher oder andere Öffnungen auf, sodass der Beutel 70.2 in einer Fluidverbindung mit der Zuführleitung 6 oder der Abführleitung 8 steht. Der elastische Beutel 70.2 überdeckt die Öffnungen in der Zuführleitung 6 oder der Abführleitung 8 vollständig. Wiederum wird der elastische Beutel 70.2 bei einem großen Druck gedehnt und zieht sich bei einem niedrigen Druck zusammen.

Bei der Realisierungsform gemäß Figur 13c) hat der elastische Pufferspeicher 70 die Form eines Faltenbalgs 70.3, der über ein Anschlussstück 72 in einer Fluidverbindung mit der Zuführleitung 6 oder der Abführleitung 8 steht. Der Faltenbalg 70.3 dehnt sich bei einem großen Druck entlang einer Längsachse aus und zieht sich bei einem geringeren Druck wieder zusammen. Der Faltenbalg 70.3 kann sich oberhalb oder unterhalb oder auch seitlich an der Zuführleitung 6 oder der Abführleitung 8 befinden.

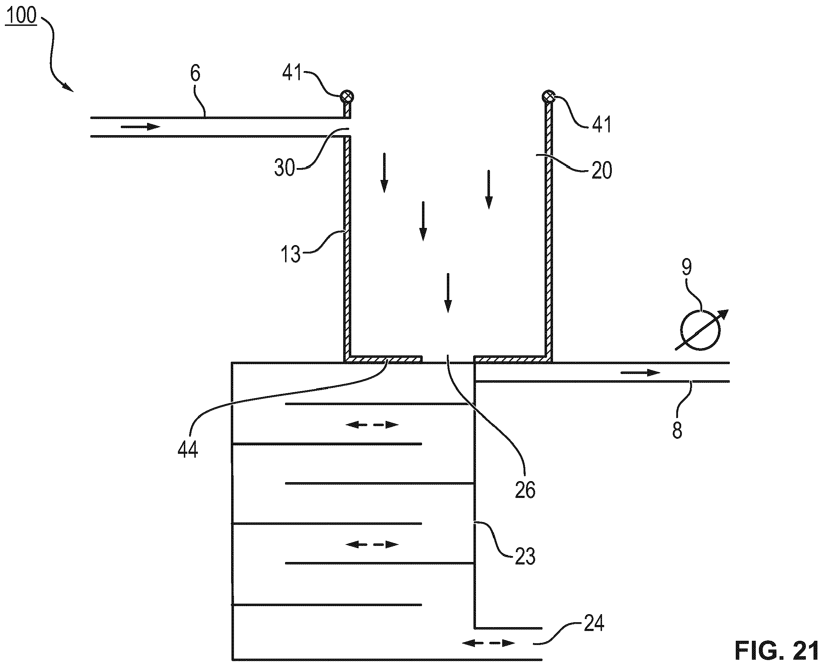

Bei der Ausgestaltung gemäß Figur 20 und Figur 21 wird der Pufferspeicher 23 durch einen separaten Pufferspeicher gebildet, der bevorzugt ein mäanderndes Rohr umfasst. Eine Einlassöffnung 30 ist im oberen Drittel der Kartusche 20 angeordnet, die Auslassöffnung 34 im Boden 39 der Kartusche 20. Der Pufferspeicher 23 steht über eine Öffnung 26 in Fluidverbindung mit dem Topf 13. Diese Öffnung 26 überlappt mit der Auslassöffnung 34. Dank der Auslassöffnung 34 im Boden 39 der Kartusche 20 steht der Pufferspeicher 23 auch in Fluidverbindung mit dem Narkosemittelfilter 11, 20. Außerdem steht der Pufferspeicher 23 in Fluidverbindung mit der Abführleitung 8 sowie über die Öffnung 24 in Fluidverbindung mit der Umgebung. Das mäandernde Rohr führt von der Öffnung 26 zu der Öffnung 24.

Ausgestoßenes Überschussgas fließt durch die Zuführleitung 6 und gelangt von der Seite zu der Einlassöffnung 30 in der Kartusche 20. Das Überschussgas fließt von oben nach unten durch den Narkosemittelfilter 11, 20 und tritt in der Auslassöffnung 25 wieder aus dem Narkosemittelfilter 11, 20 aus. Sobald die Ansaugpumpe 10 Gas ansaugt, wird dieses Gas durch die Abführleitung 8 hindurch aus dem Pufferspeicher 23 wieder abgesogen. Der Pufferspeicher 23 funktioniert also nach dem Prinzip Lastin - FirstOut. Die mäandernde Form des Pufferspeichers 23 realisiert ein langes Rohr bei relativ geringen Abmessungen. Das lange Rohr reduziert eine Durchmischung des ausgestoßenen Überschussgases mit der Umgebungsluft.

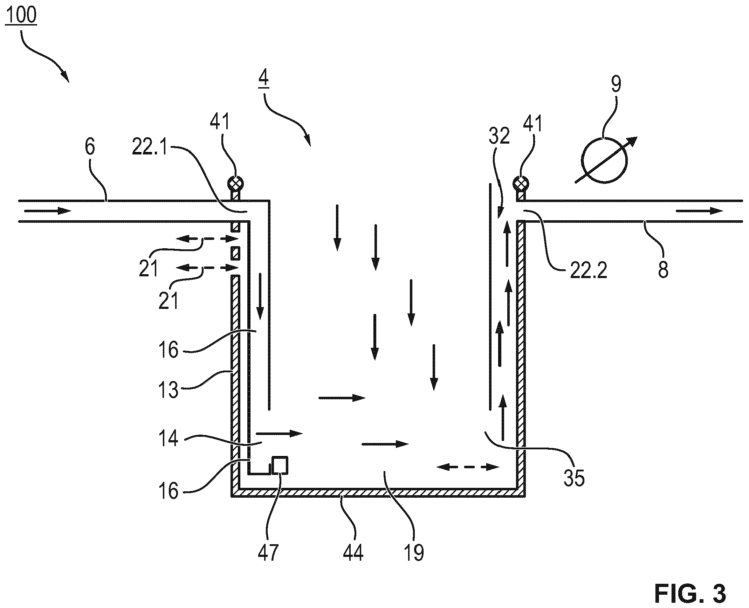

Das Filterelement 11 nimmt Narkosemittel auf und setzt sich dadurch allmählich zu und / oder wird gesättigt. Falls das Filterelement 11 sich weitgehend zugesetzt hat oder gesättigt ist und daher kein weiteres Narkosemittel mehr aufnehmen kann oder falls es aufgequollen ist, so müssen das Filterelement 11 oder der gesamte Narkosemittelfilter 11, 20 ersetzt werden. Die optionale Verbrauchsanzeige 17 zeigt in vielen Fällen dieses Ereignis an. Die Kartusche 20 mit dem Filterelement 11 und der Anzeigeeinheit 16, 17, 18 wird aus dem Topf 13 entnommen. Figur 3, Figur 10 und Figur 15 zeigen die Situation nach Entnahme der Kartusche 20. Die künstliche Beatmung des Patienten P darf nicht unterbrochen werden, während die Kartusche 20 mit dem Filterelement 11 ausgetauscht wird. Daher bleibt die Fluidverbindung zwischen dem Anästhesiegerät 1 und der Gasaufnahme 7 erhalten. Auch dann, wenn keine Kartusche 20 in den Topf 13 eingesetzt ist, wird verhindert, dass eine große Menge von ausgestoßenem Gas in die Umgebung austritt.

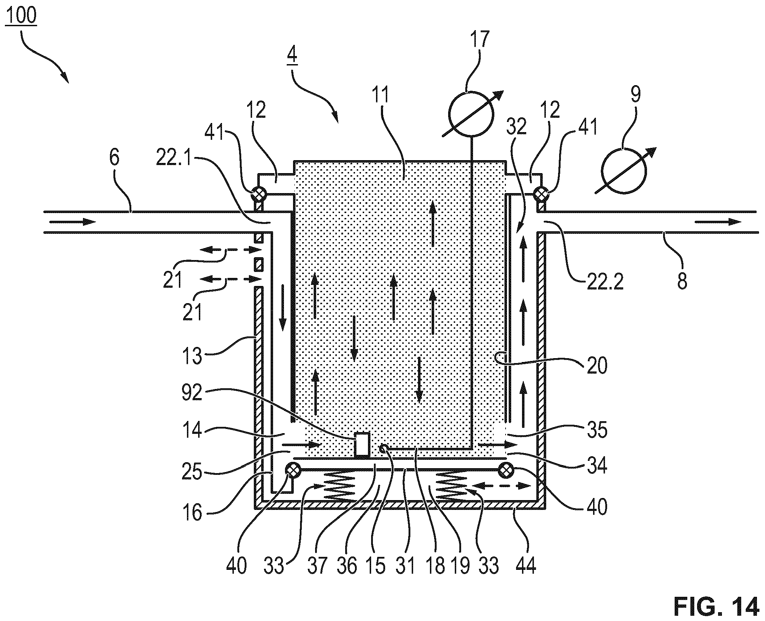

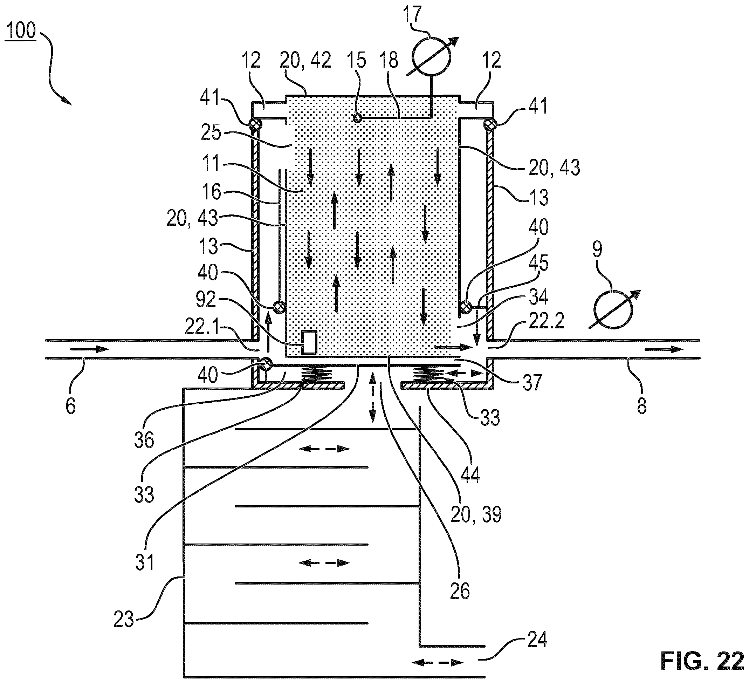

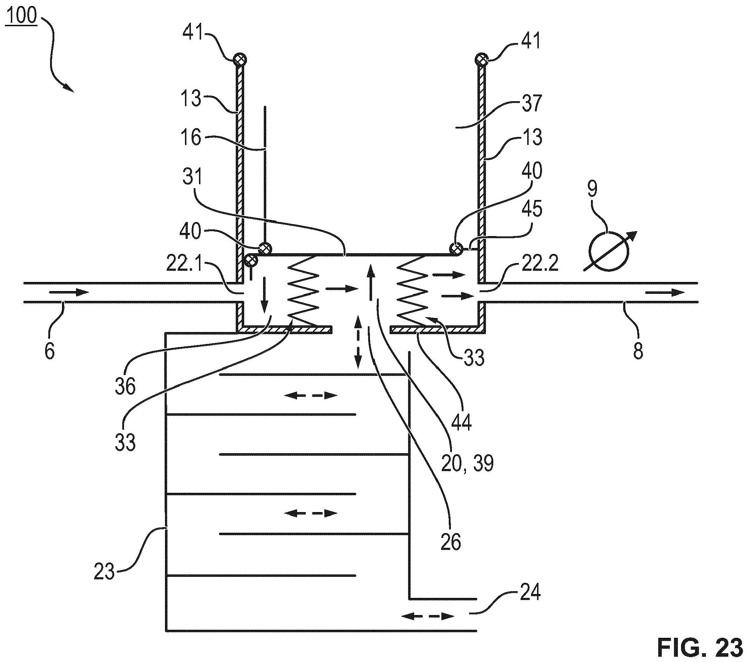

Bei der Ausgestaltung gemäß Figur 2 bis Figur 10 wird dies pneumatisch erreicht, nämlich indem Überschussgas angesogen wird, bei der Ausgestaltung gemäß Figur 14 bis Figur 17 mechanisch, nämlich durch eine federnd gelagerte Platte 31, bei der Ausgestaltung gemäß Figur 20 und Figur 21 sowie Figur 24 durch die mäandernde Form des Pufferspeichers 23 und bei der Ausgestaltung gemäß Figur 22 und Figur 23 durch eine Kombination der federnd gelagerten Platte 31 und der mäandernden Form des Pufferspeichers 23. Auch der elastische Pufferspeicher 70 von Figur 13 verhindert bis zu einem gewissen Grad, dass bei einem Austausch der Kartusche 20 eine große Menge von Narkosemittel in die Umgebung austritt.

Bei der pneumatischen Lösung gemäß Figur 2 bis Figur 10 sammelt sich das Überschussgas im Zwischenraum 19 zwischen dem Topf 13 und der Kartusche 20. Falls eine Absaugpumpe 10 vorhanden ist, so wird dieses Gas vom Boden 44 des Topfs 13 durch die Abführleitung 8 hindurch abgesogen. Die Eintrittsöffnung 35 in die Topf-Abführleitung 32 hat bevorzugt eine größere Querschnittsfläche als die gesamte Querschnittsfläche der Öffnungen 21 im Topf 13. Insbesondere deshalb wird auch dann, wenn der Volumenstrom durch die Zuführleitung 6 zeitlich oszilliert, erreicht, dass nur ein geringer Teil des Überschussgases aus dem Topf 13 nach oben oder durch die Öffnungen 21 hindurch in die Umgebung austritt. Der Topf 13 selber fungiert also als der oder ein Pufferspeicher.

Bevorzugt wird das Gas mittels einer Zwangsführung durch die Filtereinheit 4 geleitet. Diese Zwangsführung wird im Folgenden mit Bezug auf Figur 4 bis Figur 10 (pneumatische Lösung) sowie Figur 14 bis Figur 19 (mechanische Lösung) erläutert.

In das Innere des Filters 11, 20 ist eine Wand 38 eingesetzt, welche für Fluid undurchlässig ist. Diese Wand 38 erstreckt sich bevorzugt mit einem Abstand parallel zu der Mittelachse des Filters 11 und hat bevorzugt die Form einer ebenen oder entlang einer vertikalen Achse gebogenen Fläche. Die Wand 38 unterteilt die Kartusche 20 und damit das Filterelement 11 in der Kartusche 20 in einen Aufstiegsbereich Au und ein Abstiegsbereich Ab für das Gas, vgl. Figur 9. Gesehen in eine Betrachtungsrichtung parallel zu der Mittelachse des Filters 11, 20 haben sowohl der Aufstiegsbereich Au als auch der Abstiegsbereich Ab jeweils die Form eines Kreissegments. Der Aufstiegsbereich Au steht in Fluidverbindung mit der Einlassöffnung 25. Der Boden 39 der Kartusche 20 ist in dieser Realisierung zumindest im Abstiegsbereich Ab für Gas durchlässig, fungiert als die Auslassöffnung und ersetzt somit die Auslassöffnung 34 in der Mantelfläche 43. Der Abstiegsbereich Ab mündet in diesen durchlässigen Boden 39.

Das Gas aus der Zuführleitung 6 wird wie folgt durch die Filtereinheit 4 zwangsgeführt: Gas wird genau wie in den anderen Ausführungsbeispielen aus dem medizinischen Gerät 1 ausgestoßen und / oder in die Abführleitung 8 hineingesogen. Das Gas fließt durch die Topf-Zuführleitung 16 und durch die Austrittsöffnung 14 und die Einlassöffnung 25 hindurch in den Aufstiegsbereich Au, der sich zwischen der Wand der Kartusche 20 und der senkrechten Wand 38 befindet. Zwischen der Wand 38 und dem Deckel 42 der Kartusche 20 befindet sich ein Zwischenraum. Durch diesen Zwischenraum hindurch gelangt das Gas aus dem Aufstiegsbereich Au in den Abstiegsbereich Ab, ohne den Topf 13 zu verlassen. Das Gas fließt durch den Abstiegsbereich Ab hindurch zu dem durchlässigen Boden 39 und fließt dann durch die Topf-Abführleitung 32 hindurch zu der Abführleitung 8. Das Gas wird hierbei bevorzugt sowohl im Aufstiegsbereich Au als auch im Abstiegsbereich Ab, mindestens aber im Abstiegsbereich Ab, gefiltert.

Zwei untere Dichtelemente 40 oder ein umlaufender Dichtring 40 verhindern, dass angesogenes Gas aus der Topf-Zuführleitung 14 an der Kartusche 20 vorbei durch den Raum zwischen dem Boden 39 der Kartusche 20 und dem Boden 44 des Topfs 13 hindurchfließt. Dieses Gas würde am Filter 11, 20 vorbei fließen und daher nicht gefiltert werden, was unerwünscht ist.

Bei der mechanischen Lösung gemäß Figur 14 bis Figur 19 und Figur 22 ist eine Platte 31 nahe dem Boden 44 des Topf 13 gelagert. Diese Platte 31 schaltet den Gaspfad um, je nachdem, ob die Kartusche 20 eingesetzt oder entnommen ist. Die Platte 31 nimmt in einer bevorzugten Ausgestaltung die gesamte Querschnittsfläche des Topfs 13 ein - bis auf die Topf-Gasleitungen 16 und 32 und bis auf unvermeidliche Spalten. Die Platte 31 unterteilt den Innenraum des Topfs 13 in einen Filter-Hohlraum 36 und einen Pufferspeicher-Hohlraum 37. Der Filter-Hohlraum 37 befindet sich oberhalb der Platte 31 und vermag den Filter 11, 20 aufzunehmen und zu umgeben. Der Pufferspeicher-Hohlraum 36 befindet sich zwischen der Platte 31 und dem Boden 44 und gehört zu einem Pufferspeicher, was im Folgenden beschrieben wird.

Bevorzugt umgibt ein umlaufender Dichtring 40 die Umfangsfläche der Platte 31.

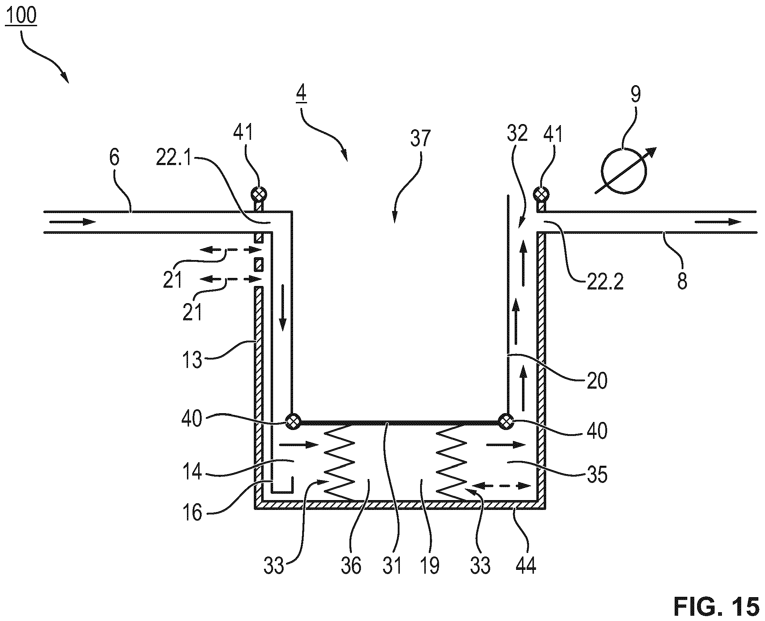

Die Zuführleitung 6 steht über die Topf-Zuführleitung 16 und die Öffnung 14 in Fluidverbindung mit der eingesetzten Kartusche 20. Die Abführleitung 8 steht über die Öffnung 35 und die Topf-Abführleitung 32 in Fluidverbindung mit der eingesetzten Kartusche 20. Die Platte 31 lässt sich zwischen einer Park-Position (Figur 14, Figur 16, Figur 18, Figur 22) und einer Pufferspeicher-Position (Figur 15, Figur 17, Figur 19) hin und her bewegen. Wenn die Kartusche 20 eingesetzt ist, so befindet sich die Platte 31 in der Park-Position, und die Kartusche 20 steht in jeweils einer Fluidverbindung mit der Zuführleitung 6 und der Abführleitung 8 oder nur mit der Abführleitung 8 (Figur 22). Die Platte 31 und der Dichtring 40 verhindern, dass Gas aus der Zuführleitung 6 an der Kartusche 20 vorbei in die Abführleitung 8 fließt. Wenn die Kartusche 20 entnommen ist, so ist die Platte 31 in der Pufferspeicher-Position, und der Pufferspeicher-Hohlraum 36 befindet sich in jeweils einer Fluidverbindung mit der Zuführleitung 6 und der Abführleitung 8. Der Dichtring 40 verhindert oder vermindert zumindest das Risiko, dass Gas aus dem Pufferspeicher-Hohlraum 36 nach oben entweicht und in die Umgebung gelangt.

Eine Ausgestaltung, wie die Platte 31 bewegt wird, wird im Folgenden beschrieben. Diese Ausgestaltung führt zu einer besonders einfachen mechanischen Ausgestaltung. Mehrere Federelemente 33 stützen sich am Boden 44 des Topf 13 ab und sind bestrebt, die Platte 31 gegen die Schwerkraft nach oben, d.h. weg vom Boden 44 des Topf 13 und in die Pufferspeicher-Position, zu drücken und in dieser zu halten. Wenn die Kartusche 20 in den Topf 13 eingesetzt wird, so wird die Platte 31 gegen die Federkraft der Federelemente 33 nach unten in die Park-Position gedrückt. Dieser Effekt wird durch das Gewicht der Kartusche 20 bewirkt - oder dadurch, dass ein Benutzer die Kartusche 20 im Topf 13 nach unten drückt. Wenn die Kartusche 20 wieder entnommen wird, so drücken die Federelemente 33 die Platte 31 nach oben in die Pufferspeicher-Position, bis die Platte 31 den unteren Abschluss der Topf-Gasleitungen 16 und 32 erreicht. Die Platte 31 verhindert in Verbindung mit den optionalen Dichtring 40 weitgehend, dass Überschussgas aus dem Topf 13 nach oben in die Umgebung austritt. Möglich ist, dass eine nicht gezeigte Rasteinheit die Platte 31 in einer Position hält.

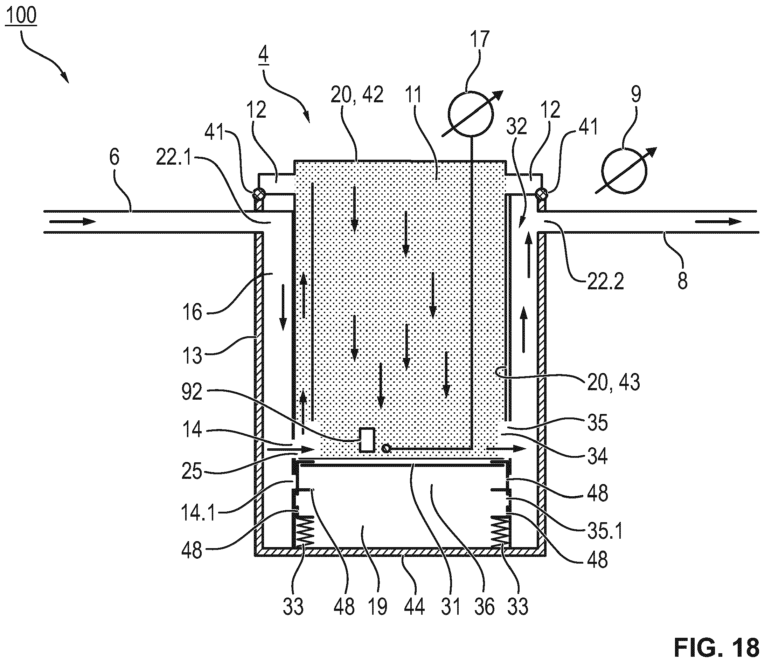

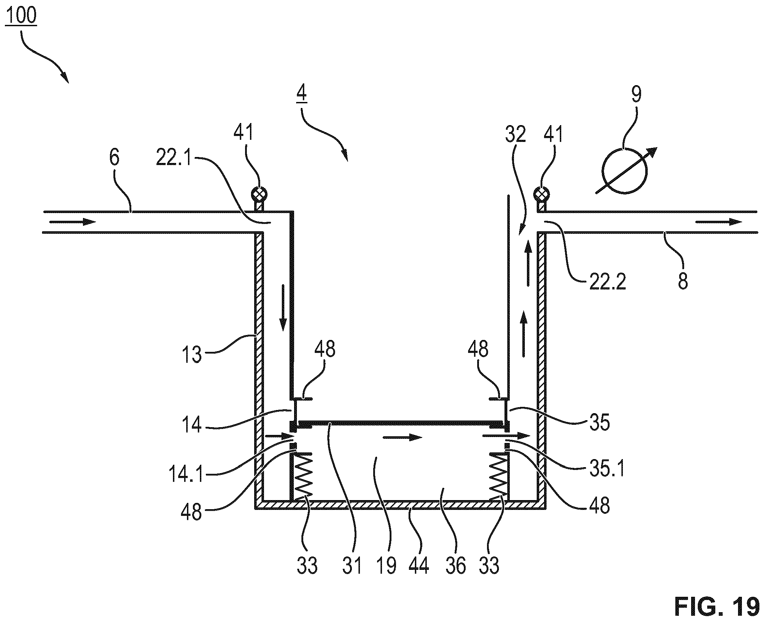

Figur 18 und Figur 19 zeigen eine weitere Ausgestaltung der mechanischen Lösung des Pufferspeichers 19. Gleiche Bezugszeichen haben die gleiche Bedeutung wie in Figur 14 bis Figur 17. Wiederum unterteilt eine Platte 31 den Innenraum des Topfs 13 in einen Filter-Hohlraum 37 und einen Pufferspeicher-Hohlraum 36. Dadurch trennt die Platte 31 einen Pufferspeicher 19 von einem eingesetzten Filter 11, 20. Dieser Pufferspeicher 19 befindet sich wiederum zwischen der Platte 31 und dem Boden 44 des Topfs 13. In der Ausgestaltung gemäß Figur 18 und Figur 19 ist keine Öffnung 21 in die Wand des Topfs 13 eingelassen. Diese Ausgestaltung wird bevorzugt in einer Anwendung ohne eine Absaugpumpe 10 eingesetzt.

Ein Schieber 48 im Inneren des Topfs 13 ist fest mit der Platte 31 verbunden und lässt sich mitsamt der Platte 31 zwischen einer Pufferspeicher-Position (Figur 19, kein Filter 11, 20 eingesetzt) und einer Park-Position (Figur 18, Filter 11, 20 in den Filter-Hohlraum 37 im Topf 13 eingesetzt) auf- und abbewegen. Die Federelemente 33 sind bestrebt, die Platte 31 mitsamt dem Schieber 48 nach oben in die Pufferspeicher-Position zu bewegen. Das Gewicht des eingesetzten Filters 11, 20 und optional ein den Filter 11, 20 einsetzender Benutzer drücken die Platte 31 und damit den Schieber 48 gegen die Federkraft der Federelemente 33 nach unten in die Park-Position. Optional rastet die Platte 31 in der Park-Position ein.

Am unteren Ende der Topf-Zuführleitung 16 sind zwei Austrittsöffnungen 14 und 14.1 angeordnet, die nachfolgend als Filter-Austrittsöffnung bzw. Pufferspeicher-Austrittsöffnung bezeichnet werden. Die Filter-Austrittsöffnung 14 befindet sich oberhalb der Pufferspeicher-Austrittsöffnung 14.1. Falls ein Filter 11, 20 korrekt eingesetzt ist (Figur 18), so überlappt genau wie bei den vorigen Ausführungsformen die Filter-Austrittsöffnung 14 mit der Einlassöffnung 25 der Kartusche 20. Die Filter-Austrittsöffnung 14 der Topf-Zuführleitung 16 führt also in die Einlassöffnung 25 des Filters 11, 20. Der nach unten gedrückte Schieber 48 gibt die Filter-Austrittsöffnung 14 frei und versperrt die Pufferspeicher-Austrittsöffnung 14.1. Falls kein Filter 11, 20 eingesetzt ist und der Schieber 48 in der Pufferspeicher-Position ist (Figur 19), so steht die Topf-Zuführleitung 16 durch die Pufferspeicher-Austrittsöffnung 14.1 hindurch mit dem Pufferspeicher 19 in einer Fluidverbindung. Die Pufferspeicher-Austrittsöffnung 14.1 führt also in den Pufferspeicher 19. Der nach oben gedrückte Schieber 48 gibt die Pufferspeicher-Austrittsöffnung 14.1 frei und versperrt die Filter-Austrittsöffnung 14.

Das entsprechende gilt für die Topf-Abführleitung 32. Am unteren Ende der Topf-Abführleitung 32 ist unterhalb der Filter-Eintrittsöffnung 35 eine Pufferspeicher-Eintrittsöffnung 35.1 eingesetzt. Falls ein Filter 11, 20 eingesetzt ist und der Schieber 48 in der Park-Position ist, so überlappt die Filter-Eintrittsöffnung 35 mit der Auslassöffnung 34 der Kartusche 20. Die Auslassöffnung 34 des Filters 11, 20 führt also in die Filter-Eintrittsöffnung 35 der Topf-Abführleitung 32. Der nach unten gedrückte Schieber 48 gibt die Filter-Eintrittsöffnung 35 frei und versperrt die Pufferspeicher-Eintrittsöffnung 35.1. Falls kein Filter 11, 20 eingesetzt ist, so steht die Topf-Abführleitung 32 durch die Pufferspeicher-Eintrittsöffnung 35.1 in Fluidverbindung mit dem Pufferspeicher 19. Der Pufferspeicher 19 führt also in die Pufferspeicher-Eintrittsöffnung 35.1 der Topf-Abführleitung 32. Der nach oben gedrückte Schieber 48 gibt die Pufferspeicher-Eintrittsöffnung 35.1 frei und versperrt die Filter-Eintrittsöffnung 35.

Falls die Platte 31 und der Schieber 48 in der Park-Position sind (Figur 18), so fließt Gas durch die Topf-Zuführleitung 16 und die Öffnungen 14 und 25 hindurch in das Filterelement 11 und anschließend durch die Öffnungen 34 und 35 hindurch in die Topf-Abführleitung 32. Der Schieber 48 verhindert, dass Gas in den Pufferspeicher 19 gelangt. Falls die Platte 31 und der Schieber 48 in der Pufferspeicher-Position sind (Figur 19), so fließt Gas durch die Topf-Zuführleitung 16 und die Öffnung 14.1 hindurch in den Pufferspeicher 19 und durch die Öffnung 35.1 in die Topf-Abführleitung 32. Die Platte 31 verhindert weitgehend, dass Gas nach oben aus dem Pufferspeicher 19 austritt.

Figur 22 und Figur 23 zeigen eine Ausführungsform, welche die federnd gelagerte Platte 31 von Figur 14 bis Figur 17 mit dem Pufferspeicher 23 von Figur 20 und Figur 21 kombiniert und ebenfalls die Topf-Zuführleitung 16 umfasst. In der in Figur 22 gezeigten Situation ist die Kartusche 20 mit dem Filterelement 11 eingesetzt, in der in Figur 23 gezeigten Situation ist sie entnommen. An der Platte 31 ist ein Dichtelement oder ein Dichtring 40 befestigt. Außerdem ist innen am Topf 13 ein weiterer umlaufender Dichtring 40 befestigt. Die Topf-Zuführleitung 16 sowie eine Halterung 45 halten diesen Dichtring 40 in einer geeigneten Position.

Die Platte 31 befindet sich in Figur 22 in der Park-Position und in Figur 23 in der Pufferspeicher-Position. Ein umlaufender Steg unten an der Platte 31 kontaktiert dann, wenn die Platte 31 in der Park-Position ist, den Boden 44 und verhindert, dass Gas aus der Zuführleitung 6 unter der Platte 31 hindurch in die Abführleitung 8 fließt. Dadurch wird verhindert, dass Gas den Narkosemittelfilter 11, 20 umgeht. Vielmehr fließt das Gas durch die Öffnung 22.1 und wird zunächst in den Zwischenraum zwischen den Topf 13 und der Kartusche 20 nach oben durch die Topf-Zuführleitung 16 hindurch bis zu der Einlassöffnung 25 geleitet, fließt dann durch den Filter 11, 20 und gelangt zu der Auslassöffnung 34. Durch die Öffnung 22.2 fließt es in die Abführleitung 8.

Wenn die Kartusche 20 entnommen und die Platte 31 in der Pufferspeicher-Position ist (Figur 23), so verhindern das Dichtelement 40 an der Platte 31 und der Dichtring 40 innen am Topf 13 das unerwünschte Ereignis, dass Gas aus dem Pufferspeicher-Hohlraum 36 an der Platte 31 vorbei nach oben fließt und in die Umgebung entweicht. Vielmehr trennen die Platte 31 und der Topf 13 den Pufferspeicher-Hohlraum 36 weitgehend von der Umgebung ab.

Auch bei dieser Abwandlung ist in den Boden 44 des Topfs 13 eine Öffnung 26 eingelassen, und der Topf 13 steht durch diese Öffnung 26 in Fluidverbindung mit einem starren Pufferspeicher 23, der ein mäanderndes Rohr umfasst. Die Einlassöffnung 25 der Kartusche 20 ist nahe dem Deckel 42 der Kartusche 20 angeordnet, die Auslassöffnung 34 nahe dem Boden 39 der Kartusche 20. Der Messfühler 15 und die Messleitung 18 sind nahe dem Deckel 42 der Kartusche 20 angeordnet. Wenn die Kartusche 20 in den Topf 13 eingesetzt wird, wird die Platte 31 gegen die Kraft der Feder 33 nach unten gedrückt und lenkt dadurch das Gas in die Kartusche 20. Wenn die Kartusche 20 entnommen wird, so drücken die Federn 33 die Platte 31 nach oben, ohne dass die Platte 31 verkantet. Wenn die Kartusche 20 eingesetzt ist, so kann Gas um die Platte 31 herum in den Pufferspeicher-Hohlraum 36 und von dort in den Pufferspeicher 23 und auch wieder zurückfließen, wodurch die Funktion eines Pufferspeichers realisiert ist.

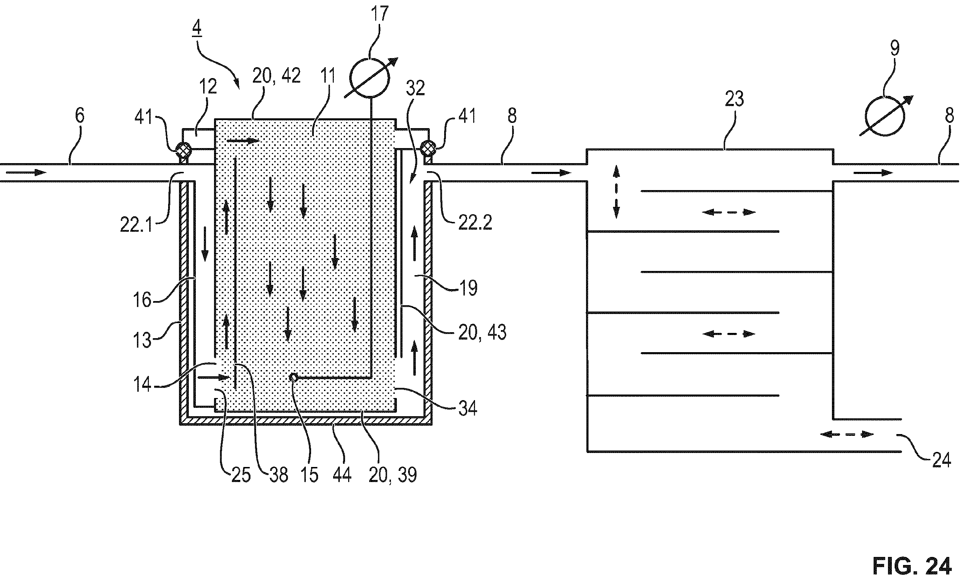

Figur 24 zeigt eine Ausgestaltung, bei welcher der starre Pufferspeicher 23 flussabwärts von der Filtereinheit 4 angeordnet ist und in einer Fluidverbindung mit der Abführleitung 8 steht. Die Bezeichnung "flussabwärts" bezieht sich auf die Fließrichtung des Gases vor dem medizinischen Gerät 1 zu der Fluidaufnahme 7. Der Pufferspeicher 23 kann auch flussaufwärts von der Filtereinheit 4 angeordnet sein und in einer Fluidverbindung mit der Zuführleitung 6 stehen.

Die Ausgestaltung gemäß Figur 24 ermöglicht es, die Filtereinheit 4 kleiner und / oder den Filter 11, 20 größer als bei der Ausgestaltung gemäß Figur 2 bis Figur 19 sowie Figur 22 und Figur 23 auszugestalten, weil nicht notwendigerweise zwischen der Kartusche 20 und dem Topf 13 ein Hohlraum aufzutreten braucht, der als Pufferspeicher fungiert. Außerdem ist die vertikale Abmessung kleiner verglichen mit den zuvor beschriebenen Ausgestaltungen. Die Abführleitung 8 führt durch den Pufferspeicher 23 hindurch. Über die Filtereinheit 4 steht der Pufferspeicher 23 indirekt auch in Fluidverbindung mit der Zuführleitung 6. Der Volumenfluss-Sensor 9 und die Ansaugpumpe 10 befinden sich bevorzugt flussabwärts von dem Pufferspeicher 23.

In der Ausgestaltung gemäß Figur 24 ist der Pufferspeicher 23 flussabwärts von der Filtereinheit 4 angeordnet. In der in Figur 13 gezeigten Abwandlung ist die Filtereinheit 4 ebenfalls flussaufwärts oder aber flussabwärts von dem elastischen Pufferspeicher 70 angeordnet. Auch in dieser Abwandlung sind bevorzugt der optionale Volumenfluss-Sensor 9 und die Ansaugpumpe 10 flussabwärts von der Filtereinheit 4 angeordnet.

Die bislang beschriebenen Ausführungsformen umfassen einen Messfühler 15, der ein Maß für die Menge oder die Konzentration des Narkosemittels innerhalb der Kartusche 20 misst. Das Ereignis, dass diese Menge oder Konzentration oberhalb einer vorgegebenen Narkosemittel-Schranke liegt, zeigt an, dass das Filterelement 11 nicht mehr genug Narkosemittel aus dem durchströmenden Gasfluss herausfiltert. Als Reaktion auf die Detektion dieses Ereignisses wird ein Alarm auf der Verbrauchsanzeige 17 ausgegeben. Bevorzugt wird auch dann ein Alarm ausgegeben, wenn das Überdruck-Ventil 50 sich geöffnet hat. Ein möglicher Grund dafür, dass das Überdruck-Ventil 50 sich geöffnet hat, ist der, dass das Filterelement 11 sich zugesetzt hat, beispielsweise weil das Filterelement 11 aufgrund von Feuchtigkeit aufgequollen ist.

Die oder eine Verbrauchsanzeige 17 kann an der Filtereinheit 4 oder an dem Topf 13 angeordnet sein. Möglich ist auch, dass eine Verbrauchsanzeige oder eine Alarmierungseinheit räumlich entfernt angeordnet ist, beispielsweise in einer Zentrale. In der Zentrale wird ein Alarm angezeigt, wenn der Messfühler 15 eine hohe Konzentration von Narkosemittel gemessen hat oder wenn das Überdruck-Ventil 50 sich geöffnet hat und es daher erforderlich ist, den Filter 11, 20 zu überprüfen und bei Bedarf auszutauschen.

Eine alternative Ausführungsform vermeidet die Notwendigkeit, einen Messfühler 15 für die Menge oder Konzentration von Narkosemittel im Inneren des Topfs 13 vorzusehen. Vielmehr wird die Menge von Narkosemittel, die durch die Zuführleitung 6 zum Narkosemittelfilter 11, 20 fließt, näherungsweise ermittelt. Gemäß einer möglichen Realisierungsform wird an mehreren aufeinanderfolgenden Abtast-Zeitpunkten der jeweils aktuelle Volumenfluss in die Zuführleitung 6 hinein gemessen, also das Volumen von Gas pro Zeiteinheit, welches das Anästhesiegerät 1 in die Zuführleitung 6 ausstößt. Oder der aktuelle Volumenfluss wird auf eine andere Weise ermittelt. Außerdem wird näherungsweise gemessen oder auf andere Weise ermittelt, welche Konzentration das Narkosemittel in diesem Gasstrom aktuell aufweist. Aus diesen beiden Werten wird der Volumenfluss von Narkosemittel berechnet, der an einem bestimmten Abtast-Zeitpunkt durch die Zuführleitung 6 in der Filtereinheit 4 fließt und idealerweise vollständig von dem Filterelement 11 aufgenommen wird. Über diese Werte für den Narkosemittel-Volumenfluss wird numerisch aufintegriert. Dieses Vorgehen liefert einen Näherungswert für die Menge von Narkosemittel, welche das Filterelement 11 bislang aufgenommen hat. Der auf diese Weise berechnete Näherungswert kann größer sein als die tatsächlich aufgenommene Menge, insbesondere wenn das Überdruck-Ventil 50 sich zeitweise öffnet oder wenn mindestens ein Leck zwischen dem Anästhesiegerät 1 und dem Filter 11, 20 auftritt.

In einer alternativen Ausgestaltung wird wiederholt ein Maß für die Menge des flüssigen Narkosemittels im Narkosemittel-Tank 49 gemessen, beispielsweise mithilfe eines Schwimmers im Tank 49. Diese Menge nimmt mit der Zeit ab, weil dem Trägergas Narkosemittel aus dem Tank 49 zugesetzt wird. Ein Teil desjenigen Narkosemittels, das den Tank 49 verlässt, wird dem Trägergas zugesetzt, beispielsweise durch Verdampfen, und gelangt in die Zuführleitung 6. Die Menge von Narkosemittel, die bislang den Tank 49 verlassen hat, ist eine obere Schranke für die Menge des Narkosemittels, welche das Filterelement 11 bislang aufgenommen hat.

Die beiden gerade beschriebenen Ausgestaltungen lassen sich kombinieren, insbesondere um die Ungenauigkeit zu verringern.

Bevorzugt wird der Wert für die aufgenommene Menge auf null gesetzt, wenn ein neuer Filter 11, 20 in den Topf 13 eingesetzt wird. Beispielsweise umfasst der Filter 11, 20 einen Datenträger 92, beispielsweise einen RFID-Chip, wobei der Datenträger 92 bevorzugt an einer Außenwand der Kartusche 20 angeordnet ist. Die Aufnahme-Anordnung 100 umfasst ein Lese- und Schreibgerät für einen solchen Datenträger, beispielsweise ein RFID-Lese- und Schreibgerät. In einer alternativen Ausgestaltung gibt ein Benutzer mithilfe eines stationären oder tragbaren Rechners oder mittels einer sonstigen Eingabeeinheit die Information ein, dass ein neuer Filter 11, 20 in den Topf 13 eingesetzt wird.

In einer Ausgestaltung wird die vorgegebene Konzentration ermittelt, welche an dem Narkosemittelverdampfer 2 eingestellt ist, vgl. Figur 1. In einer anderen Ausgestaltung wird die tatsächliche Konzentration von Narkosemittel an mindestens einem Messpunkt im Anästhesiegerät 1 gemessen.

In der Regel umfasst der Narkosemittelverdampfer 2 einen Vorratstank 49 für flüssiges Narkosemittel. In einer Ausgestaltung misst ein Füllstands-Sensor ein Maß für die Menge des flüssigen Narkosemittel in diesem Tank, beispielsweise mithilfe eines Schwimmers. Aus den Messwerten dieses Füllstands-Sensors wird hergeleitet, welche Menge an flüssigen Narkosemittel bislang verdampft wurde. Diese bislang verdampfte Menge ist eine obere Schranke für die Menge von Narkosemittel, welche das Filterelement 11 bislang aufgenommen hat.

In einer Ausgestaltung stößt das Anästhesiegerät 1 nur in einer Exspirationsphase Narkosemittel in die Zuführleitung 6 aus. Daher werden bei der Berechnung der Narkosemittel-Menge nur Werte an solchen Abtast-Zeitpunkten berücksichtigt, die in einer Exspirationsphase liegen. Ein solches Vorgehen wird beispielsweise in DE 10 2006 027 052 B3 beschrieben. In einer anderen Ausgestaltung ist die Zufuhr von Atemluft oder anderem Frischgas so groß, dass das Anästhesiegerät 1 auch in einer Inspirationsphase Narkosemittel ausstößt.

In einer Ausgestaltung ist auf einem Datenspeicher die Information hinterlegt, welche Menge an Narkosemittel das Filterelement 11 maximal aufnehmen kann, beispielsweise auf dem gerade beschriebenen Datenspeicher 92 an der Außenwand der Kartusche 20. Diese Menge ist vorgegeben. Ein Komparator vergleicht wiederholt die Menge von Narkosemittel, welche dieses Filterelement 11 bislang aufgenommen hat und die so wie gerade beschrieben näherungsweise berechnet wird, mit der vorgegebene und abgespeicherten Menge, die das Filterelement 11 maximal aufnehmen kann. Sobald die tatsächlich aufgenommene Menge sich um weniger als eine vorgegebene absolute oder prozentuale Schranke von der vorgegebenen und abgespeicherten maximalen Menge unterscheidet, wird eine Warnung in einer von einem Menschen wahrnehmbaren Form ausgegeben, beispielsweise auf einer räumlich entfernten Ausgabeeinheit, wobei die Ausgabeeinheit insbesondere in einer Zentrale positioniert ist.

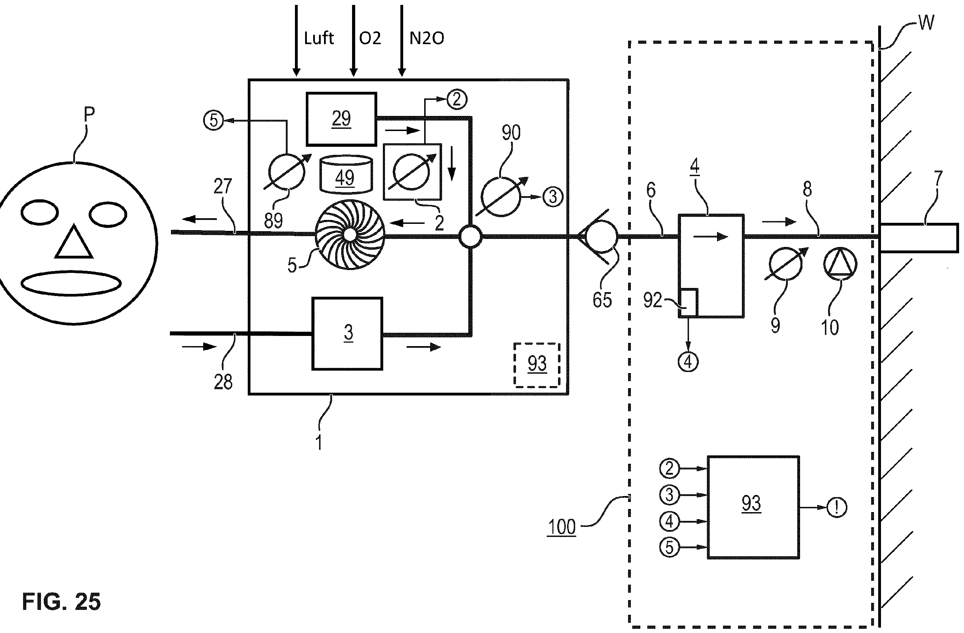

Figur 25 veranschaulicht eine beispielhafte Ausgestaltung, wobei die entsprechenden Bestandteile gegenüber der Anordnung von Figur 1 ergänzt sind. Gleiche Bestandteile haben die gleichen Bezugszeichen wie in Figur 1. Zusätzlich dargestellt werden:
- ein Füllstands-Sensor 89, der ein Maß für den aktuellen Füllstand im Narkosemittel-Tank 49 misst und beispielsweise einen Schwimmer auf der Oberfläche des Narkosemittels im Tank 49 und / oder eine Waage umfasst,
- ein Volumenfluss-Sensor 90, der zum Anästhesiegerät 1 gehört und den Volumenfluss, also das Volumen pro Zeiteinheit, von Gas misst, den das Anästhesiegerät 1 in die Zuführleitung 6 ausstößt,
- ein Konzentrations-Sensor 91, der die Konzentration von Narkosemittel misst, die am Narkosemittelverdampfer 2 eingestellt wird und / oder die der Narkosemittelverdampfer 2 tatsächlich erzielt,
- der Datenspeicher 92 eines Filters 11, 20 der Filtereinheit 4 und
- ein datenverarbeitender Narkosemittel-Mengen-Ermittler 93.

Beispielhaft wird in Figur 25 ein Rückschlag-Ventil 65 gezeigt, welches zwischen dem Anästhesiegerät 1 und der Aufnahme-Anordnung 100 angeordnet ist, also flussaufwärts von der Zuführleitung 6. Das Rückschlag-Ventil 65 lässt Gas aus dem Anästhesiegerät 1 passieren, verhindert aber, dass Gas aus der Zuführleitung 6 in das Anästhesiegerät 1 fließen kann. Insbesondere kann kein Gas aus der Fluidaufnahme 7 in das Anästhesiegerät 1 fließen.

In dem in Figur 25 gezeigten Beispiel ist auf dem Datenspeicher 92 eine Information über die maximale Menge von Narkosemittel gespeichert ist, die das Filterelement 11 aufnehmen kann. Diese maximale Menge kann als Volumen des gasförmigen oder flüssigen Narkosemittels oder auch als dessen Gewicht angegeben werden.

Der Narkosemittel-Mengen-Ermittler 93 empfängt Messwerte von den Sensoren 89, 90 und 91 und eine Information über die maximale Menge vom Datenspeicher 92. Der Narkosemittel-Mengen-Ermittler 93 berechnet so wie gerade geschrieben ein Maß für die Menge von Narkosemittel, die das Filterelement 11 bislang aufgenommen hat, vergleicht die bislang aufgenommene Menge mit der maximalen Menge und gibt eine Warnung aus, wenn die bislang aufgenommene Menge nahe der maximalen Menge des Filterelements 11 liegt.

In einer Ausgestaltung ist der Narkosemittel-Mengen-Ermittler 93 in dem Anästhesiegerät 1 realisiert, was in Figur 25 angedeutet ist. Möglich ist auch, dass der Narkosemittel-Mengen-Ermittler 93 auf einem separaten Rechner, beispielsweise auf einem Smartphone, implementiert ist.

Eine Ausgestaltung berücksichtigt die Möglichkeit, dass dasselbe Filterelement 11 nacheinander oder auch gleichzeitig unterschiedliche Narkosemittel aufnehmen kann. Möglich ist beispielsweise, dass die Aufnahme-Anordnung 100 nacheinander bei verschiedenen Operationen eingesetzt wird, bei denen unterschiedliche Narkosemittel zur Anwendung kommen. Möglich ist auch, dass die Aufnahme-Anordnung 100 bei einer Operation eingesetzt wird, bei welcher eine Mischung von mehreren Narkosemitteln angewendet wird.

Auf dem Datenspeicher 92 ist gemäß dieser Ausgestaltung die Information abgespeichert, welche maximale Menge eines Referenz-Narkosemittels das Filterelement 11 maximal aufnehmen kann. Außerdem ist für jedes Narkosemittel, bei dem das Filterelement 11 eingesetzt werden kann, jeweils ein Umrechnungsfaktor abgespeichert, und zwar auf dem Datenspeicher 92 oder auf einem anderen Datenspeicher. Das Filterelement 11 vermag von einem Narkosemittel eine maximale Menge aufzunehmen, die gleich der Produkt aus dem Umrechnungsfaktor und der maximalen Menge des Referenz-Narkosemittels ist.

Der Narkosemittel-Mengen-Ermittler 93 empfängt im Einsatz Messwerte von den Sensoren 90 und 91 sowie vom Datenspeicher 92 eine Information über die maximale Menge des Referenz-Narkosemittels, die Umrechnungsfaktoren sowie die Informationen, in welchem Zeitraum jeweils welches Narkosemittel eingesetzt wird. Der Narkosemittel-Mengen-Ermittler 93 steht beispielsweise in einer Datenverbindung mit dem Anästhesiegerät 1, und das Anästhesiegerät 1 übermittelt an den Narkosemittel-Mengen-Ermittler 93 die Information, welches Narkosemittel aktuell verwendet wird und daher durch das Filterelement 11 fließt oder fließen könnte.

Der Narkosemittel-Mengen-Ermittler 93 berechnet so wie gerade beschrieben für jedes verwendeten Narkosemittel, welche Menge dieses Narkosemittels das Filterelement 11 bislang aufgenommen hat. Anhand der Kehrwerte der Umrechnungsfaktoren rechnet der Narkosemittel-Mengen-Ermittler 93 für jedes mögliche Narkosemittel die bislang aufgenommene Menge dieses Narkosemittels in die entsprechende Menge des Referenz-Narkosemittels um. Der Narkosemittel-Mengen-Ermittler 93 addiert die entsprechenden Mengen des Referenz-Narkosemittels auf und berechnet dadurch eine äquivalente aufgenommene Gesamt-Menge des Referenz-Narkosemittels. Das Filterelement 11 ist verbraucht und muss ausgetauscht werden, wenn diese Summe der äquivalenten Mengen die maximale Menge des Referenz-Narkosemittels erreicht oder übersteigt.

Welche Menge eines Narkosemittels ein Filterelement 11 aufzunehmen vermag, hängt in vielen Fällen nicht nur von der Art des Narkosemittels ab, sondern zusätzlich auch von Einsatzbedingungen. In einer Ausgestaltung hängt der gerade beschriebene Umrechnungsfaktor für ein Narkosemittel zusätzlich von mindestens einer der folgenden messbaren Einsatzbedingungen ab:
- der Temperatur, dem Druck und / oder der Feuchte des Gases, welches durch das Filterelement 11 fließt,
- der Konzentration des Narkosemittels in diesem Gas,
- der Temperatur, dem Druck und / oder der Feuchte der umgebenden Luft.

In einer Ausgestaltung wird die Warnung, dass das Filterelement 11 nahezu oder vollständig verbraucht ist, in Form einer Ampel ausgegeben. Grün bedeutet, dass die tatsächlich aufgenommene Menge um mehr als einen vorgegebenen Abstand unter der maximalen Menge liegt. Gelb bedeutet, dass die tatsächlich aufgenommene Menge noch unterhalb der maximalen Menge liegt, aber um weniger als der vorgegebene Abstand. Rot bedeutet, dass die tatsächlich aufgenommene Menge die maximale Menge erreicht oder überstiegen hat. In einer anderen Ausgestaltung wird die bislang aufgenommene Menge eines Narkosemittels als Prozentsatz der maximal möglichen Menge dieses Narkosemittels ausgegeben, beispielsweise getrennt für jedes Narkosemittel.

Die gerade beschriebene Ausgestaltung verwendet eine Information über die maximal mögliche Menge von Narkosemittel, welche das aktuell verwendete Filterelement 11 aufnehmen kann. In einer Ausgestaltung ist diese Information über die maximale Menge auf dem Datenspeicher 92 des eingesetzten Filters 11, 20 abgespeichert. Ein Lesegerät der Aufnahme-Anordnung 100 vermag diesen Datenspeicher auszulesen. In einer anderen Ausgestaltung liest ein Lesegerät ein, von welchem Typ der eingesetzte Filter 11, 20 ist. In einem Datenspeicher ist für jeden möglichen Typ einer Filtereinheit 4 die jeweilige maximale Menge von Narkosemittel hinterlegt, die eine Filtereinheit dieses Typs aufnehmen kann.

In einer Ausgestaltung speichert das Lese- und Schreibgerät auf dem Datenspeicher 92 für jedes verwendete Narkosemittel die Information ab, welche Menge dieses Narkosemittels das Filterelement 11 bislang aufgenommen hat. Bevorzugt wird diese Information laufend aktualisiert. Diese Information lässt sich später dafür verwenden, um das Filterelement 11 wieder aufzubereiten und / oder aus dem Filterelement 11 aufgenommenes Narkosemittel zu gewinnen. Prozessschritte bei der Aufarbeitung eines Filterelements 11 können nämlich von der Art und Menge der aufgenommenen Narkosemittel abhängen.

Optional wird auf dem Datenspeicher 92 oder auf einem zentralen Datenspeicher zusätzlich eine Information über die bislang aufgenommene Menge des Referenz-Narkosemittels abgespeichert. Diese Information wird bevorzugt laufend aktualisiert, sodass ein zeitlicher Verlauf der verbrauchten Menge an Referenz-Narkosemittel erzeugt wird. Dieser zeitliche Verlauf ermöglicht es, näherungsweise eine verbleibende Nutzungsdauer des Filterelements 11 vorherzusagen.

In einer Ausgestaltung wird auf dem Datenspeicher 92 oder auf einem zentralen Datenspeicher für jedes Narkosemittel die bislang aufgenommene Menge abgespeichert. Bevorzugt wird diese Abspeicherung nach jeder Operation oder sonstigem Einsatz des Filters 11, 20 erneut durchgeführt. Vor oder zu Beginn einer Operation werden die bislang aufgenommenen Mengen ausgelesen oder auf andere Weise ermittelt. Die Differenz zwischen den aufgenommenen Mengen nach und vor der Operation liefert ein Maß für die Verbräuche während der Operation.

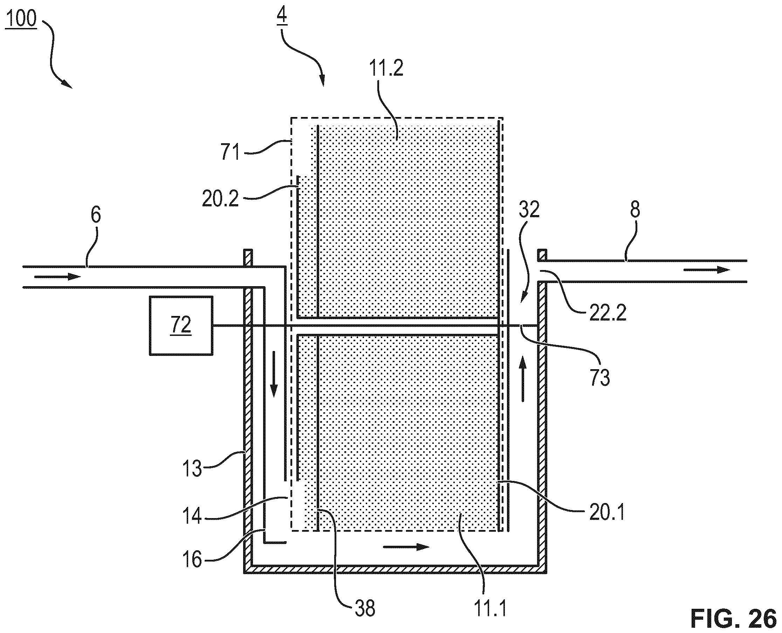

Bei allen bislang beschriebenen Ausgestaltungen umfasst die Filtereinheit 4 ein einziges Filterelement 11, optional in einer Kartusche 20. Figur 26 zeigt eine beispielhafte Ausgestaltung, bei der die Filtereinheit 4 zwei Filterelemente 11.1 und 11.2 in jeweils einer Kartusche 20.1, 20.2 aufweist. Gleiche Bezugszeichen haben die gleichen Bedeutungen wie in den vorigen Figuren.

Die beiden Filter 11.1, 20.1 und 11.2, 20.2 sind in zwei entsprechende Aufnahmen einer Aufnahmeeinheit 71 eingesetzt, wobei die Aufnahmeeinheit 71 die Form einer Revolveraufnahme aufweist und um eine Drehachse 73 drehbar ist. Die horizontale Drehachse 73 ist drehbar mit der Mantelwand des Topfs 13 verbunden und liegt in der Zeichenebene von Figur 26. Dadurch lässt sich wahlweise der Filter 11.1, 20.1 oder der Filter 11.2, 20.2 in eine Position zwischen der Austrittsöffnung 14 am unteren Ende der Topf-Zuführleitung 16 und dem unteren Ende der Topf-Abführleitung 32 bringen. Dadurch ist wahlweise der Filter 11.1, 20.1 oder der Filter 11.2, 20.2 in einer Position, in der Gas durch die Zuführleitung 6, durch diesen Filter 11.1, 20.1 oder 11.2, 20.2 und die Abführleitung 8 strömt. In der in Figur 26 gezeigten Situation kann Gas durch den Filter 11.1, 20.1 strömen, während der Filter 11.2, 20.2 in einer Parkstellung ist.

Bevorzugt rastet die Aufnahmeeinheit 71 ein, wenn sie relativ zum Topf 13 in einer Position ist, in welche die Einlassöffnung 25 des Filters 11.1, 20.1 oder 11.2, 20.2 mit der Austrittsöffnung 14 überlappt. Dadurch wird das Risiko reduziert, dass Gas an einem Filterelement 11.1 oder 11.2 vorbeifließt.

Möglich ist, dass ein Benutzer manuell die Aufnahmeeinheit 71 um die Achse 73 dreht. In einer anderen Ausgestaltung vermag ein optionaler Motor 72 die Aufnahmeeinheit 71 relativ zum Topf 13 um die Achse 73 zu drehen. In einer Ausgestaltung lässt der Motor 72 sich von außen ansteuern oder durch eine Benutzereingabe, beispielsweise durch einen Knopfdruck, aktivieren. Der angesteuerte oder aktivierte Motor 72 dreht die Aufnahmeeinheit 71 um eine Position weiter. Dadurch lässt sich auch durch eine Ansteuerung oder Aktivierung von außen bewirken, dass wahlweise der Filter 11.1, 20.1 oder der Filter 11.2, 20.2 in einer Position ist, in der er von Gas durchströmt wird.

In einer Realisierungsform empfängt ein Steuergerät Messwerte von dem oben beschriebenen Messfühler 15 im Topf 13 und vermag den Motor 72 anzusteuern. Sobald der Messfühler 15 eine hohe Konzentration eines Narkosemittels gemessen hat, steuert das Steuergerät den Motor 72 an, und der Motor 72 dreht die Aufnahmeeinheit 71 um eine Position weiter.

In einer Ausgestaltung sind die beiden Filter 11.1, 20.1 und 11.2, 20.2 gleichartig aufgebaut und vermögen das gleiche Narkosemittel oder die gleichen Narkosemittel aus einem durchströmende Gas herauszufiltern. Die Aufnahmeeinheit 71 wird manuell oder vom Motor 72 gedreht, wenn ein Filter verbraucht ist. In einer anderen Ausgestaltung vermag das Filterelement 11.1 ein erstes Narkosemittel herauszufiltern, das Filterelement 11.2 ein zweites Narkosemittel. Je nachdem, welches Narkosemittel aktuell aus einem Gasstrom herauszufiltern ist, wird der Filter 11.1, 20.1 oder der Filter 11.2, 20.2 in eine Position gebracht, in welcher die Einlassöffnung 25 mit der Austrittsöffnung 14 überlappt.

Möglich ist natürlich, dass die Aufnahmeeinheit 71 Aufnahmen für mehr als zwei Filter umfasst. Möglich ist auch, dass die Aufnahmeeinheit 71 sich relativ zum Topf 13 nicht drehen, sondern linear bewegen oder verschwenken lässt.

In einer Alternative umfasst die Aufnahme-Anordnung 100 eine Weiche, die zwischen der Zuführleitung 6 und dem Topf 13 angeordnet ist. Diese Weiche leitet Gas, welches durch die Zuführleitung 6 fließt, wahlweise zu dem ersten Filter 11.1, 20.2 oder zu dem zweiten Filter 11.2, 20.2 oder zu einem optionalen nicht gezeigten dritten Filter.

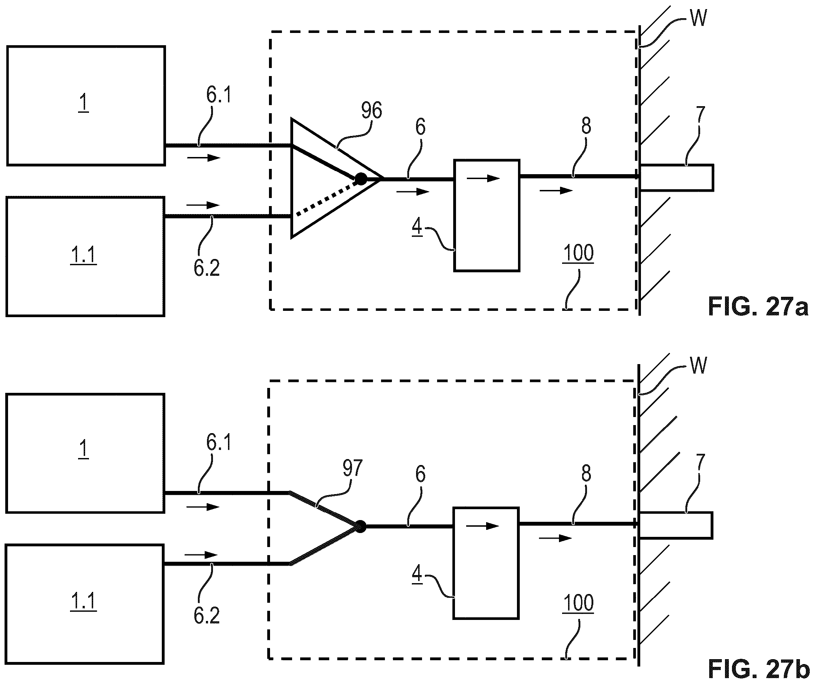

In den bislang beschriebenen Ausgestaltungen ist die erfindungsgemäße Aufnahme-Anordnung 100 mit einem einzigen medizinischen Gerät 1 verbunden. Figur 27 zeigt zwei alternative Ausgestaltungen, bei denen dieselbe erfindungsgemäße Aufnahme-Anordnung 100 mit zwei medizinischen Geräten 1 und 1.1 verbunden oder verbindbar ist. Gleiche Bezugszeichen haben die gleichen Bedeutungen wie in den zuvor beschriebenen Ausgestaltungen.

Bei der Ausgestaltung gemäß Figur 27a) lässt sich die Aufnahme-Anordnung 100 wahlweise mit dem medizinischen Gerät 1 oder mit dem medizinischen Gerät 1.1 verbinden. Eine Leitung 6.1 führt vom medizinischen Gerät 1 zu einer Weiche 96, die zur Aufnahme-Anordnung 100 gehört. Eine Leitung 6.2 führt vom medizinischen Gerät 1.1 ebenfalls zu der Weiche 96. In der in Figur 27a) gezeigten Position stellt die Weiche 96 eine Fluidverbindung zwischen der Leitung 6.1 und der Zuführleitung 6 her. Die Weiche 96 lässt sich in eine Position verstellen, in der sie eine Fluidverbindung zwischen der Leitung 6.2 der Zuführleitung 6 herstellt.

Bei der Ausgestaltung gemäß Figur 27b) umfasst die Aufnahme-Anordnung 100 anstelle einer Weiche 96 ein Y-Stück 97. Die beiden Schenkel dieses Y-Stücks 97 sind mit den beiden Leitungen 6.1 und 6.2 verbunden. Der Balken des Y-Stücks 97 ist mit der Zuführleitung 6 verbunden. Dank des Y-Stücks 97 sind beide medizinischen Geräte 1 und 1.1 gleichzeitig mit der erfindungsgemäßen Aufnahme-Anordnung 100 verbunden.

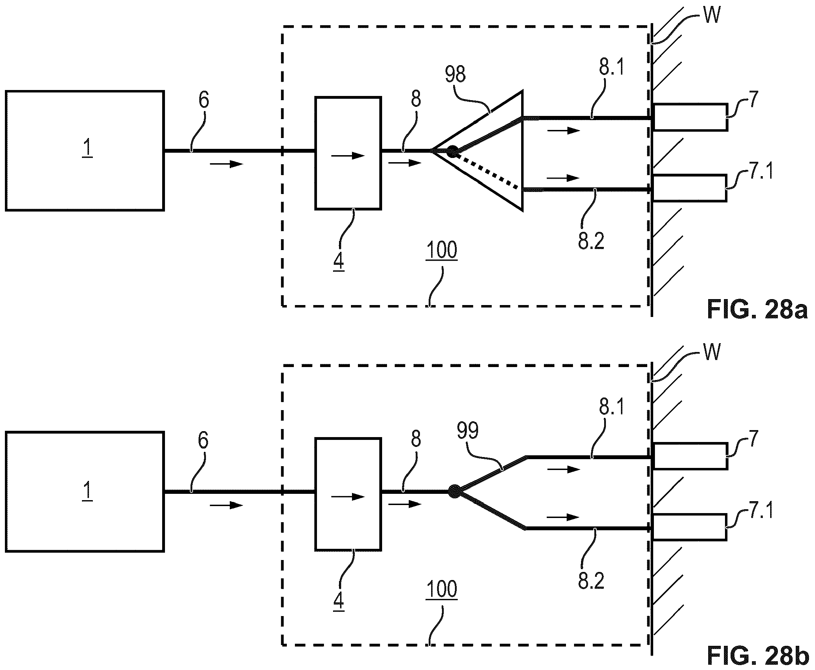

Bei den bislang beschriebenen Ausgestaltungen ist die Aufnahme-Anordnung 100 mit einer stationären Fluidaufnahme 7 in einer Wand W verbunden. Figur 28 zeigt zwei alternative Ausgestaltungen, bei welcher die Aufnahme-Anordnung 100 wahlweise oder auch gleichzeitig mit der stationären Fluidaufnahme 7 oder einer weiteren stationären Fluidaufnahme 7.1 verbunden oder verbindbar ist.

Bei der Ausgestaltung gemäß Figur 28a) verbindet eine Weiche 98 die Abführleitung 8 wahlweise mit einer Leitung 8.1 oder mit einer Leitung 8.2. Die Leitung 8.1 führt zu der Fluidaufnahme 7, die Leitung 8.2 zu der weiteren Fluidaufnahme 7.1. In der in Figur 28a) gezeigten Position ist die Filtereinheit 4 über die Leitungen 8 und 8.1 mit der Fluidaufnahme 7 verbunden.

Bei der Ausgestaltung gemäß Figur 28b) sind die beiden Schenkel eines Y-Stücks 99 mit den beiden Leitungen 8.1 und 8.2 verbunden. Der Balken des Y-Stücks 99 ist mit der Abführleitung 8 verbunden. Dadurch steht die Filtereinheit 4 gleichzeitig mit beiden Fluidaufnahme 7 und 7.1 in einer Fluidverbindung. Dank dieser Ausgestaltung wird auch dann Gas aufgenommen, wenn eine stationäre Fluidaufnahme 7, 7.1 defekt ist oder allein nicht die gesamte Menge des ausgestoßenen Gases aufnehmen kann.

**Bezugszeichenliste**

| | |
|---|---|
| 1 | Anästhesiegerät, umfasst den Narkosemittelverdampfer 2, den Mischer 29, den Kalkfilter 3 und die Ventilatoreinheit 5 |
| 2 | Narkosemittelverdampfer im Anästhesiegerät 1, umfasst den Narkosemitteltank 49 |
| 3 | Kalkfilter, der CO2 aus der ausgeatmeten Luft herausfiltert |
| 4 | Filtereinheit, die aus dem vom Anästhesiegerät 1 abgegebenen Gas Narkosemittel herausfiltert, umfasst den Aktivkohlefilter 11 in der Kartusche 20, die Kartusche 20 und den Topf 13, mit der Zuführleitung 6 und der Abführleitung 8 verbunden |
| 5 | Ventilatoreinheit des Anästhesiegeräts 1, welche Gas im Beatmungskreislauf bewegt |
| 6 | Zuführleitung, führt vom Anästhesiegerät 1 zur Filtereinheit 4 |
| 6.1, 6.2 | Leitungen, welche die Weiche 96 oder das Y-Stück 97 mit den medizinischen Geräten 1 und 1.1 verbinden |
| 7 | stationäre Gasaufnahme der Krankenhaus-Infrastruktur, von der Wand W aufgenommen, über die Abführleitung 8 mit der Filtereinheit 4 verbunden |
| 7.1 | weitere stationäre Gasaufnahme, von der Wand W aufgenommen |
| 8 | Abführleitung, führt von der Filtereinheit 4 zur Gasaufnahme 7 |
| 8.1, 8.2 | Leitungen, welche die Weiche 98 oder das Y-Stück 99 mit den Gasaufnahmen 7 und 7.1 verbinden |
| 9 | Volumenfluss-Sensor in der Abführleitung 8 |
| 10 | Ansaugpumpe an der Abführleitung 8 oder in der Fluidaufnahme 7, steht in einer Fluidverbindung mit der Abführleitung 8 |
| 11 | zylinderförmiger Aktivkohlefilter der Filtereinheit 4, fungiert als Filterelement, wird von der Kartusche 20 umgeben |
| 11.1, 11.2 | zylinderförmige Filterelemente in der Aufnahmeeinheit 71 |
| 12 | umlaufender Vorsprung der Kartusche 20, stützt sich am oberen Rand des Topfs 13 ab |
| 13 | Topf, in den die Zuführleitung 6 hinein führt und aus dem die Abführleitung 8 herausführt, fungiert als Filteraufnahme |
| 14 | Austrittsöffnung der Topf-Zuführleitung 16, am unteren Ende der Topf-Zuführleitung 16 angeordnet, überlappt bei eingesetzter Kartusche 20 mit der Einlassöffnung 25 |
| 14.1 | Pufferspeicher-Austrittsöffnung der Topf-Zuführleitung 16, unterhalb der Austrittsöffnung 14 angeordnet, steht dann, wenn kein Filter 11, 20 eingesetzt ist, in einer Fluidverbindung mit dem Pufferspeicher 19 |
| 15 | Messfühler, der den Zustand des Aktivkohlefilters 11 misst |
| 16 | Topf-Zuführleitung im Inneren des Topfs 13, bildet eine fluiddichte Fortsetzung der Zuführleitung 6, leitet Gas von der Zuführleitung 6 zum Boden 44 des Topfs 13, endet in der Austrittsöffnung 14 |
| 17 | Verbrauchsanzeige für den Aktivkohlefilter 11 |
| 18 | Signalleitung vom Messfühler 15 zur Verbrauchsanzeige 17 |
| 19 | Pufferspeicher in Form eines Zwischenraums zwischen dem Topf 13 und der Kartusche 20 |
| 20 | zylinderförmige Kartusche, umgibt den Aktivkohlefilter 11, umfasst den umlaufenden Vorsprung 12, trägt den Datenspeicher 92 |
| 20.1, 20.2 | zylinderförmige Kartusche für das Filterelement 11.1, 11.2 |
| 21 | Öffnungen im Topf 13, mit der Umgebung verbunden |
| 22.1 | Öffnung im Topf 13, in welcher die Zuführleitung 6 endet |
| 22.2 | Öffnung im Topf 13, in welcher die Abführleitung 8 beginnt |
| 23 | Pufferspeicher in Form eines mäandernden Rohrs, weist die Öffnung 24 auf |
| 24 | Öffnung des Pufferspeichers 23, stellt eine Verbindung mit der Umgebung her |
| 25 | Einlassöffnung nahe dem Boden 39 oder dem Deckel 42 der Kartusche 20, überlappt bei eingesetztem Filter 11, 20 mit der Austrittsöffnung 14 |
| 26 | Öffnung des Pufferspeichers 23, die mit der Einlassöffnung 25 in Verbindung steht |
| 27 | Inspirations-Gasleitung, um den Patienten P mit Atemluft zu versorgen |
| 28 | Exspirations-Gasleitung, um vom Patienten P ausgeatmete Atemluft abzusaugen |
| 29 | Mischer des Anästhesiegeräts 1, erzeugt das Trägergas für das Narkosemittel |
| 30 | Einlassöffnung im Deckel 42 der Kartusche 20 |
| 31 | federnd gelagerte Platte nahe dem Boden 44 des Topfs 13, unterteilt den Topf in einen Filter-Hohlraum 37 und einen Pufferspeicher-Hohlraum 36 |
| 32 | Topf-Abführleitung im Inneren des Topfs 13, leitet Gas vom Boden 44 des Topfs 13 in die Abführleitung 8 |
| 33 | Federelemente, die sich am Boden 44 des Topfs 13 abstützen und bestrebt sind, die Platte 31 vom Boden 44 weg nach oben zu drücken |
| 34 | Auslassöffnung nahe dem Boden 39 der Kartusche 20, überlappt bei eingesetztem Filter 11, 20 mit der Eintrittsöffnung 35 |
| 35 | Eintrittsöffnung der Topf-Abführleitung 32, steht bei eingesetztem Filter 11, 20 in Fluidverbindung mit der Auslassöffnung 34 |
| 35.1 | Pufferspeicher-Eintrittsöffnung der Topf-Abführleitung 32, steht bei entnommenem Filter 11, 20 in Fluidverbindung mit dem Pufferspeicher 19 |
| 36 | Pufferspeicher-Hohlraum in einem unteren Bereich des Topfs 13, von der Platte 31 und der Wand und dem Boden 44 des Topfs 13 gebildet, gehört zum Pufferspeicher, durch die Platte 31 von dem Filter-Hohlraum 37 getrennt |
| 37 | Filter-Hohlraum in einem oberen Bereich des Topfs 13, vermag den Filter 11, 20 aufzunehmen, durch die Platte 31 von dem Pufferspeicher-Hohlraum 36 getrennt |
| 38 | vertikale Trennwand im Inneren des Filters 11, 20, trennt den Aufstiegsbereich Au von dem Abstiegsbereich Ab |
| 39 | Boden der Kartusche 20 |
| 40 | untere Dichtelemente, verhindern, dass Gas aus der Topf-Zuführleitung 16 um die Kartusche 20 herum zu der Topf-Abführleitung 32 fließt |
| 41 | obere Dichtelemente, zwischen dem oberen Rand des Topfs 13 und dem umlaufenden Rand 12 der Kartusche 20 |
| 42 | Deckel der Kartusche 20 |
| 43 | Mantelfläche der Kartusche 20 |
| 44 | Boden des Topfs 13 |
| 45 | Halteelement für ein unteres Dichtelement 40 |
| 47 | Filter-Sensor im Topf 13, der feststellt, ob ein Filter 11, 20 eingesetzt ist oder nicht |
| 48 | Schieber im Inneren des Topfs 13, mit der Platte 31 verbunden |
| 49 | Narkosemitteltank des Narkosemittelverdampfers 2 |
| 50 | Überdruck-Ventil in der Zuführleitung 6 |
| 50.1 | Realisierungsform des Überdruck-Ventils 50, umfasst die Ventilplatte 51 und den Ventilkrater 52 |
| 50.2 | Realisierungsform des Überdruck-Ventils 50, umfasst die Ventilplatte 51, den Ventilkrater 52 und die Druckfeder 55 |
| 50.3 | Realisierungsform des Überdruck-Ventils 50, umfasst die Ventilplatte 51 und die Ventilkugel 56 |
| 50.4 | Realisierungsform des Überdruck-Ventils 50, umfasst den Ventilkrater 52 und die Ventilklappe 57 |
| 50.5 | Realisierungsform des Überdruck-Ventils 50, umfasst das U-förmige Rohr 58 mit der Flüssigkeit 59 |
| 50.6 | Realisierungsform des Überdruck-Ventils 50, umfasst den Druck-Sensor 60 und das ansteuerbare Schaltventil 61 |
| 50.7 | Realisierungsform des Überdruck-Ventils 50, umfasst den Ventilkrater 52 und den Entenschnabel 62 |
| 51 | Ventilplatte des Überdruck-Ventil 50, liegt auf dem Ventilkrater 52 auf |
| 52 | Ventilkrater des Überdruck-Ventils 50 |
| 53 | Trennwand in dem Topf 13 zwischen der Filtereinheit 4 und dem Pufferspeicher 19 |
| 54 | Raum zwischen der Filtereinheit 4 und der Trennwand 53 |
| 55 | Druckfeder, welche bestrebt ist, die Ventilplatte 51 nach oben zu drücken |
| 56 | Ventilkugel, liegt auf dem Ventilkrater 52 auf |
| 57 | Ventilklappe, drehbar mit dem Ventilkrater 52 verbunden |
| 58 | U-förmiges Rohr, in dem sich die Flüssigkeit 59 befindet |
| 59 | Flüssigkeit im Rohr 58 |
| 60 | Druck-Sensor in der Zuführleitung zum ansteuerbaren Schaltventil 61 |
| 61 | ansteuerbares Schaltventil |
| 62 | Entenschnabel, sitzt auf dem Ventilkrater 72 |
| 64 | Schlauch, der das Überdruck-Ventil 50 mit der Abführleitung 8 verbindet |
| 65 | Rückschlag-Ventil in der Abführleitung 8, verhindert, dass Gas aus der Fluidaufnahme 7 durch die Abführleitung 8 zurück zu dem Anästhesiegerät 1 strömt |
| 70 | elastischer Pufferspeicher an der Zuführleitung 6 |
| 71 | Aufnahmeeinheit für die beiden Filter 11.1, 20.1 und 11.2, 20.2, ist als Revolveraufnahme ausgestaltet und um die Achse 73 drehbar |
| 72 | Motor, welcher die Aufnahmeeinheit 71 um die Achse 73 zu drehen vermag |
| 73 | Drehachse der Aufnahmeeinheit 71, drehbar mit dem Topf 13 verbunden |
| 80 | Unterdruck-Ventil am Topf 13 |
| 89 | Füllstands-Sensor, misst ein Maß für den aktuellen Füllstand im Narkosemittel-Tank 49 |
| 90 | Volumenfluss-Sensor des Anästhesiegeräts 1, misst den Volumenfluss von Gas, den das Anästhesiegerät 1 in die Zuführleitung 6 ausstößt |
| 91 | Konzentrations-Sensor, misst die Konzentration von Narkosemittel, die am Narkosemittelverdampfer 2 eingestellt wird oder die der Narkosemittelverdampfer 2 tatsächlich erzielt |
| 92 | Datenspeicher des Filters 11, 20, auf dem eine Information über die maximale Menge von Narkosemittel gespeichert ist, den das Filterelement 11 aufnehmen kann, ist in einer Ausgestaltung ein RFID-Chip oder ein Barcode an der Kartusche 20 |
| 93 | Narkosemittel-Mengen-Ermittler, erhält Messwerte vom Volumenfluss-Sensor 90, vom Konzentrations-Sensor 91 und die Information über die maximale Menge vom Datenspeicher 92, ermittelt die Menge an Narkosemittel, welche das Filterelement 11 bislang aufgenommen hat, und vergleicht die bislang aufgenommene Menge mit der maximalen Menge |
| 96 | Weiche, welche die Zuführleitung 6 wahlweise mit der Leitung 6.1 oder der Leitung 6.2 verbindet |
| 97 | Y-Stück, welches die Leitungen 6.1 und 6.2 mit der Zuführleitung 6 verbindet |
| 98 | Weiche, welche die Abführleitung 8 wahlweise mit der Leitung 8.1 oder der Leitung 8.2 verbindet |
| 99 | Y-Stück, welches die Leitungen 8.1 und 8.2 mit der Abführleitung 8 verbindet |
| 100 | Aufnahme-Anordnung, umfasst die Filtereinheit 4, die Zuführleitung 6; die Abführleitung 8, den Volumenfluss-Sensor 9 und die Ansaugpumpe 10 |
| Ab | Abstiegsbereich im Filter 11, 20, in dem das Gas in der Kartusche 20 nach unten zum Boden 39 absinkt |
| Au | Aufstiegsbereich im Filter 11, 20, in dem das Gas in der Kartusche 20 nach oben zum Deckel 42 aufsteigt |
| P | Patient, der mit dem Anästhesiegerät 1 verbunden ist und mindestens ein Narkosemittel einatmet |
| W | Wand, welche die stationäre Gasaufnahme 7 aufnimmt |

## Patentansprüche

1. Aufnahme-Anordnung (100) zur Aufnahme von Gas aus einem medizinischen Gerät (1), insbesondere aus einem Anästhesiegerät,
wobei die Aufnahme-Anordnung (100)
- eine Zuführleitung (6, 16),
- eine Abführleitung (8, 32),
- eine Filtereinheit (4) mit mindestens einem Filter (11, 20, 11.1, 20.1, 11.2, 20.2) und
- mindestens einen Pufferspeicher (19, 23, 36, 70)
umfasst,
wobei der oder jeder Filter (11, 20, 11.1, 20.1, 11.2, 20.2) der Filtereinheit (4) dazu ausgestaltet ist, mindestens einen vorgegebenen Gas-Bestandteil, Insbesondere mindestens ein Narkosemittel, aus einem Gas, welches durch diesen Filter (11, 20, 11.1, 20.1, 11.2, 20.2) hindurchgeleitet wird, herauszufiltern,
wobei die Zuführleitung (6, 16) dazu ausgestaltet ist, wenigstens zeitweise eine Fluidverbindung mit dem medizinischen Gerät (1) herzustellen,
wobei die Abführleitung (8, 32) dazu ausgestaltet ist, wenigstens zeitweise eine Fluidverbindung mit einer Fluidaufnahme (7) oder der Umgebung herzustellen,
wobei jeweils eine Fluidverbindung (14, 30) zwischen der Zuführleitung (6, 16) und der Filtereinheit (4) sowie der Abführleitung (8, 32) und der Filtereinheit (4) hergestellt oder herstellbar ist,
wobei der oder jeder Pufferspeicher (19, 23, 36, 70)
- in jeweils einer Fluidverbindung (22.1) mit der Zuführleitung (6, 16) und / oder in jeweils einer Fluidverbindung (22.2) mit der Abführleitung (8, 32) steht und
- dazu ausgestaltet ist, Gas aus der Zuführleitung (6, 16) und / oder der Abführleitung (8, 32) zwischenzuspeichern und später wieder in die Zuführleitung (6, 16) und / oder die Abführleitung (8, 32) abzugeben, und
wobei die Aufnahme-Anordnung (100) dazu ausgestaltet ist,
- Gas aus dem medizinischen Gerät (1) durch die Zuführleitung (6, 16) zu der Filtereinheit (4) zu leiten,
- durch die Filtereinheit (4) und hierbei wenigstens teilweise durch den oder einen Filter (11, 20, 11.1, 20.1, 11.2, 20.2) hindurchzuführen und
- durch die Abführleitung (8, 32) zu der Fluidaufnahme (7) oder in die Umgebung zu leiten.

2. Aufnahme-Anordnung (100) nach Anspruch 1,
**dadurch gekennzeichnet, dass**
der oder ein Pufferspeicher (23) zusätzlich in einer Fluidverbindung (24) mit der Umgebung steht und
dazu ausgestaltet ist, Gas in die Umgebung abzugeben.

3. Aufnahme-Anordnung (100) nach einem der vorhergehenden Ansprüche,
**dadurch gekennzeichnet, dass**
die Filtereinheit (4) zusätzlich eine Filteraufnahme (13) umfasst, die in einer Fluidverbindung (14, 30) mit der Zuführleitung (6, 16) und in einer Fluidverbindung (26, 35) mit der Abführleitung (8, 32) steht,
wobei der oder ein Filter (11, 20, 11.1, 20.1, 11.2, 20.2) in die Filteraufnahme (13) eingesetzt oder einsetzbar ist,
wobei die Filtereinheit (4) so ausgestaltet ist, dass nach dem Einsetzen des Filters (11, 20, 11.1, 20.1, 11.2, 20.2) in die Filteraufnahme (13) zwischen dem Filter (11, 20, 11.1, 20.1, 11.2, 20.2) und der Filteraufnahme (13) ein Zwischenraum (19) entsteht, und
wobei dieser Zwischenraum (19)
- in einer Fluidverbindung (25) mit der Zuführleitung (6, 16) und in einer Fluidverbindung (34) mit der Abführleitung (8, 32) steht und
- den oder einen Pufferspeicher (19) bereitstellt oder zu dem oder einem Pufferspeicher (19, 23, 36, 70) gehört.

4. Aufnahme-Anordnung (100) nach einem der vorhergehenden Ansprüche,
**dadurch gekennzeichnet, dass**
der oder ein Pufferspeicher (19, 23, 36, 70) ein Gehäuse (23) umfasst,
welches in einer Fluidverbindung (26) mit der Filtereinheit (4) steht und einen Innenraum zur Aufnahme von Gas bereitstellt.

5. Aufnahme-Anordnung (100) nach einem der vorhergehenden Ansprüche,
**dadurch gekennzeichnet, dass**
der oder mindestens ein Pufferspeicher (23) - gesehen in die Fließrichtung von der Zuführleitung (6, 16) zu der Abführleitung (8, 32) - flussabwärts von der Filtereinheit (4) angeordnet ist oder
die Filtereinheit (4) flussabwärts von dem oder mindestens einem Pufferspeicher (70) angeordnet ist.

6. Aufnahme-Anordnung (100) nach einem der vorhergehenden Ansprüche,
**dadurch gekennzeichnet, dass**
die Filtereinheit (4) zusätzlich eine Filteraufnahme (13) umfasst, die
- einen Innenraum umschließt und
- in einer Fluidverbindung (14, 30) mit der Zuführleitung (6, 16) und in einer Fluidverbindung (26, 35) mit der Abführleitung (8, 32) steht,
wobei eine beweglich angeordnete Platte (31) den Innenraum der Filteraufnahme (13) in einen Filter-Hohlraum (37) und einen Pufferspeicher-Hohlraum (36) unterteilt, bevorzugt fluiddicht unterteilt,
wobei der oder ein Filter (11, 20, 11.1, 20.1, 11.2, 20.2) in die Filteraufnahme (13) eingesetzt oder einsetzbar ist,
wobei der Filter-Hohlraum (37) zur Aufnahme des Filters (11, 20, 11.1, 20.1, 11.2, 20.2) ausgestaltet ist und
wobei der Pufferspeicher-Hohlraum (36)
- zu dem oder einem Pufferspeicher (19, 23, 36, 70) gehört oder den oder einen Pufferspeicher (19, 23, 36, 70) ausbildet und
- mindestens dann, wenn kein Filter (11, 20, 11.1, 20.1, 11.2, 20.2) in die Filteraufnahme (13) eingesetzt ist, in einer Fluidverbindung mit der Zuführleitung (6, 16) und in einer Fluidverbindung mit der Abführleitung (8, 32) steht.

7. Aufnahme-Anordnung (100) nach Anspruch 6,
**dadurch gekennzeichnet, dass**
die Platte (31) relativ zu der Filteraufnahme (13) zwischen einer Pufferspeicher-Position und einer Park-Position hin und her beweglich ist,
wobei die Platte (31)
- dann, wenn kein Filter (11, 20, 11.1, 20.1, 11.2, 20.2) eingesetzt ist, in der Pufferspeicher-Position ist und
- dann, wenn ein Filter (11, 20, 11.1, 20.1, 11.2, 20.2) eingesetzt ist, in der Park-Position ist,
wobei die Zuführleitung (6, 16) mindestens eine Austrittsöffnung (14, 14.1) aufweist,
wobei die Abführleitung (8, 32) mindestens eine Eintrittsöffnung (35, 35.1) aufweist,
wobei dann, wenn die Platte (31) in der Pufferspeicher-Position ist,
- die oder eine Austrittsöffnung (14.1) die Zuführleitung (6, 16) mit dem Pufferspeicher-Hohlraum (30) verbindet und
- die oder eine Eintrittsöffnung (35.1) die Abführleitung (8, 32) mit dem Pufferspeicher-Hohlraum (30) verbindet und
wobei dann, wenn die Platte (31) in der Park-Position ist,
- die oder eine Austrittsöffnung (14) die Zuführleitung (6, 16) mit dem Filter-Hohlraum (37) verbindet und
- die oder eine Eintrittsöffnung (35) die Abführleitung (8, 32) mit dem Filter-Hohlraum (37) verbindet.

8. Aufnahme-Anordnung (100) nach Anspruch 6 oder Anspruch 7,
**dadurch gekennzeichnet, dass**
die Aufnahme-Anordnung (100) einen Schieber (48) umfasst, der
- im Inneren der Filteraufnahme (13) angeordnet ist,
- relativ zu der Filteraufnahme (13) zwischen einer Pufferspeicher-Position und einer Park-Position hin und her beweglich ist und
- bevorzugt mechanisch mit der Platte (31) verbunden ist,
die Zuführleitung (6, 16) eine Filter-Austrittsöffnung (14) und eine Pufferspeicher-Austrittsöffnung (14.1) aufweist und
die Abführleitung (8, 32) eine Filter-Eintrittsöffnung (35) und eine Pufferspeicher-Eintrittsöffnung (35.1) aufweist,
wobei dann, wenn der Schieber (48) in der Pufferspeicher-Position ist,
- die Pufferspeicher-Austrittsöffnung (14.1) die Zuführleitung (6, 16) mit dem Pufferspeicher-Hohlraum (30) verbindet,
- die Pufferspeicher-Eintrittsöffnung (35.1) die Abführleitung (8, 32) mit dem Pufferspeicher-Hohlraum (30) verbindet und
- der Schieber (48) die Filter-Austrittsöffnung (14) und die Filter-Eintrittsöffnung (35) versperrt und
wobei dann, wenn der Schieber (48) in der Park-Position ist,
- die Filter-Austrittsöffnung (14) die Zuführleitung (6, 16) mit dem Filter-Hohlraum (37) verbindet,
- die Filter-Eintrittsöffnung (35) die Abführleitung (8, 32) mit dem Filter-Hohlraum (37) verbindet und
- der Schieber (48) die Pufferspeicher-Austrittsöffnung (14.1) und die Pufferspeicher-Eintrittsöffnung (35.1) versperrt.

9. Aufnahme-Anordnung (100) nach einem der vorhergehenden Ansprüche,
**dadurch gekennzeichnet, dass**
die Filtereinheit (4) zusätzlich eine Filteraufnahme (13) und einen Filter-Sensor (47) umfasst,
wobei die Filtereinheit (4)
- einen Innenraum umschließt und
- in einer Fluidverbindung (14, 30) mit der Zuführleitung (6, 16) und in einer Fluidverbindung (26, 35) mit der Abführleitung (8, 32) steht,
wobei der oder ein Filter (11, 20, 11.1, 20.1, 11.2, 20.2) in die Filteraufnahme (13) eingesetzt oder einsetzbar ist und
wobei der Filter-Sensor (47) dazu ausgestaltet ist, automatisch festzustellen, ob ein Filter (11, 20, 11.1, 20.1, 11.2, 20.2) in die Filteraufnahme (13) eingesetzt ist oder nicht.

10. Aufnahme-Anordnung (100) nach einem der vorhergehenden Ansprüche,
**dadurch gekennzeichnet, dass**
das Volumen des oder mindestens eines Pufferspeichers (70) veränderbar ist,
insbesondere der oder mindestens ein Pufferspeicher (70) aus einem elastischen Material besteht,
wobei eine Zufuhr von Gas in den Pufferspeicher (70) den Pufferspeicher (70) dehnt und
wobei der gedehnte Pufferspeicher (70) bestrebt ist, sich zusammenzuziehen und dadurch aufgenommenes Gas wieder abzugeben.

11. Aufnahme-Anordnung (100) nach einem der vorhergehenden Ansprüche, **dadurch gekennzeichnet, dass**
die Aufnahme-Anordnung (100) mindestens ein Überdruck-Ventil (50) umfasst,
wobei das Überdruck-Ventil (50) sich dann öffnet, wenn die Differenz zwischen dem Druck in der Zuführleitung (6, 16) und einem Druck um die Zuführleitung (6, 16) herum oberhalb einer vorgegeben Überdruck-Schranke liegt.

12. Aufnahme-Anordnung (100) nach Anspruch 11,
**dadurch gekennzeichnet, dass**
das Überdruck-Ventil (50) durch eine Fluidführungseinheit (64) mit der Abführleitung (8, 32) verbunden ist,
wobei bei geöffnetem Überdruck-Ventil (50) eine Fluidverbindung zwischen dem Überdruck-Ventil (50) und der Fluidführungseinheit (64) hergestellt ist.

13. Aufnahme-Anordnung (100) nach Anspruch 11 oder Anspruch 12,
**dadurch gekennzeichnet, dass**
die Aufnahme-Anordnung (100) einen Konzentrations-Sensor (15) umfasst, welcher dazu ausgestaltet ist, die Konzentration mindestens eines Gas-Bestandteils an einer Messposition flussabwärts von dem Filter (11, 20, 11.1, 20.1, 11.2, 20.2) zu ermitteln,
wobei die Aufnahme-Anordnung (100) dazu ausgestaltet ist, dann eine Meldung zu generieren, wenn
- das Überdruck-Ventil (50) geöffnet ist oder
- der Konzentrations-Sensor (15) eine Konzentration oberhalb einer vorgegebenen Konzentrations-Schranke detektiert hat.

14. Aufnahme-Anordnung (100) nach einem der vorhergehenden Ansprüche,
**dadurch gekennzeichnet, dass**
die Aufnahme-Anordnung (100) ein Unterdruck-Ventil (80) umfasst, wobei das Unterdruck-Ventil (80) sich dann öffnet, wenn die Differenz zwischen dem Druck in der Zuführleitung (6, 16) oder im Inneren der Filtereinheit (4) und einem umgebenden Druck unterhalb einer vorgegeben Unterdruck-Schranke liegt.

15. Aufnahme-Anordnung (100) nach Anspruch 14,
**dadurch gekennzeichnet, dass**
in eine Wand (13) der Filtereinheit (4) mindestens eine Öffnung (21) eingelassen ist,
wobei bei geöffnetem Unterdruck-Ventil (80) das Innere der Filtereinheit (4) durch die oder mindestens eine Öffnung (21) in der Wand (13) der Filtereinheit (4) in einer Fluidverbindung mit der Umgebung steht und
wobei das geschlossene Unterdruck-Ventil (80) die Fluidverbindung zwischen dem Inneren der Filtereinheit (4) und der Umgebung verschließt.

16. Aufnahme-Anordnung (100) nach einem der vorhergehenden Ansprüche,
**dadurch gekennzeichnet, dass**
die Filtereinheit (4) zusätzlich eine Filteraufnahme (13) umfasst,
die in einer Fluidverbindung (14, 30) mit der Zuführleitung (6, 16) und in einer Fluidverbindung (26, 35) mit der Abführleitung (8, 32) steht,
wobei der oder ein Filter (11, 20, 11.1, 20.1, 11.2, 20.2) sich in die Filteraufnahme (13) einsetzen und wieder aus der Filteraufnahme (13) herausnehmen lässt.

17. Aufnahme-Anordnung (100) nach einem der vorhergehenden Ansprüche,
**dadurch gekennzeichnet, dass**
die Filtereinheit (4) einen ersten Filter (11.1) und einen zweiten Filter (11.2) umfasst,
wobei die Aufnahme-Anordnung (100) eine Umschalteinrichtung (72) umfasst,
welche dazu ausgestaltet ist, wahlweise den ersten Filter (11.1) oder den zweiten Filter (11.2) in eine Fluidverbindung mit der Zuführleitung (6, 16) und mit der Abführleitung (8, 32) zu verbringen.

18. Aufnahme-Anordnung (100) nach Anspruch 17,
**dadurch gekennzeichnet, dass**
der erste Filter (11.1) dazu ausgestaltet ist, einen ersten vorgegebenen Gas-Bestandteil herauszufiltern und
der zweite Filter (11.2) dazu ausgestaltet ist, einen zweiten vorgegebenen Gas-Bestandteil, der sich von dem ersten Gas-Bestandteil unterscheidet, herauszufiltern.

19. Aufnahme-Anordnung (100) nach einem der vorhergehenden Ansprüche,
**dadurch gekennzeichnet, dass**
die Filtereinheit (4) so ausgestaltet ist, dass
- Gas beim Weg von der Zuführleitung (6, 16) zu der Abführleitung (8, 32) durch den Filter (11, 20, 11.1, 20.1, 11.2, 20.2) hindurch geführt wird und
- verhindert wird, dass das Gas den Filter (11, 20, 11.1, 20.1, 11.2, 20.2) umgeht.

20. Aufnahme-Anordnung (100) nach einem der vorhergehenden Ansprüche,
**dadurch gekennzeichnet, dass**
eine Wand (38) den Filter (11, 20, 11.1, 20.1, 11.2, 20.2) in einen ersten Bereich (Ab) und in einen zweiten Bereich (Au) unterteilt,
wobei der erste Bereich (Au) in einer Fluidverbindung mit der Zuführleitung (6, 16) steht und der zweite Bereich (Ab) in einer Fluidverbindung mit der Abführleitung (8, 32) steht und
wobei die Filtereinheit (4) so ausgestaltet ist, dass Gas von der Zuführleitung (6, 16) durch den ersten Bereich (Au) und dann durch den zweiten Bereich (Ab) hindurch in die Abführleitung (8, 32) fließt.

21. Aufnahme-Anordnung (100) nach einem der vorhergehenden Ansprüche,
**dadurch gekennzeichnet, dass**
die Filtereinheit (4) einen Datenspeicher (92) umfasst und
die Aufnahme-Anordnung (100) ein Lesegerät zum Auslesen des Datenspeichers (92) umfasst,
wobei in dem Datenspeicher (92) mindestens eine der folgenden Informationen abgespeichert ist:
- eine eindeutige Kennung der Filtereinheit (4),
- die Information, ob die Filtereinheit (4) neu oder bereits verwendet worden ist,
- eine Information, für welche Gas-Bestandteile sich diese Filtereinheit (4) verwenden und / oder nicht verwenden lässt,
- eine Information über den Zeitpunkt, an dem die Verwendung der Filtereinheit (4) zum Filtern begonnen worden ist.

22. Aufnahme-Anordnung (100) nach einem der vorhergehenden Ansprüche,
**dadurch gekennzeichnet, dass**
die Aufnahme-Anordnung (100) einen Bestandteil-Mengen-Ermittler (93) umfasst,
welcher dazu ausgestaltet ist,
- ein Signal für den zeitlich veränderlichen Volumenfluss von Fluid durch die Zuführleitung (6) zur Filtereinheit (4) hin und
- ein Signal für die zeitlich veränderliche Konzentration von dem oder mindestens einem Gas-Bestandteil in der Zuführleitung (6) oder
- ein Signal dafür, welche Menge des Gas-Bestandteils in die Zuführleitung (6) eingespeist ist,
zu empfangen,
abhängig von diesen beiden Signalen näherungsweise die Menge des Gas-Bestandteils, welche die Filtereinheit (4) bislang aufgenommen hat, zu ermitteln,
die bislang aufgenommene Menge mit einer vorgegebenen maximalen Menge, welche die Filtereinheit (4) aufzunehmen vermag, zu vergleichen und abhängig vom Ergebnis des Vergleichs eine Meldung zu erzeugen.

23. Aufnahme-Anordnung (100) nach Anspruch 22,
**dadurch gekennzeichnet, dass**
die Filtereinheit (4) einen Datenspeicher (92) umfasst,
in welchem eine Information über die maximale Menge des oder mindestens eines Gas-Bestandteils, welche der Filter (11, 20, 11.1, 20.1, 11.2, 20.2) aufzunehmen vermag, abgespeichert ist,
wobei der Bestandteil-Mengen-Ermittler (93) dazu ausgestaltet ist, beim Vergleich als vorgegebene maximale Menge die in dem Datenspeicher (92) abgespeicherte Information zu verwenden.

24. Aufnahme-Anordnung (100) nach einem der vorhergehenden Ansprüche,
**dadurch gekennzeichnet, dass**
die Aufnahme-Anordnung (100) einen Unterdruck-Erzeuger, insbesondere eine Ansaugpumpe (10), umfasst,
wobei die Abführleitung (8) in einer Fluidverbindung mit dem Unterdruck-Erzeuger (10) steht und
wobei der Unterdruck-Erzeuger (10) dazu ausgestaltet ist, einen Unterdruck in der Abführleitung (8) zu erzeugen.

25. Verwendung einer Aufnahme-Anordnung (100) nach einem der vorhergehenden Ansprüche
zur Aufnahme von Gas aus einem medizinischen Gerät (1),
insbesondere zur Aufnahme mindestens eines Narkosemittels aus einem Anästhesiegerät.

26. Medizinische Anordnung umfassend
- ein medizinisches Gerät (1), insbesondere ein Anästhesiegerät, und
- mindestens eine Aufnahme-Anordnung (100) nach einem der Ansprüche 1 bis 24,
wobei die jeweilige Zuführleitung (6, 16) der oder jeder Aufnahme-Anordnung (100) wenigstens zeitweise in einer Fluidverbindung mit dem medizinischen Gerät (1) steht.

27. Medizinische Anordnung nach Anspruch 26,
**dadurch gekennzeichnet, dass**
die medizinische Anordnung ein weiteres medizinisches Gerät (1.1) umfasst,
wobei sich wahlweise oder gleichzeitig eine Fluidverbindung (6.1, 6.2) zwischen dem medizinischen Gerät (1) und der Zuführleitung (6) oder dem weiteren medizinischen Gerät (1.1) und der Zuführleitung (6) derselben Aufnahme-Anordnung (100) herstellen lässt.

28. Medizinische Anordnung nach Anspruch 26 oder Anspruch 27,
**dadurch gekennzeichnet, dass**
die Aufnahme-Anordnung (100) im Inneren des medizinischen Geräts (1) angeordnet ist.

29. Medizinisches System umfassend
- eine medizinische Anordnung nach einem der Ansprüche 26 bis 28 und
- eine Fluidaufnahme (7), bevorzugt eine stationäre Fluidaufnahme (7), wobei die Abführleitung (8, 32) der Aufnahme-Anordnung (100) wenigstens zeitweise in einer Fluidverbindung mit der Fluidaufnahme (7) steht.

30. Medizinisches System nach Anspruch 29,
**dadurch gekennzeichnet, dass**
das medizinische System eine weitere Fluidaufnahme (7.1), bevorzugt eine weitere stationäre Fluidaufnahme (7.1), umfasst,
wobei sich wahlweise oder gleichzeitig eine Fluidverbindung (7.1, 7.2) zwischen der Fluidaufnahme (7) und der Abführleitung (8, 32) oder der weiteren Fluidaufnahme (7.1) und der Abführleitung (8, 32) herstellen lässt.

31. Verfahren zur Aufnahme von Gas aus einem medizinischen Gerät (1), insbesondere aus einem Anästhesiegerät,
wobei das Verfahren unter Verwendung einer Aufnahme-Anordnung (100) durchgeführt wird,
wobei die Aufnahme-Anordnung (100)
- eine Zuführleitung (6, 16),
- eine Abführleitung (8, 32),
- eine Filtereinheit (4) mit mindestens einem Filter (11, 20, 11.1, 20.1, 11.2, 20.2) und
- mindestens einen Pufferspeicher (19, 23, 36, 70)
umfasst und
wobei das Verfahren die Schritte umfasst, dass
- eine Fluidverbindung zwischen dem medizinischen Gerät (1) und der Zuführleitung (6, 16) hergestellt wird,
- eine Fluidverbindung zwischen der Abführleitung (8, 32) und einer Fluidaufnahme (7) oder der Umgebung hergestellt wird,
- Gas aus dem medizinischen Gerät (1) austritt, insbesondere ausgestoßen und / oder abgesogen wird,
- die Aufnahme-Anordnung (100) das ausgetretene Gas durch die Zuführleitung (6, 16), die Filtereinheit (4) und die Abführleitung (8, 32) hindurch zu der Fluidaufnahme (7) oder in die Umgebung leitet,
- wobei das gesamte Gas oder wenigstens ein Teil des Gases beim Geführtwerden durch die Filtereinheit (4) hindurch durch den oder einen Filter (11, 20, 11.1, 20.1, 11.2, 20.2) geleitet wird, und
- dieser Filter (11, 20, 11.1, 20.1, 11.2, 20.2) aus dem Gas, das durch den Filter (11, 20, 11.1, 20.1, 11.2, 20.2) geleitet wird, mindestens einen vorgegebenen Gas-Bestandteil, insbesondere ein Narkosemittel, herausfiltert,
wobei dann, wenn mehr Gas aus dem medizinischen Gerät (1) austritt als in die Fluidaufnahme (7) oder die Umgebung geleitet wird, Gas aus der Zuführleitung (6, 16) und / oder aus der Abführleitung (8, 32) in den oder mindestens einen Pufferspeicher (19, 23, 36, 70) geleitet wird und der Pufferspeicher (19, 23, 36, 70) das Gas zwischengespeichert und
wobei dann, wenn weniger Gas austritt, zwischengespeichertes Gas aus dem oder mindestens einen Pufferspeicher (19, 23, 36, 70) in die Abführleitung (8, 32) und / oder in die Zuführleitung (6, 16) geleitet wird.

32. Verfahren nach Anspruch 31,
**dadurch gekennzeichnet, dass**
die Filtereinheit (4) zusätzlich eine Filteraufnahme (13) umfasst, die
- einen Innenraum umschließt und
- wenigstens zeitweise in einer Fluidverbindung (14, 30) mit der Zuführleitung (6, 16) und wenigstens zeitweise in einer Fluidverbindung (26, 35) mit der Abführleitung (8, 32) steht,
wobei eine beweglich angeordnete Platte (31) den Innenraum der Filteraufnahme (13) in einen Filter-Hohlraum (37) und einen Pufferspeicher-Hohlraum (36) unterteilt, bevorzugt fluiddicht unterteilt,
wobei der oder ein Filter (11, 20, 11.1, 20.1, 11.2, 20.2) in den Filter-Hohlraum (37) eingesetzt wird oder ist,
wobei mindestens einmal der Filter (11, 20, 11.1, 20.1, 11.2, 20.2) aus der Filteraufnahme (13) entnommen wird und
wobei mindestens bei entnommenen Filter (11, 20, 11.1, 20.1, 11.2, 20.2) der Pufferspeicher-Hohlraum (36) in einer Fluidverbindung mit der Zuführleitung (6, 16) und in einer Fluidverbindung mit der Abführleitung (8, 32) steht.
